# EUROPEAN PATENT APPLICATION

(11) **EP 3 988 655 A2**
(43) Date of publication of application: **27.04.2022**
(21) Application number: 21193857.6
(22) Date of filing: 22.12.2016
(51) Int. Cl.: C12N 9/22, C12N 15/90, A61K 39/00, A61P 37/04, C07K 14/725, C12N 15/52, A61P 35/00, C12N 15/62

(54) **ENGINEERED MEGANUCLEASES WITH RECOGNITION SEQUENCES FOUND IN THE HUMAN BETA-2 MICROGLOBULIN GENE**

(30) Priority: 23.12.2015 US 201562387318 P; 02.11.2016 US 201662416513 P
(62) Divisional of application: 16825979.4
(71) Applicant: Precision Biosciences, Inc., Durham, NC 27701 (US)
(72) Inventor: BARTSEVICH, Victor, Durham, NC (US); PHAM, Christina, Westlake Village, CA (US); MARTIN, Aaron, Carrboro, NC (US); JANTZ, Derek, Durham, NC (US); SMITH, James, Jefferson, Morrisville, NC (US); NICHOLSON, Michael, G., Chapel Hill, NC (US)
(74) Representative: Grund, Martin

(57) **Abstract**

Disclosed herein are recombinant meganucleases engineered to recognize and cleave a recognition sequence present in the human beta-2 microglobulin gene. The disclosure further relates to the use of such recombinant meganucleases in methods for producing genetically-modified eukaryotic cells, and to a population of genetically-modified T cells having reduced cell-surface expression of beta-2 microglobulin.

## Description

### CROSS REFERENCE TO RELATED APPLICATIONS

This application claims priority to U.S. Provisional Application No. 62/387,318, entitled "Engineered Meganucleases with Recognition Sequences Found in the Human Beta-2 Microglobulin Gene," filed December 23, 2015, and U.S. Provisional Application No. 62/416,513, entitled "Engineered Meganucleases with Recognition Sequences Found in the Human Beta-2 Microglobulin Gene," filed November 2, 2016, the disclosures of which are hereby incorporated by reference in their entireties.

### FIELD OF THE INVENTION

The present disclosure relates to the field of molecular biology and recombinant nucleic acid technology. In particular, the present disclosure relates to recombinant meganucleases engineered to recognize and cleave recognition sequences found in the human beta-2 microglobulin gene. The present disclosure further relates to the use of such recombinant meganucleases in methods for producing genetically-modified eukaryotic cells, and to a population of genetically-modified cells having reduced cell-surface expression of beta-2 microglobulin.

### REFERENCE TO A SEQUENCE LISTING SUBMITTED AS

### A TEXT FILE VIA EFS-WEB

Filed with the instant application is a paper copy of a Sequence Listing which is hereby incorporated by reference in its entirety. This paper copy corresponds to a copy in ASCII format which is also hereby incorporated by reference in its entirety. Said ASCII copy, created on December 20, 2016, is named 2000706-00181WO1.txt, and is 741,693 bytes in size.

### BACKGROUND OF THE INVENTION

T cell adoptive immunotherapy is a promising approach for cancer treatment. This strategy utilizes isolated human T cells that have been genetically-modified to enhance their specificity for a specific tumor associated antigen. Genetic modification may involve the expression of a chimeric antigen receptor (CAR) or an exogenous T cell receptor to graft antigen specificity onto the T cell. By contrast to exogenous T cell receptors, CARs derive their specificity from the variable domains of a monoclonal antibody. Thus, T cells expressing CARs induce tumor immunoreactivity in a major histocompatibility complex (MHC) non-restricted manner. To date, T cell adoptive immunotherapy has been utilized as a clinical therapy for a number of cancers, including B cell malignancies (e.g., acute lymphoblastic leukemia (ALL), B cell non-Hodgkin lymphoma (NHL), and chronic lymphocytic leukemia), multiple myeloma, neuroblastoma, glioblastoma, advanced gliomas, ovarian cancer, mesothelioma, melanoma, and pancreatic cancer.

Despite its potential usefulness as a cancer treatment, adoptive immunotherapy has been limited, in part, by alloreactivity between host tissues and allogeneic CAR T cells. One cause of alloreactivity arises from the presence of non-host MHC class I molecules on the cell-surface of CAR T cells. MHC class I molecules consist of two polypeptide chains, α and β. In humans, the α chain consists of three subunits, α1, α2, and α3, which are encoded by polymorphic human leukocyte antigen (HLA) genes on chromosome 6. The variability of HLA loci, and the encoded α chain subunits, can cause allogeneic CAR T cells to be seen by the host immune system as foreign cells because they bear foreign MHC class I molecules. As a result, CAR T cells administered to a patient can be subject to host versus graft (HvG) rejection, where they are recognized and killed by the host's cytotoxic T cells.

The β chain of MHC class I molecules consists of beta-2 microglobulin, which is encoded by the non-polymorphic beta-2 microglobulin (B2M) gene on chromosome 15 (SEQ ID NO: 1). Beta-2 microglobulin is non-covalently linked to α3 subunit and is common to all MHC class I molecules. Furthermore, expression of MHC class I molecules at the cell surface requires its association with beta-2 microglobulin. As such, beta-2 microglobulin represents a logical target for suppressing the expression of MHC class I molecules on CAR T cells, which could render the cells invisible to host cytotoxic T cells and reduce alloreactivity.

Another cause of alloreactivity to CAR T cells is the expression of the endogenous T cell receptor on the cell surface. T cell receptors typically consist of variable α and β chains or, in smaller numbers, variable γ and δ chains. The T cell receptor complexes with accessory proteins, including CD3, and functions with cell-surface co-receptors (e.g., CD4 and CD8) to recognize antigens bound to MHC molecules on antigen presenting cells. In the case of allogeneic CAR T cells, expression of endogenous T cell receptors may cause the cell to recognize host MHC antigens following administration to a patient, which can lead to the development of graft-versus-host-disease (GVHD).

To forestall alloreactivity, clinical trials have largely focused on the use of autologous CAR T cells, wherein a donor's T cells are isolated, genetically-modified to incorporate a chimeric antigen receptor, and then re-infused into the same subject. An autologous approach provides immune tolerance to the administered CAR T cells; however, this approach is constrained by both the time and expense necessary to produce patient-specific CAR T cells after a patient's cancer has been diagnosed.

Therefore, a need exists for the development of allogeneic CAR T cells that lack expression of beta-2 microglobulin and MHC class I molecules, as well as cells that further lack expression of endogenous T cell receptors.

### SUMMARY OF THE INVENTION

The present disclosure provides a recombinant meganuclease that is engineered to recognize and cleave a recognition sequence within the human beta-2 microglobulin gene (SEQ ID NO: 1). Such a meganuclease is useful for disrupting the beta-2 microglobulin gene and, consequently, disrupting the expression and/or function of endogenous MHC class I receptors. Meganuclease cleavage can disrupt gene function either by the mutagenic action of non-homologous end joining or by promoting the introduction of an exogenous polynucleotide into the gene via homologous recombination. The present disclosure further provides methods comprising the delivery of a recombinant meganuclease protein, or genes encoding a recombinant meganuclease, to a eukaryotic cell in order to produce a genetically-modified eukaryotic cell.

The present disclosure further provides for "off the shelf' CAR T cells, prepared using T cells from a third party donor, that are not alloreactive and do not induce HvG rejection or GVHD because they lack expression of beta-2 microglobulin and MHC class I molecules and/or further lack expression of endogenous T cell receptors. Such products can be generated and validated in advance of diagnosis, and can be made available to patients as soon as necessary.

The present disclosure further relates to the use of site-specific, rare-cutting, homing endonucleases (also called "meganucleases") that are engineered to recognize specific DNA sequences in a locus of interest. Homing endonucleases are a group of naturally-occurring nucleases which recognize 15-40 base pair cleavage sites commonly found in the genomes of plants and fungi. They are frequently associated with parasitic DNA elements, such as group 1 self-splicing introns and inteins. They naturally promote homologous recombination or gene insertion at specific locations in the host genome by producing a double-stranded break in the chromosome, which recruits the cellular DNA-repair machinery (Stoddard (2006), Q. Rev. Biophys. 38:49-95). Homing endonucleases are commonly grouped into four families: the LAGLIDADG (SEQ ID NO:11) family, the GIY-YIG family, the His-Cys box family and the HNH family. These families are characterized by structural motifs, which affect catalytic activity and recognition sequence. For instance, members of the LAGLIDADG (SEQ ID NO:11) family are characterized by having either one or two copies of the conserved LAGLIDADG (SEQ ID NO:11) motif (Chevalier et al. (2001), Nucleic Acids Res. 29(18): 3757-3774). The LAGLIDADG (SEQ ID NO:11) homing endonucleases with a single copy of the LAGLIDADG (SEQ ID NO:11) motif form homodimers, whereas members with two copies of the LAGLIDADG (SEQ ID NO:11) motif are found as monomers.

Methods for producing engineered, site-specific recombinant meganucleases are known in the art. I-CreI (SEQ ID NO:10) is a member of the LAGLIDADG (SEQ ID NO:11) family of homing endonucleases which recognizes and cuts a 22 base pair recognition sequence in the chloroplast chromosome of the algae *Chlamydomonas reinhardtii.* Genetic selection techniques have been used to modify the wild-type I-CreI cleavage site preference (Sussman et al. (2004), J. Mol. Biol. 342: 31-41; Chames et al. (2005), Nucleic Acids Res. 33: e178; Seligman et al. (2002), Nucleic Acids Res. 30: 3870-9, Arnould et al. (2006), J. Mol. Biol. 355: 443-58). More recently, a method of rationally-designing mono-LAGLIDADG (SEQ ID NO:11) homing endonucleases was described which is capable of comprehensively redesigning I-CreI and other homing endonucleases to target widely-divergent DNA sites, including sites in mammalian, yeast, plant, bacterial, and viral genomes (WO 2007/047859).

As first described in WO 2009/059195, I-CreI and its engineered derivatives are normally dimeric but can be fused into a single polypeptide using a short peptide linker that joins the C-terminus of a first subunit to the N-terminus of a second subunit (Li, et al. (2009) Nucleic Acids Res. 37:1650-62; Grizot, et al. (2009) Nucleic Acids Res. 37:5405-19.) Thus, a functional "single-chain" meganuclease can be expressed from a single transcript. Such engineered meganucleases exhibit an extremely low frequency of off-target cutting. By delivering a gene encoding a single-chain meganuclease to a cell, it is possible to specifically and preferentially target, cleave, and disrupt the beta-2 microglobulin gene.

The use of engineered meganucleases for cleaving DNA targets in the human beta-2 microglobulin gene was previously disclosed in International Publication Nos. WO 2008/102199 ("the '199 application") and WO 2008/102274 ("the '274 application"). The '199 application and the '274 application each disclose I-CreI variants having amino acid substitutions that are intended to increase selectivity of the meganuclease for recognition sequences found in the beta-2 microglobulin gene. However, the meganucleases described were only shown to have activity against beta-2 microglobulin DNA targets in yeast or CHO reporter cell systems. The present disclosure improves upon the teachings of the prior art by providing recombinant meganucleases that efficiently target and cleave recognition sequences in the beta-2 microglobulin gene in human T cells.

Thus, in one aspect, the disclosure provides a recombinant meganuclease that recognizes and cleaves a recognition sequence within the human beta-2 microglobulin gene (SEQ ID NO: 1). Such a recombinant meganuclease comprises a first subunit and a second subunit, wherein the first subunit binds to a first recognition half-site of the recognition sequence and comprises a first hypervariable (HVR1) region, and wherein the second subunit binds to a second recognition half-site of the recognition sequence and comprises a second hypervariable (HVR2) region.

In one embodiment, the recognition sequence comprises SEQ ID NO:2 *(i.e.,* the B2M 13-14 recognition sequence).

In one such embodiment, the first meganuclease subunit comprises an amino acid sequence having at least 80%, at least 85%, at least 90%, or at least 95% sequence identity to residues 198-344 of any one of SEQ ID NOs: 12-96 or residues 7-153 of any one of SEQ ID NOs:97-100, and the second meganuclease subunit comprises an amino acid sequence having at least 80%, at least 85%, at least 90%, or at least 95% sequence identity to residues 7-153 of any one of SEQ ID NOs: 12-96 or residues 198-344 of any one of SEQ ID NOs:97-100.

In another such embodiment, the HVR1 region comprises Y at a position corresponding to: (a) position 215 of any one of SEQ ID NOs: 12-96; or (b) position 24 of any one of SEQ ID NOs:97-100. In another such embodiment, the HVR1 region comprises F at a position corresponding to: (a) position 261 of any one of SEQ ID NOs: 12-96; or (b) position 70 of any one of SEQ ID NOs:97-100. In another such embodiment, the HVR1 region comprises one or more of Y and F at positions corresponding to: (a) positions 215 and 261, respectively, of any one of SEQ ID NOs: 12-96; or (b) positions 24 and 70, respectively, of any one of SEQ ID NOs:97-100.

In another such embodiment, the HVR2 region comprises Y at a position corresponding to: (a) position 24 of any one of SEQ ID NOs: 12-96; or (b) position 215 of any one of SEQ ID NOs:97-100. In another such embodiment, the HVR2 region comprises Y at a position corresponding to: (a) position 42 of any one of SEQ ID NOs: 12-96; or (b) position 233 of any one of SEQ ID NOs:97-100. In another such embodiment, the HVR2 region comprises one or more of Y and Y at positions corresponding to: (a) positions 24 and 42, respectively, of any one of SEQ ID NOs:97-100; or (b) positions 215 and 233, respectively, of any one of SEQ ID NOs:97-100.

In another such embodiment, the HVR1 region comprises residues 215-270 of any one of SEQ ID NOs: 12-96 or residues 24-79 of any one of SEQ ID NOs:97-100. In another such embodiment, the HVR2 region comprises residues 24-79 of any one of SEQ ID NOs:12-96 or residues 215-270 of any one of SEQ ID NOs:97-100.

In another such embodiment, the first meganuclease subunit comprises residues 198-344 of any one of SEQ ID NOs: 12-96 or residues 7-153 of any one of SEQ ID NOs:97-100. In another such embodiment, the second meganuclease subunit comprises residues 7-153 of any one of SEQ ID NOs: 12-96 or residues 198-344 of any one of SEQ ID NOs:97-100.

In another such embodiment, the recombinant meganuclease is a single-chain meganuclease comprising a linker, wherein the linker covalently joins the first subunit and the second subunit.

In another such embodiment, the recombinant meganuclease comprises the amino acid sequence of any one of SEQ ID NOs: 12-100.

In a further embodiment, the recognition sequence comprises SEQ ID NO:4 *(i.e.,* the B2M 5-6 recognition sequence).

In one such embodiment, the first meganuclease subunit comprises an amino acid sequence having at least 80%, at least 85%, at least 90%, or at least 95% sequence identity to residues 7-153 of any one of SEQ ID NOs:101-111 or residues 198-344 of any one of SEQ ID NOs:112 or 113, and the second meganuclease subunit comprises an amino acid sequence having at least 80%, at least 85%, at least 90%, or at least 95% sequence identity to residues 198-344 of any one of SEQ ID NOs:101-111 or residues 7-153 of any one of SEQ ID NOs:112 or 113.

In another such embodiment, the HVR1 region comprises Y at a position corresponding to: (a) position 24 of any one of SEQ ID NOs:101-111; or (b) position 215 of any one of SEQ ID NOs:112 or 113.

In another such embodiment, the HVR2 region comprises Y at a position corresponding to: (a) position 215 of any one of SEQ ID NOs:101-111; or (b) position 24 of any one of SEQ ID NOs:112 or 113. In another such embodiment, the HVR2 region comprises W at a position corresponding to: (a) position 233 of any one of SEQ ID NOs:101-111; or (b) position 42 of any one of SEQ ID NOs:112 or 113. In another such embodiment, the HVR2 region comprises one or more of Y and W at positions corresponding to: (a) positions 215 and 233, respectively, of any one of SEQ ID NOs:101-111; or (b) positions 24 and 42, respectively, of any one of SEQ ID NOs:112 or 113.

In another such embodiment, the HVR1 region comprises residues 24-79 of any one of SEQ ID NOs:101-111 or residues 215-270 of any one of SEQ ID NOs:112 or 113. In another such embodiment, the HVR2 region comprises residues 215-270 of any one of SEQ ID NOs:101-111 or residues 24-79 of any one of SEQ ID NOs:112 or 113.

In another such embodiment, the first meganuclease subunit comprises residues 7-153 of any one of SEQ ID NOs:101-111 or residues 198-344 of any one of SEQ ID NOs:112 or 113. In another such embodiment, the second meganuclease subunit comprises residues 198-344 of any one of SEQ ID NOs:101-111 or residues 7-153 of any one of SEQ ID NOs:112 or 113.

In another such embodiment, the recombinant meganuclease is a single-chain meganuclease comprising a linker, wherein the linker covalently joins the first subunit and the second subunit.

In another such embodiment, the recombinant meganuclease comprises the amino acid sequence of any one of SEQ ID NOs:101-113.

In a further embodiment, the recognition sequence comprises SEQ ID NO:6 *(i.e.,* the B2M 7-8 recognition sequence).

In one such embodiment, the first meganuclease subunit comprises an amino acid sequence having at least 80%, at least 85%, at least 90%, or at least 95% sequence identity to residues 7-153 of any one of SEQ ID NOs:114-118 or residues 198-344 of any one of SEQ ID NOs:119-124, and the second meganuclease subunit comprises an amino acid sequence having at least 80%, at least 85%, at least 90%, or at least 95% sequence identity to residues 198-344 of any one of SEQ ID NOs:114-118 or residues 7-153 of any one of SEQ ID NOs:119-124.

In another such embodiment, the HVR1 region comprises S at a position corresponding to: (a) position 44 of any one of SEQ ID NOs:114-118; or (b) position 235 of any one of SEQ ID NOs:119-124. In another such embodiment, the HVR1 region comprises F at a position corresponding to: (a) position 46 of any one of SEQ ID NOs:114-118; or (b) position 237 of any one of SEQ ID NOs:119-124. In another such embodiment, the HVR1 region comprises one or more of S and F at positions corresponding to: (a) positions 44 and 46, respectively, of any one of SEQ ID NOs:114-118; or (b) positions 235 and 237, respectively, of any one of SEQ ID NOs:119-124.

In another such embodiment, the HVR2 region comprises Y at a position corresponding to: (a) position 215 of any one of SEQ ID NOs:114-118; or (b) position 24 of any one of SEQ ID NOs:119-124.

In another such embodiment, the HVR1 region comprises residues 24-79 of any one of SEQ ID NOs:114-118 or residues 215-270 of any one of SEQ ID NOs:119-124. In another such embodiment, the HVR2 region comprises residues 215-270 of any one of SEQ ID NOs:114-118 or residues 24-79 of any one of SEQ ID NOs:119-124.

In another such embodiment, the first meganuclease subunit comprises residues 7-153 of any one of SEQ ID NOs:114-118 or residues 198-344 of any one of SEQ ID NOs:119-124. In another such embodiment, the second meganuclease subunit comprises residues 198-344 of any one of SEQ ID NOs:114-118 or residues 7-153 of any one of SEQ ID NOs:119-124.

In another such embodiment, the recombinant meganuclease is a single-chain meganuclease comprising a linker, wherein the linker covalently joins the first subunit and the second subunit.

In another such embodiment, the recombinant meganuclease comprises the amino acid sequence of any one of SEQ ID NOs:114-124.

In a further embodiment, the recognition sequence comprises SEQ ID NO:8 *(i.e.,* the B2M 11-12 recognition sequence).

In one such embodiment, the first meganuclease subunit comprises an amino acid sequence having at least 80%, at least 85%, at least 90%, or at least 95% sequence identity to residues 7-153 of SEQ ID NO:125 or residues 198-344 of SEQ ID NO:126, and the second meganuclease subunit comprises an amino acid sequence having at least 80%, at least 85%, at least 90%, or at least 95% sequence identity to residues 198-344 of SEQ ID NO:125 or residues 7-153 of SEQ ID NO:126.

In another such embodiment, the HVR1 region comprises Y at a position corresponding to: (a) position 24 of SEQ ID NO:125; or (b) position 215 of SEQ ID NO:126. In another such embodiment, the HVR1 region comprises W at a position corresponding to: (a) position 28 of SEQ ID NO: 125; or (b) position 219 of SEQ ID NO: 126. In another such embodiment, the HVR1 region comprises G at a position corresponding to: (a) position 42 of SEQ ID NO: 125; or (b) position 233 of SEQ ID NO: 126. In another such embodiment, the HVR1 region comprises one or more of Y, W, and G at positions corresponding to: (a) positions 24, 28, and 42, respectively, of SEQ ID NO: 125; or (b) positions 215, 219, and 233, respectively, of SEQ ID NO: 126.

In another such embodiment, the HVR2 region comprises V at a position corresponding to: (a) position 233 of SEQ ID NO: 125; or (b) position 42 of SEQ ID NO: 126.

In another such embodiment, the HVR1 region comprises residues 24-79 of SEQ ID NO: 125 or residues 215-270 of SEQ ID NO: 126. In another such embodiment, the HVR2 region comprises residues 215-270 of SEQ ID NO: 125 or residues 24-79 of SEQ ID NO: 126.

In another such embodiment, the first meganuclease subunit comprises residues 7-153 of SEQ ID NO: 125 or residues 198-344 of SEQ ID NO: 126. In another such embodiment, the second meganuclease subunit comprises residues 198-344 of SEQ ID NO: 125 or residues 7-153 of SEQ ID NO: 126.

In another such embodiment, the recombinant meganuclease is a single-chain meganuclease comprising a linker, wherein the linker covalently joins the first subunit and the second subunit.

In another such embodiment, the recombinant meganuclease comprises the amino acid sequence of any one of SEQ ID NOs:125 and 126.

In another aspect, the present disclosure provides an isolated polynucleotide comprising a nucleic acid sequence encoding a recombinant meganuclease described herein.

In the various aspects of the present disclosure, the polynucleotide is an mRNA. In one embodiment, the mRNA can encode a single recombinant meganuclease described herein. In other embodiments, the mRNA is a polycistronic mRNA (e.g., bicistronic, tricistronic, etc.) encoding at least one meganuclease described herein and one additional gene. In particular embodiments, a polycistronic mRNA can encode two or more meganucleases of the present disclosure which target different recognition sequences within the same gene. In other embodiments, a polycistronic mRNA can encode a meganuclease described herein and a second nuclease which targets a different recognition sequence within the same gene or, alternatively, targets a different recognition sequence within another gene. In a specific embodiment, a polycistronic mRNA can encode a meganuclease described herein which recognizes and cleaves a B2M recognition sequence described herein and a nuclease which recognizes and cleaves a recognition sequence within the T cell receptor alpha constant region.

In another aspect, the present disclosure provides a recombinant DNA construct comprising an isolated polynucleotide, wherein the isolated polynucleotide comprises a nucleic acid sequence encoding a recombinant meganuclease described herein. In one embodiment, the recombinant DNA construct encodes a viral vector. In some embodiments, the viral vector can be a retroviral vector, a lentiviral vector, an adenoviral vector, or an adeno-associated viral (AAV) vector. In a particular embodiment, the viral vector is a recombinant AAV vector.

In another aspect, the present disclosure provides a viral vector comprising an isolated polynucleotide, wherein the isolated polynucleotide comprises a nucleic acid sequence encoding a recombinant meganuclease described herein. In one embodiment, the viral vector is a retroviral vector, a lentiviral vector, an adenoviral vector, or an AAV vector. In a particular embodiment, the viral vector is a recombinant AAV vector.

In another aspect, the present disclosure provides a method for producing a genetically-modified eukaryotic cell comprising an exogenous sequence of interest inserted in a chromosome of the eukaryotic cell, the method comprising transfecting a eukaryotic cell with one or more nucleic acids including: (a) a nucleic acid sequence encoding a recombinant meganuclease described herein; and (b) a nucleic acid sequence comprising the sequence of interest; wherein the recombinant meganuclease produces a cleavage site in the chromosome at a recognition sequence comprising SEQ ID NO:2, SEQ ID NO:4, SEQ ID NO:6, or SEQ ID NO:8, and wherein the sequence of interest is inserted into the chromosome at the cleavage site.

In one embodiment of the method, cell-surface expression of beta-2 microglobulin is reduced when compared to a control cell.

In another embodiment of the method, the genetically-modified cell exhibits reduced alloreactivity and/or reduced allogenicity when introduced into a host or when administered to a subject, as compared to a control cell.

In another embodiment of the method, the nucleic acid comprising the sequence of interest further comprises sequences homologous to sequences flanking the cleavage site, and the sequence of interest is inserted at the cleavage site by homologous recombination.

In another embodiment of the method, the nucleic acid comprising the sequence of interest lacks substantial homology to the cleavage site, and the sequence of interest is inserted into the chromosome by non-homologous end-joining.

In another embodiment of the method, the sequence of interest encodes a chimeric antigen receptor. In another embodiment of the method, the sequence of interest encodes an exogenous T cell receptor.

In another embodiment of the method, at least the nucleic acid comprising the sequence of interest is introduced into the eukaryotic cell by a viral vector. In another embodiment of the method, the nucleic acid sequence encoding the recombinant meganuclease and the nucleic acid sequence encoding the sequence of interest are introduced into the eukaryotic cell by the same viral vector or, alternatively, by separate viral vectors. In some embodiments of the method, the viral vector is a retroviral vector, a lentiviral vector, an adenoviral vector, or an AAV vector. In particular embodiments of the method, the viral vector is a recombinant AAV vector.

In another embodiment of the method, at least the nucleic acid encoding the sequence of interest is introduced into the eukaryotic cell using a single-stranded DNA template.

In another embodiment of the method, the eukaryotic cell is a human T cell, or a cell derived therefrom.

In another embodiment of the method, the eukaryotic cell has been genetically-modified to exhibit reduced cell-surface expression of an endogenous T cell receptor when compared to a control cell.

In another aspect, the method can further comprise: (a) transfecting the eukaryotic cell with a nucleic acid encoding an endonuclease which recognizes and cleaves a second recognition sequence; or (b) introducing into the eukaryotic cell an endonuclease which recognizes and cleaves a second recognition sequence; wherein the second recognition sequence is located in a gene encoding a component of an endogenous T cell receptor, and wherein the genetically-modified eukaryotic cell exhibits reduced cell-surface expression of beta-2 microglobulin and the endogenous T cell receptor when compared to a control cell.

In one such embodiment, the endonuclease is a recombinant meganuclease. In another such embodiment, the second recognition sequence is located in the human T cell receptor alpha constant region gene, as set forth in SEQ ID NO: 127. In another such embodiment, the second recognition sequence can comprise SEQ ID NO: 128, 129, or 130 *(i.e.,* the TRC 1-2, TRC 3-4, and TRC 5-6 recognition sequences, respectively).

In another such embodiment, the nucleic acid is a polycistronic mRNA which encodes both a recombinant meganuclease described herein and the endonuclease which recognizes and cleaves a second recognition sequence.

In another aspect, the present disclosure provides a method for producing a genetically-modified eukaryotic cell comprising an exogenous sequence of interest inserted in a chromosome of the eukaryotic cell, the method comprising: (a) introducing a recombinant meganuclease described herein into a eukaryotic cell; and (b) transfecting the eukaryotic cell with a nucleic acid comprising a sequence of interest; wherein the recombinant meganuclease produces a cleavage site in the chromosome at a recognition sequence comprising SEQ ID NO:2, SEQ ID NO:4, SEQ ID NO:6, or SEQ ID NO:8, and wherein the sequence of interest is inserted into the chromosome at the cleavage site.

In one embodiment of the method, cell-surface expression of beta-2 microglobulin is reduced when compared to a control cell.

In another embodiment of the method, the genetically-modified cell exhibits reduced alloreactivity and/or reduced allogenicity when introduced into a host or when administered to a subject, as compared to a control cell.

In another embodiment of the method, the nucleic acid comprising the sequence of interest further comprises sequences homologous to sequences flanking the cleavage site, and the sequence of interest is inserted at the cleavage site by homologous recombination.

In another embodiment of the method, the nucleic acid comprising the sequence of interest lacks substantial homology to the cleavage site, and the sequence of interest is inserted into the chromosome by non-homologous end-joining.

In another embodiment of the method, the sequence of interest encodes a chimeric antigen receptor. In another embodiment of the method, the sequence of interest encodes an exogenous T cell receptor.

In another embodiment of the method, the nucleic acid comprising the sequence of interest is introduced into the eukaryotic cell by a viral vector. In some embodiments of the method, the viral vector is a retroviral vector, a lentiviral vector, an adenoviral vector, or an AAV vector. In particular embodiments, the viral vector is a recombinant AAV vector.

In another embodiment of the method, the nucleic acid encoding the sequence of interest is introduced into the eukaryotic cell using a single-stranded DNA template.

In another embodiment of the method, the eukaryotic cell is a human T cell, or a cell derived therefrom.

In another embodiment of the method, the eukaryotic cell has been genetically-modified to exhibit reduced cell-surface expression of an endogenous T cell receptor when compared to a control cell.

In another embodiment, the method can further comprise: (a) transfecting the eukaryotic cell with a nucleic acid encoding an endonuclease which recognizes and cleaves a second recognition sequence; or (b) introducing into the eukaryotic cell an endonuclease which recognizes and cleaves a second recognition sequence; wherein the second recognition sequence is located in a gene encoding a component of an endogenous T cell receptor, and wherein the genetically-modified eukaryotic cell exhibits reduced cell-surface expression of beta-2 microglobulin and the endogenous T cell receptor when compared to a control cell.

In one such embodiment, the endonuclease is a recombinant meganuclease. In another such embodiment, the second recognition sequence is located in the human T cell receptor alpha constant region gene, as set forth in SEQ ID NO: 127. In another such embodiment, the second recognition sequence can comprise SEQ ID NO: 128, 129, or 130 *(i.e.,* the TRC 1-2, TRC 3-4, and TRC 5-6 recognition sequences, respectively).

In another aspect, the present disclosure provides a method for producing a genetically-modified eukaryotic cell by disrupting a target sequence in a chromosome of the eukaryotic cell, the method comprising: transfecting the eukaryotic cell with a nucleic acid encoding a recombinant meganuclease described herein or, alternatively, introducing a recombinant meganuclease described herein into the eukaryotic cell; wherein the meganuclease produces a cleavage site in the chromosome at a recognition sequence comprising SEQ ID NO:2, SEQ ID NO:4, SEQ ID NO:6, or SEQ ID NO:8, and wherein the target sequence is disrupted by non-homologous end-joining at the cleavage site. In such a method, the genetically-modified eukaryotic cell exhibits reduced cell-surface expression of beta-2 microglobulin when compared to a control cell.

In one embodiment of the method, the genetically-modified cell exhibits reduced alloreactivity and/or reduced allogenicity when introduced into a host or when administered to a subject, as compared to a control cell.

In another embodiment of the method, the eukaryotic cell is a human T cell, or a cell derived therefrom.

In another embodiment of the method, the eukaryotic cell has been genetically-modified to exhibit reduced cell-surface expression of an endogenous T cell receptor when compared to a control cell.

In another embodiment the method further comprises: (a) transfecting the eukaryotic cell with a nucleic acid encoding an endonuclease which recognizes and cleaves a second recognition sequence; or (b) introducing into the eukaryotic cell an endonuclease which recognizes and cleaves a second recognition sequence. In such an embodiment, the endonuclease is a recombinant meganuclease. In another such embodiment, the second recognition sequence is located in a gene encoding a component of an endogenous T cell receptor, and the genetically-modified eukaryotic cell exhibits reduced cell-surface expression of both beta-2 microglobulin and the endogenous T cell receptor when compared to a control cell.

In such an embodiment, the nucleic acid is a polycistronic mRNA which encodes both a recombinant meganuclease described herein and the endonuclease which recognizes and cleaves a second recognition sequence.

In another embodiment of the method, the eukaryotic cell expresses a cell-surface chimeric antigen receptor. In another embodiment of the method, the sequence of interest encodes an exogenous T cell receptor.

In another embodiment, the method further comprises transfecting the eukaryotic cell with a nucleic acid comprising an exogenous sequence of interest. In such an embodiment, the nucleic acid comprising the exogenous sequence of interest further comprises sequences homologous to sequences flanking the second cleavage site, and the sequence of interest is inserted at the second cleavage site by homologous recombination. In another such embodiment, the nucleic acid comprising the sequence of interest lacks substantial homology to the cleavage site, and the sequence of interest is inserted into the chromosome by non-homologous end-joining.

In another such embodiment, the exogenous sequence of interest encodes a chimeric antigen receptor. In another such embodiment, the exogenous of interest encodes an exogenous T cell receptor.

In another such embodiment, at least the nucleic acid comprising the exogenous sequence of interest is introduced into the eukaryotic cell by a viral vector. In another such embodiment, the nucleic acid sequence encoding the endonuclease and the nucleic acid sequence encoding the sequence of interest are introduced into the eukaryotic cell by the same viral vector or, alternatively, by separate viral vectors. In some embodiments, the viral vector is a retroviral vector, a lentiviral vector, an adenoviral vector, or an AAV vector. In a particular embodiment, the viral vector is a recombinant AAV vector.

In another such embodiment, the nucleic acid encoding the sequence of interest is introduced into the eukaryotic cell using a single-stranded DNA template.

In another aspect, the present disclosure provides a method of producing a genetically-modified non-human organism comprising: producing a genetically-modified non-human eukaryotic cell according to the methods described herein; and (b) growing the genetically-modified non-human eukaryotic cell to produce the genetically-modified non-human organism.

In one embodiment of the method, the non-human eukaryotic cell is selected from the group consisting of a gamete, a zygote, a blastocyst cell, an embryonic stem cell, and a protoplast cell.

In another aspect, the present disclosure provides a genetically-modified cell comprising in its genome a modified human beta-2 microglobulin gene, wherein the modified beta-2 microglobulin gene comprises from 5' to 3': (a) a 5' region of the human beta-2 microglobulin gene; (b) an exogenous polynucleotide; and (c) a 3' region of the human beta-2 microglobulin gene. The genetically-modified cell can be a genetically-modified human T cell or a genetically-modified cell derived from a human T cell. Further, the genetically-modified cell can have reduced cell-surface expression of beta-2 microglobulin when compared to an unmodified control cell.

In one embodiment, the exogenous polynucleotide comprises a nucleic acid sequence encoding a chimeric antigen receptor, wherein the chimeric antigen receptor comprises an extracellular ligand-binding domain and one or more intracellular signaling domains.

In another embodiment, the exogenous polynucleotide is inserted into the beta-2 microglobulin gene at a position within a recognition sequence comprising SEQ ID NO:2 *(i.e.,* the B2M 13-14 recognition sequence), SEQ ID NO:4 *(i.e.,* the B2M 5-6 recognition sequence), SEQ ID NO:6 *(i.e.,* the B2M 7-8 recognition sequence), or SEQ ID NO:8 *(i.e.,* the B2M 11-12 recognition sequence).

In another aspect, the present disclosure provides a genetically-modified eukaryotic cell described herein for use as a medicament. The present disclosure further provides the use of a genetically-modified eukaryotic cell described herein in the manufacture of a medicament for treating a disease in a subject in need thereof. In one such embodiment, the medicament is useful in the treatment of cancer. In another embodiment, the medicament is useful in the treatment of cancer using immunotherapy.

In another aspect, the present disclosure provides a population of genetically-modified eukaryotic cells. In one embodiment, the population comprises at least 1×10⁶, 2×10⁶, 5×10⁶, 1×10⁹, 2×10⁹, 5×10⁹, or more, genetically-modified eukaryotic cells.

In another embodiment, at least 80%, at least 85%, at least 90%, at least 95%, or more of the genetically-modified eukaryotic cells in the population exhibit reduced cell-surface expression of an endogenous T cell receptor when compared to a control cell, and at least 80%, at least 85%, at least 90%, at least 95%, or more of the genetically-modified eukaryotic cells exhibit reduced cell-surface expression of beta-2 microglobulin when compared to a control cell.

In another embodiment, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or more of the eukaryotic cells express a chimeric antigen receptor.

In another embodiment, the genetically-modified eukaryotic cells are genetically-modified T cells, or cells derived therefrom.

In another aspect, the present disclosure provides a pharmaceutical composition comprising a genetically-modified eukaryotic cell, or a population of genetically-modified eukaryotic cells, described herein and a pharmaceutically acceptable carrier. In one embodiment, the genetically-modified eukaryotic cells are genetically-modified T cells, or cells derived therefrom. In particular embodiments, the genetically-modified T cells express a chimeric antigen receptor and exhibit reduced cell surface expression of beta-2 microglobulin and an endogenous T cell receptor. Such pharmaceutical compositions of the disclosure are suitable for use as immunotherapy for the treatment of cancer.

In further aspects, the genetically-modified T cells exhibit reduced alloreactivity and/or reduced allogenicity when introduced into a host or when administered to a subject, as compared to control cells.

In another aspect, the present disclosure provides a method for treating cancer in a subject in need thereof, the method comprising administering to the subject a pharmaceutical composition described herein. Such a method represents immunotherapy for the treatment of cancer.

In some embodiments, a chimeric antigen receptor disclosed herein comprises at least an extracellular ligand-binding domain or moiety and an intracellular domain that comprises one or more signaling domains and/or co-stimulatory domains. In some embodiments, the extracellular ligand-binding domain or moiety is in the form of a single-chain variable fragment (scFv) derived from a monoclonal antibody, which provides specificity for a particular epitope or antigen (e.g., an epitope or antigen preferentially present on the surface of a cell, such as a cancer cell or other disease-causing cell or particle). The scFv is attached via a linker sequence. The extracellular ligand-binding domain is specific for any antigen or epitope of interest. In some embodiments, the scFv is humanized or fully human. The extracellular domain of a chimeric antigen receptor can also comprise an autoantigen (see, Payne et al. (2016) Science, Vol. 353 (6295): 179-184), which is recognized by autoantigen-specific B cell receptors on B lymphocytes, thus directing T cells to specifically target and kill autoreactive B lymphocytes in antibody-mediated autoimmune diseases. Such CARs are referred to as chimeric autoantibody receptors (CAARs), and their use is encompassed by the present disclosure.

The foregoing and other aspects and embodiments of the present disclosure can be more fully understood by reference to the following detailed description and claims. Certain features of the disclosure, which are, for clarity, described in the context of separate embodiments, may also be provided in combination in a single embodiment. All combinations of the embodiments are specifically embraced by the present disclosure and are disclosed herein just as if each and every combination was individually and explicitly disclosed. Conversely, various features of the disclosure, which are, for brevity, described in the context of a single embodiment, may also be provided separately or in any suitable sub-combination. All sub-combinations of features listed in the embodiments are also specifically embraced by the present disclosure and are disclosed herein just as if each and every such sub-combination was individually and explicitly disclosed herein. Embodiments of each aspect of the present disclosure disclosed herein apply to each other aspect of the disclosure *mutatis mutandis.*

### BRIEF DESCRIPTION OF THE FIGURES

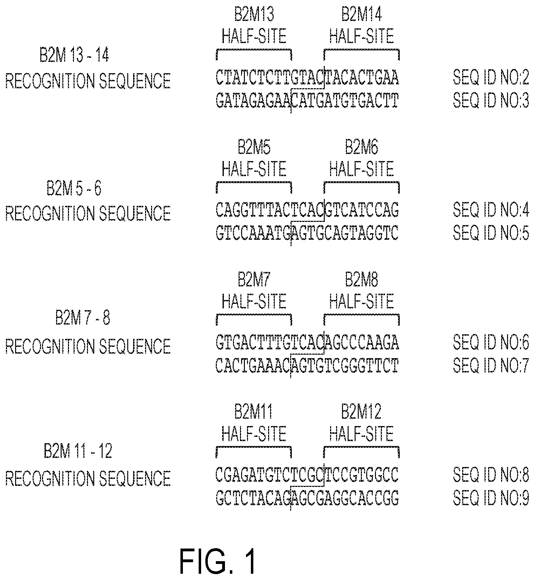

Fig. 1. B2M recognition sequences in the human beta-2 microglobulin gene. Each recognition sequence targeted by a recombinant meganuclease of the disclosure comprises two recognition half-sites. Each recognition half-site comprises 9 base pairs, separated by a 4 base pair central sequence. The B2M 13-14 recognition sequence (SEQ ID NO:2) spans nucleotides 9115-9136 of the human beta-2 microglobulin gene (SEQ ID NO:1), and comprises two recognition half-sites referred to as B2M13 and B2M14. The B2M 5-6 recognition sequence (SEQ ID NO:4) spans nucleotides 8951-8972 of the human beta-2 microglobulin gene (SEQ ID NO:1), and comprises two recognition half-sites referred to as B2M5 and B2M6. The B2M 7-8 recognition sequence (SEQ ID NO:6) spans nucleotides 9182-9203 of the human beta-2 microglobulin gene (SEQ ID NO:1), and comprises two recognition half-sites referred to as B2M7 and B2M8. The B2M 11-12 recognition sequence (SEQ ID NO:8) spans nucleotides 5057-5078 of the human beta-2 microglobulin gene (SEQ ID NO:1), and comprises two recognition half-sites referred to as B2M11 and B2M12.

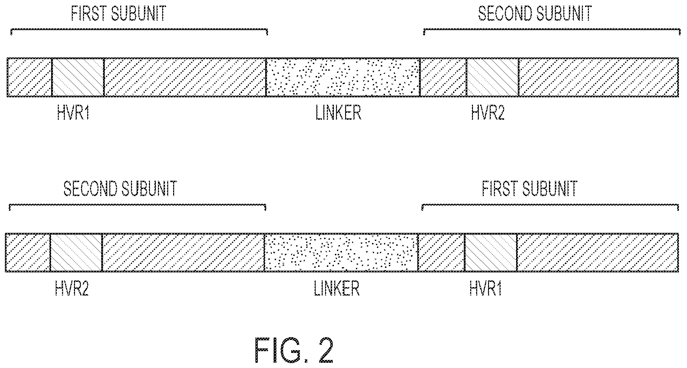

Fig. 2. The recombinant meganucleases of the disclosure comprise two subunits, wherein the first subunit comprising the HVR1 region binds to a first recognition half-site *(e.g.,* B2M13, B2M5, B2M7, or B2M11) and the second subunit comprising the HVR2 region binds to a second recognition half-site (e.g., B2M14, B2M6, B2M8, or B2M12). In embodiments where the recombinant meganuclease is a single-chain meganuclease, the first subunit comprising the HVR1 region can be positioned as either the N-terminal or C-terminal subunit. Likewise, the second subunit comprising the HVR2 region can be positioned as either the N-terminal or C-terminal subunit.

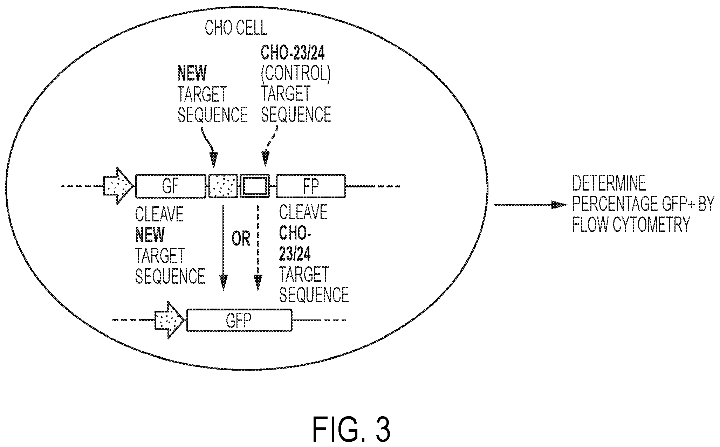

Fig. 3. Schematic of reporter assay in CHO cells for evaluating recombinant meganucleases targeting recognition sequences found in the beta-2 microglobulin gene (SEQ ID NO:1). For the recombinant meganucleases described herein, a CHO cell line was produced in which a reporter cassette was integrated stably into the genome of the cell. The reporter cassette comprised, in 5' to 3' order: an SV40 Early Promoter; the 5' 2/3 of the GFP gene; the recognition sequence for an engineered meganuclease of the disclosure (e.g., the B2M 13-14 recognition sequence, the B2M 5-6 recognition sequence, the B2M 7-8 recognition sequence, or the B2M 11-12 recognition sequence); the recognition sequence for the CHO-23/24 meganuclease (WO/2012/167192); and the 3' 2/3 of the GFP gene. Cells stably transfected with this cassette did not express GFP in the absence of a DNA break-inducing agent. Meganucleases were introduced by transduction of plasmid DNA or mRNA encoding each meganuclease. When a DNA break was induced at either of the meganuclease recognition sequences, the duplicated regions of the GFP gene recombined with one another to produce a functional GFP gene. The percentage of GFP-expressing cells could then be determined by flow cytometry as an indirect measure of the frequency of genome cleavage by the meganucleases.

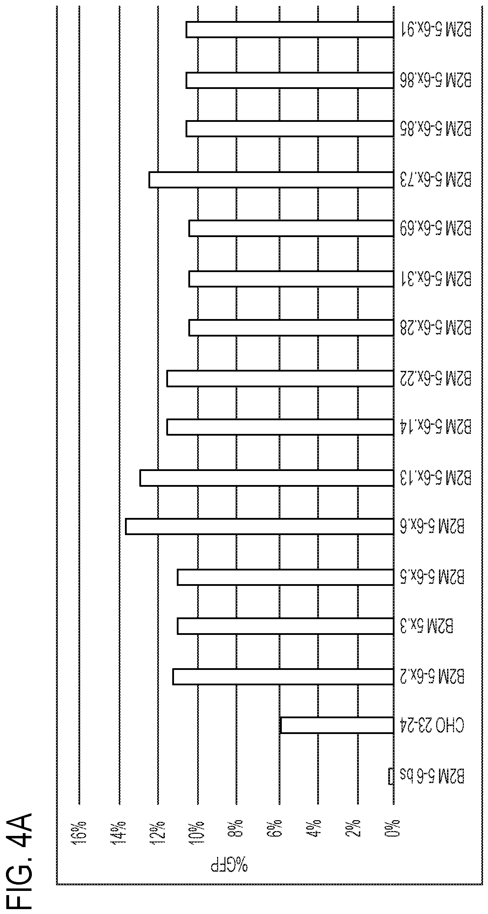

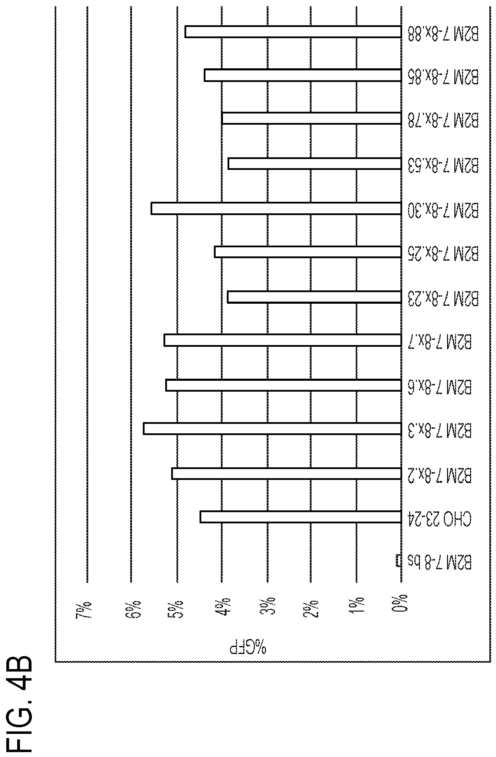

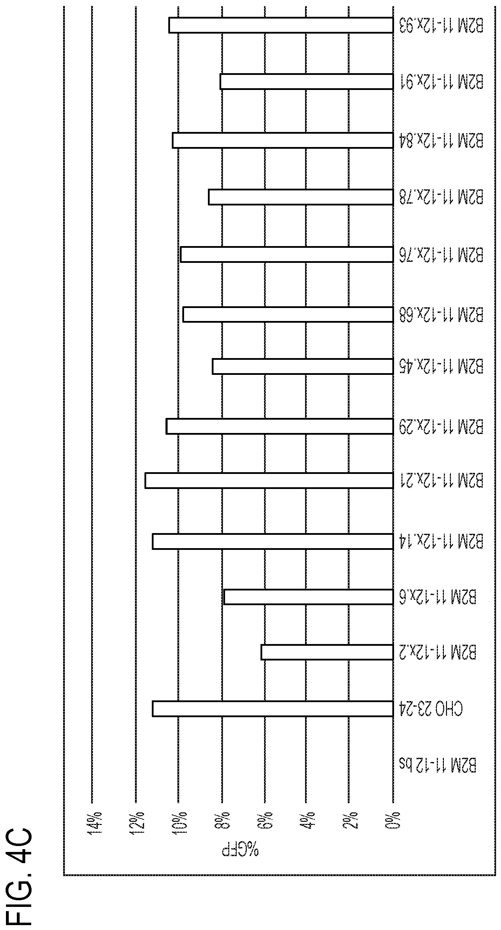

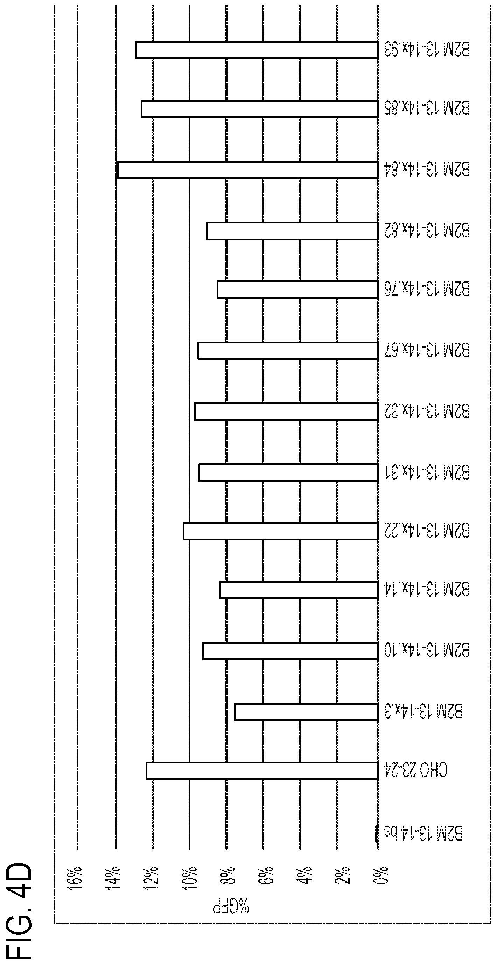

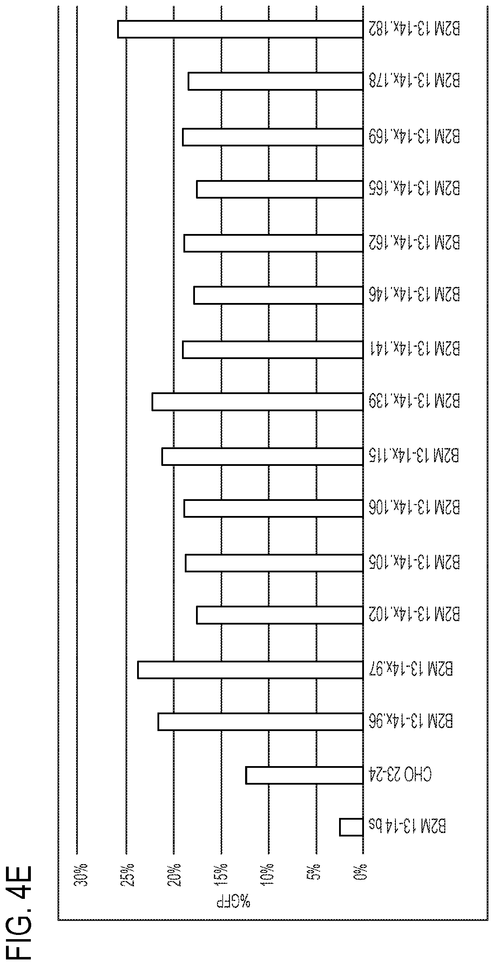

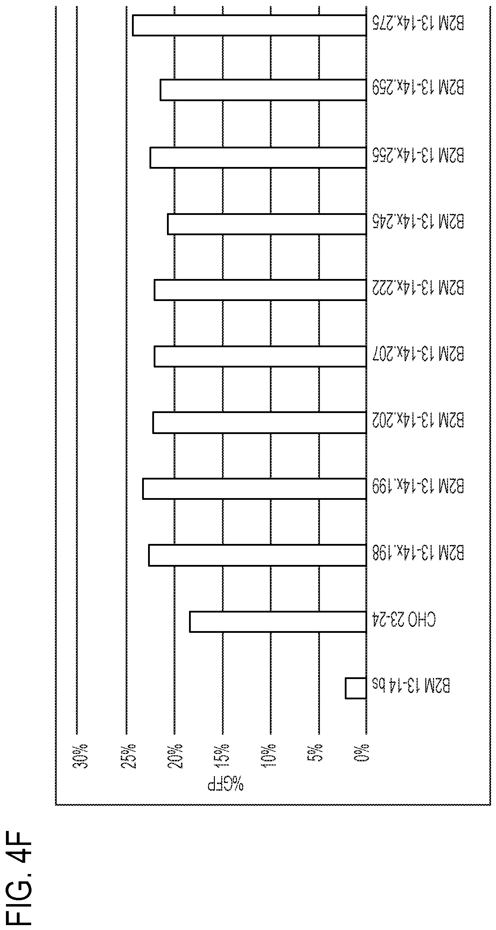

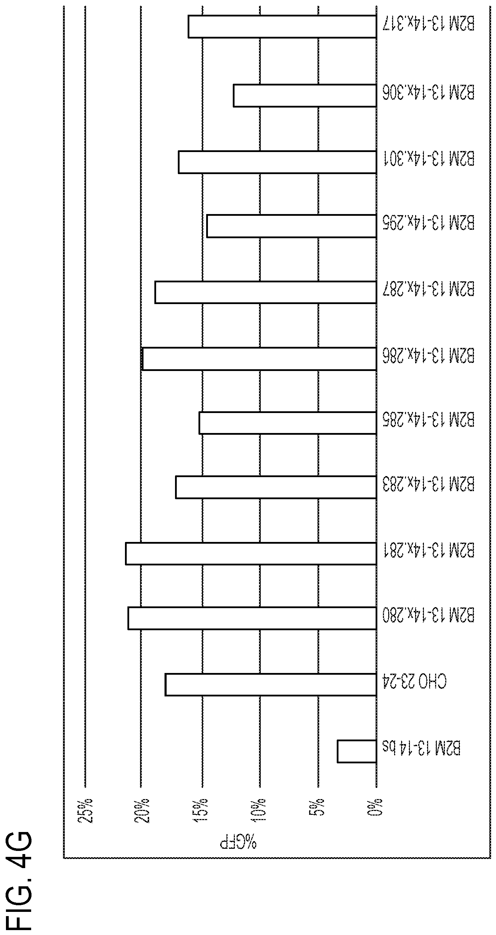

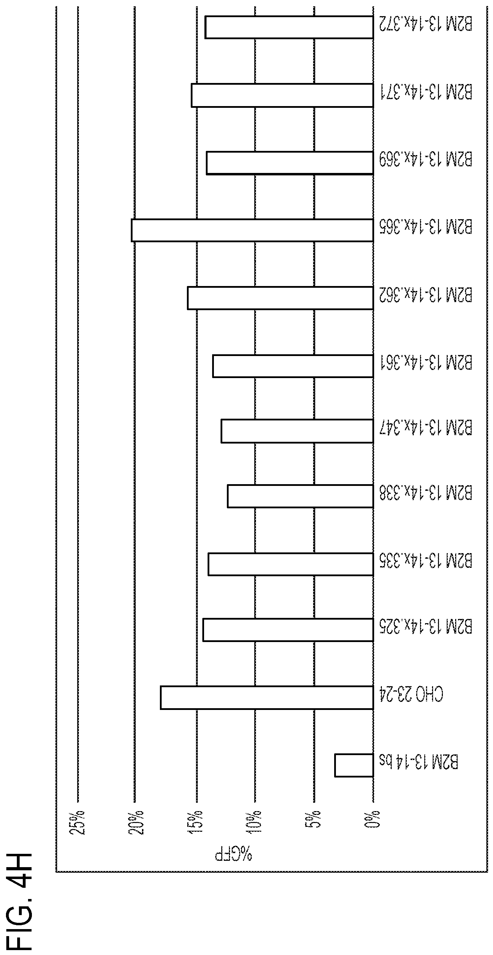

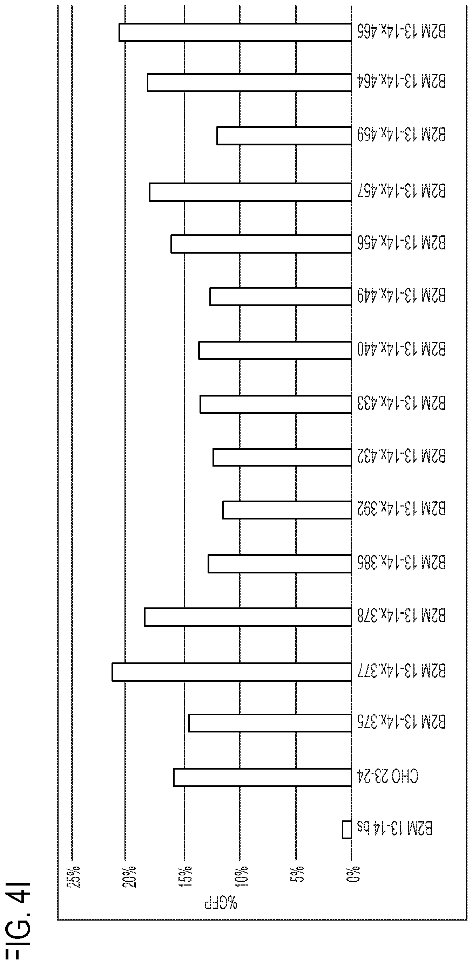

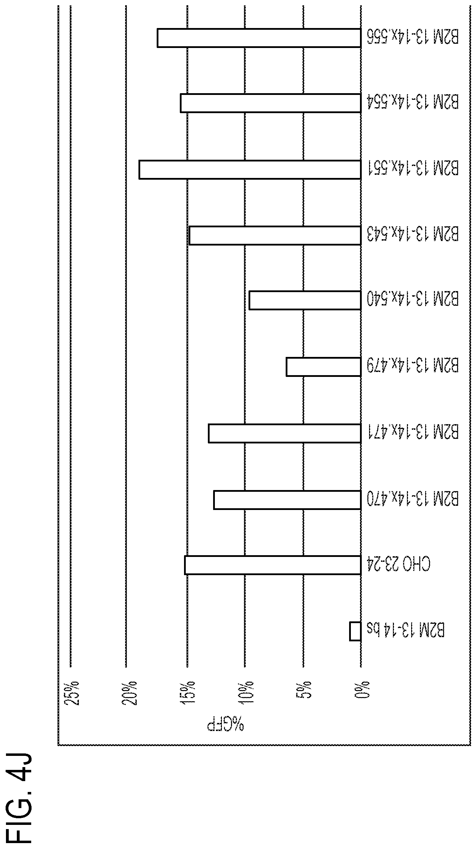

Figs. 4A-4J. Efficiency of recombinant meganucleases for recognizing and cleaving recognition sequences in the human beta-2 microglobulin gene (SEQ ID NO:1) in a CHO cell reporter assay. Each of the recombinant meganucleases set forth in SEQ ID NOs: 12-126 were engineered to target the B2M 13-14 recognition sequence (SEQ ID NO:2), the B2M 5-6 recognition sequence (SEQ ID NO:4), the B2M 7-8 recognition sequence (SEQ ID NO:6), or the B2M 11-12 recognition sequence (SEQ ID NO:8), and were screened for efficacy in the CHO cell reporter assay. The results shown provide the percentage of GFP-expressing cells observed in each assay, which indicates the efficacy of each meganuclease for cleaving a beta-2 microglobulin target recognition sequence or the CHO-23/24 recognition sequence. A negative control (B2M bs) was further included in each assay. A) Meganucleases targeting the B2M 5-6 recognition sequence. B) Meganucleases targeting the B2M 7-8 recognition sequence. C) Meganucleases targeting the B2M 11-12 recognition sequence. D)-J) Meganucleases targeting the B2M 13-14 recognition sequence.

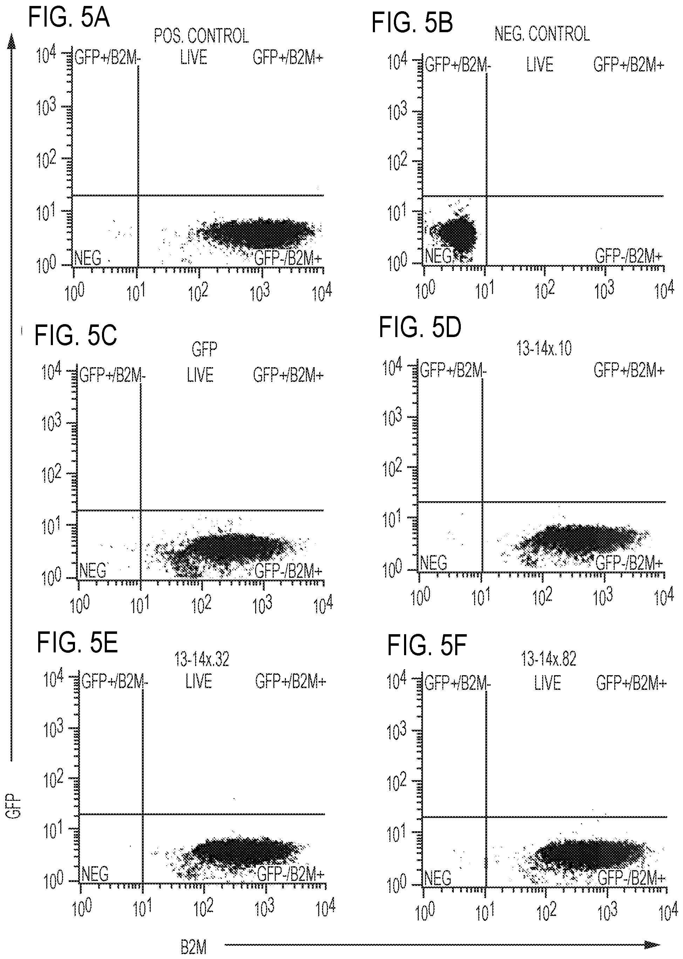

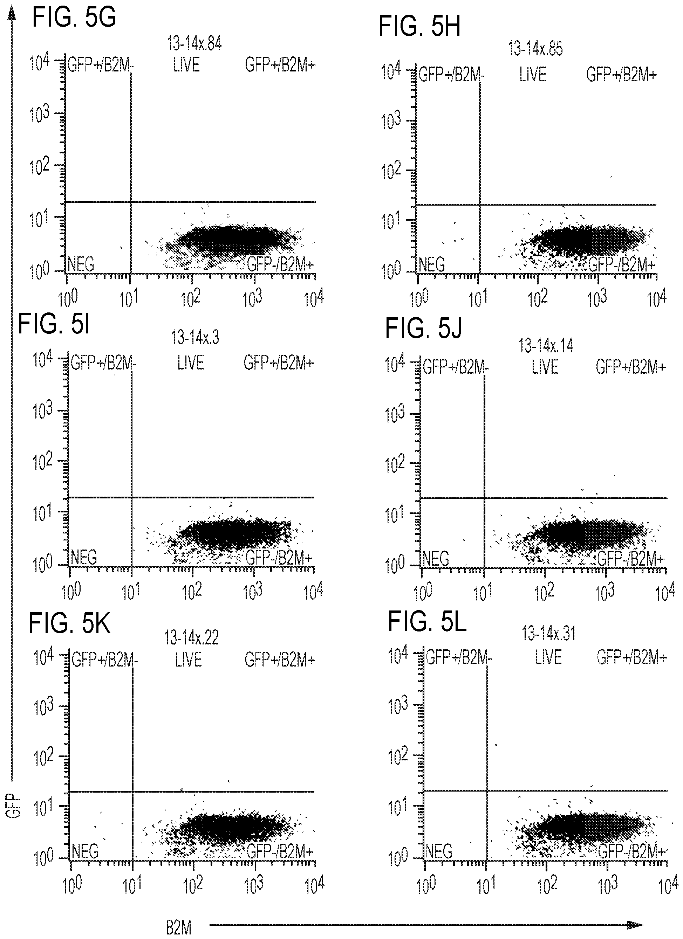

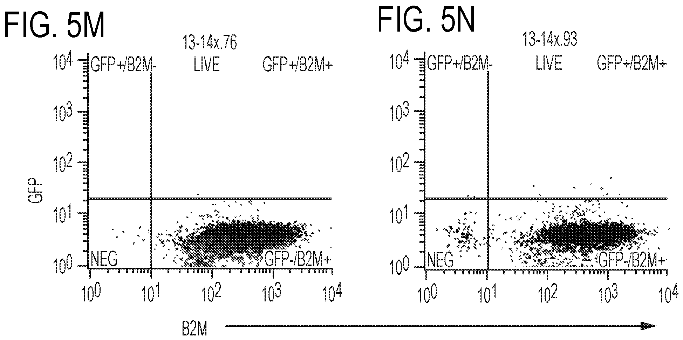

Figs. 5A-5N. Efficiency of recombinant B2M 13-14 meganucleases for inhibiting cell-surface expression of beta-2 microglobulin in human T cells. 5A-5N) Donor CD3⁺ human T cells were stimulated with anti-CD3 and anti-CD28 antibodies for 3 days, then electroporated with mRNA encoding a given B2M 13-14 meganuclease. As a positive control, cells were mock electroporated. In an additional control for electroporation efficiency, cells were electroporated with mRNA encoding GFP. At 3 days post-electroporation, cells were stained with an antibody recognizing beta-2 microglobulin and analyzed by flow cytometry.

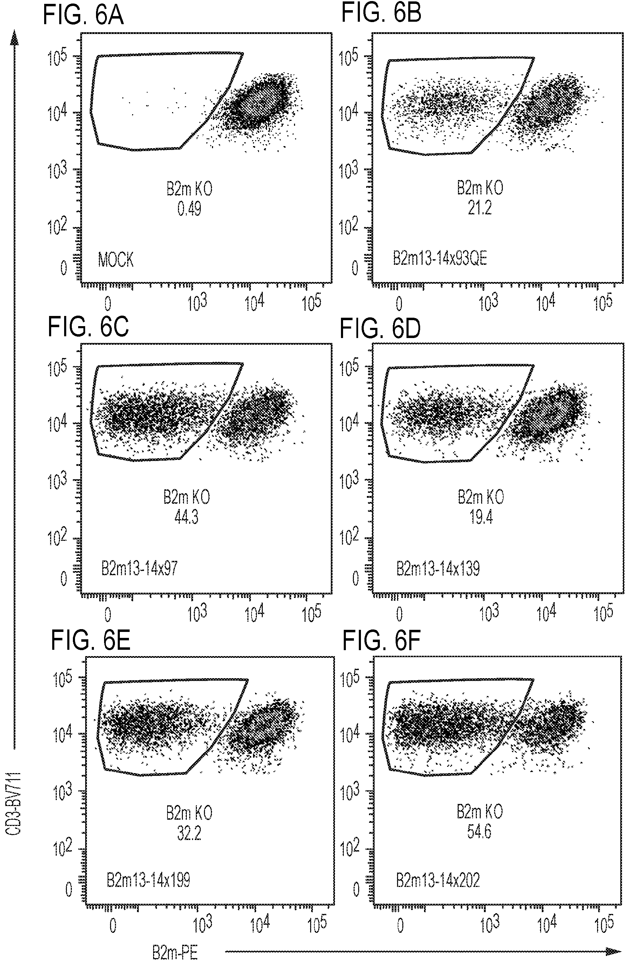

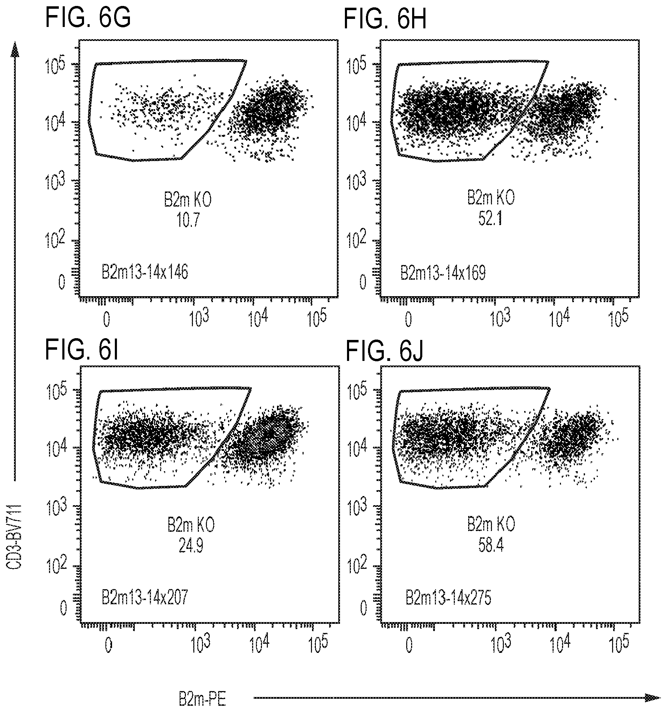

Figs. 6A-6J. Efficiency of recombinant B2M 13-14 meganucleases for inhibiting cell-surface expression of beta-2 microglobulin in human T cells. Additional B2M 13-14 meganucleases were engineered in which the first meganuclease subunit remained the same as in B2M 13-14x.93, but the second meganuclease subunit contained new amino acid substitutions at positions contacting the B2M 13-14 recognition sequence. 6A-6J) Donor CD3⁺ human T cells were stimulated with anti-CD3 and anti-CD28 antibodies for 3 days, then electroporated with mRNA encoding a given B2M 13-14 meganuclease (1µg) using the Amaxa 4D-Nucleofector (Lonza) according to the manufacturer's instructions. B2M 13-14x.93 QE was included to allow for comparison to previous variants. At 6 days post-electroporation, cells were stained with an antibody recognizing beta-2 microglobulin as well as an antibody recognizing CD3. Data is presented by flow cytometry plots.

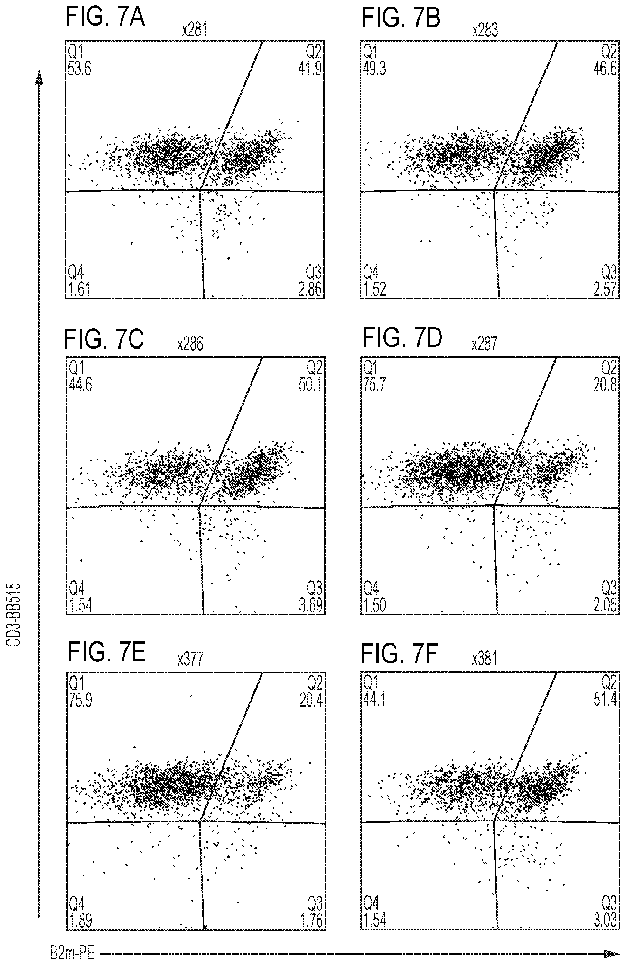

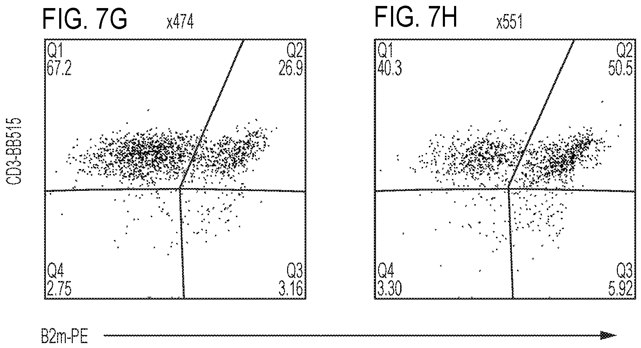

Figs. 7A-7H. Efficiency of recombinant B2M 13-14 meganucleases for inhibiting cell-surface expression of beta-2 microglobulin in human T cells. Further B2M 13-14 meganucleases were generated and evaluated for their ability to eliminate cell-surface expression of beta-2 microglobulin on human T cells. These nucleases were based on B2M 13-14x.169. Amino acid substitutions were made in the first meganuclease subunit to introduce alternative base contacts, while the second meganuclease subunit remained the same as in B2M 13-14x.169. 7A-7H) Donor CD3⁺ human T cells were stimulated with anti-CD3 and anti-CD28 antibodies for 3 days, then electroporated with mRNA encoding a given B2M 13-14 meganuclease using the Amaxa 4D-Nucleofector. B2M 13-14x.202 was included to allow for comparison to previous variants shown in Figs. 6A-6J. Cells were stained with an antibody recognizing beta-2 microglobulin as well as an antibody recognizing CD3. Flow cytometry data for the B2M 13-14 meganucleases that showed B2M knockout efficiency of >40% are shown.

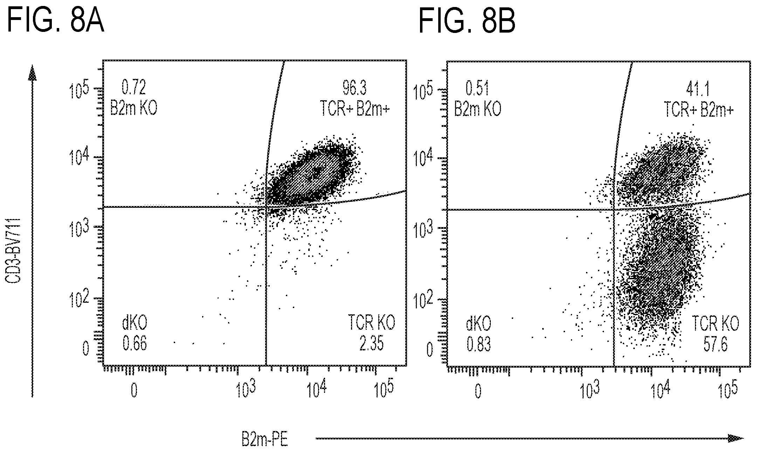

Figs. 8A-8D. Double knockout of beta-2 microglobulin and T cell receptor in human T cells by simultaneous nucleofection of two mRNAs encoding different meganucleases. Donor CD3⁺ human T cells were stimulated with anti-CD3 and anti-CD28 antibodies for 2 days, then co-electroporated with mRNA encoding B2M 13-14x.202 and mRNA encoding TRC 1-2x.87 EE using the Amaxa 4D-Nucleofector. As controls, human T cells were mock electroporated or electroporated with mRNA encoding a single meganuclease, either B2M 13-14x.202 or TRC 1-2x.87 EE. At 6 days post-electroporation, cells were stained with an antibody against CD3 and an antibody against B2M and analyzed by flow cytometry. A) Mock electroporated cells. B) TRC 1-2x.87 EE nucleofected cells. C) B2M 13-14x.202 nucleofected cells. D) Cells double nucleofected with B2M 13-14x.202 and TRC 1-2x.87 EE.

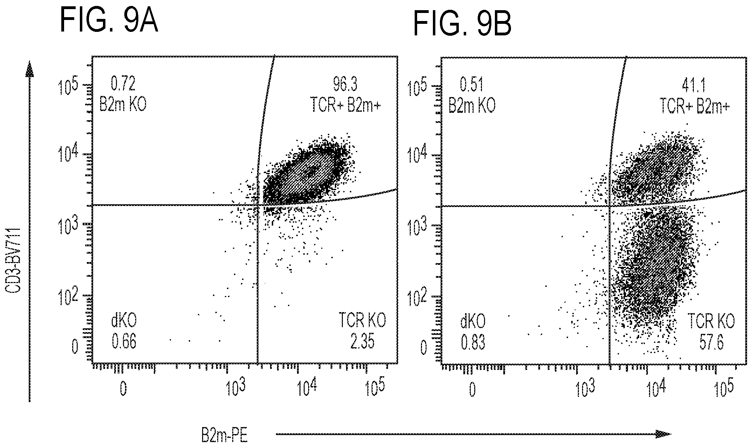

Figs. 9A-9D. Double knockout of beta-2 microglobulin and T cell receptor in human T cells by simultaneous nucleofection of two mRNAs encoding different meganucleases. Donor CD3⁺ human T cells were stimulated with anti-CD3 and anti-CD28 antibodies for 2 days, then co-electroporated with mRNA encoding B2M 13-14x.169 and mRNA encoding TRC 1-2x.87 EE using the Amaxa 4D-Nucleofector. As controls, human T cells were mock electroporated or electroporated with mRNA encoding a single meganuclease, either B2M 13-14x.169 or TRC 1-2x.87 EE. At 6 days post-electroporation, cells were stained with an antibody against CD3 and an antibody against B2M and analyzed by flow cytometry. A) Mock nucleofected cells. B) TRC 1-2x.87 EE nucleofected cells. C) B2M 13-14x.169 nucleofected cells. D) Cells double nucleofected with B2M 13-14x.169 and TRC 1-2x.87 EE.

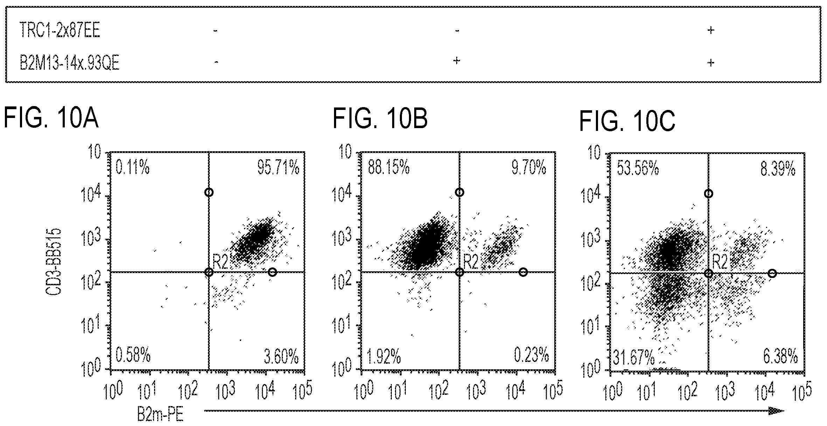

Figs. 10A-10C. Double knockout of beta-2 microglobulin and T cell receptor in human T cells by sequential nucleofection. Donor CD3⁺ human T cells were stimulated with anti-CD3 and anti-CD28 antibodies for 3 days, then electroporated with mRNA encoding B2M 13-14x.93 QE using the Amaxa 4D-Nucleofector. At 4 days post-electroporation, B2M-negative cells were enriched using a biotinylated anti-B2M antibody and a human biotin selection cocktail kit. The B2M-negative enriched cells were re-stimulated with anti-CD3 and anti-CD28 antibodies for 3 days, then electroporated with mRNA encoding TRC 1-2x.87 EE using the Amaxa 4D-Nucleofector. At 5 days post-electroporation, cells were stained with antibodies against B2M and TCR and analyzed by flow cytometry. A) Mock nucleofected cells. B) B2M 13-14x.93 QE nucleofected cells. C) Cells double nucleofected with B2M 13-14x.93 QE and TRC 1-2x.87 EE.

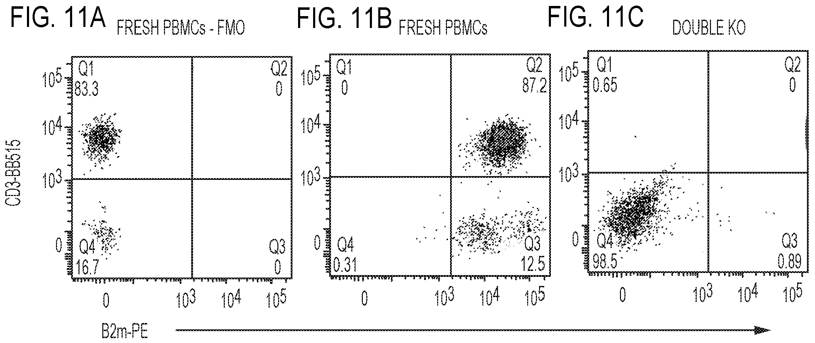

Figs. 11A-11C. Enrichment of a beta-2 microglobulin and T cell receptor double knockout population. Donor human peripheral blood mononuclear cells (PMBCs) were stimulated with anti-CD3 and anti-CD28 antibodies for 2 days, then electroporated with mRNA encoding B2M 13-14x.93 QE using the Amaxa 4D-Nucleofector. B2M-negative cells were enriched, re-stimulated with anti-CD3 and anti-CD28 antibodies for 3 days, and electroporated with mRNA encoding TRC 1-2x.87 EE. At 6 days post-electroporation, CD3-negative cells were enriched using a CD3 positive selection kit followed by another enrichment for B2M-negative cells using a biotinylated anti-B2M antibody and a biotin selection kit. Enriched cells were incubated 3 days in the presence of IL-2, IL-7 and IL-15, then stained with antibodies against B2M and CD3 and analyzed by flow cytometry. A) Non-nucleofected PBMCs stained with anti-CD3 antibody. B) Non-nucleofected PBMCs stained with anti-CD3 and anti-B2M antibodies. C) Enriched population of beta-2 microglobulin and T cell receptor double knock cells stained with anti-CD3 and anti-B2M antibodies.

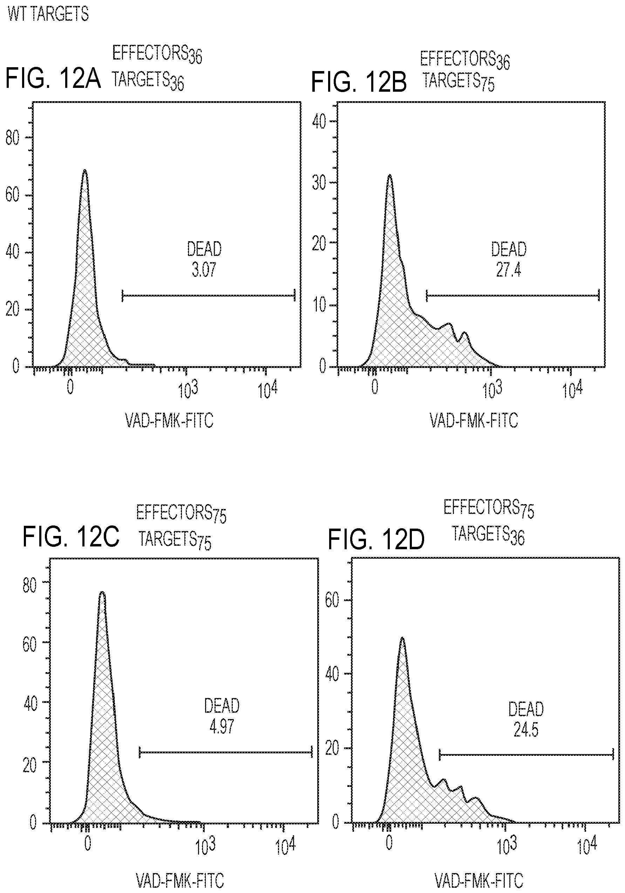

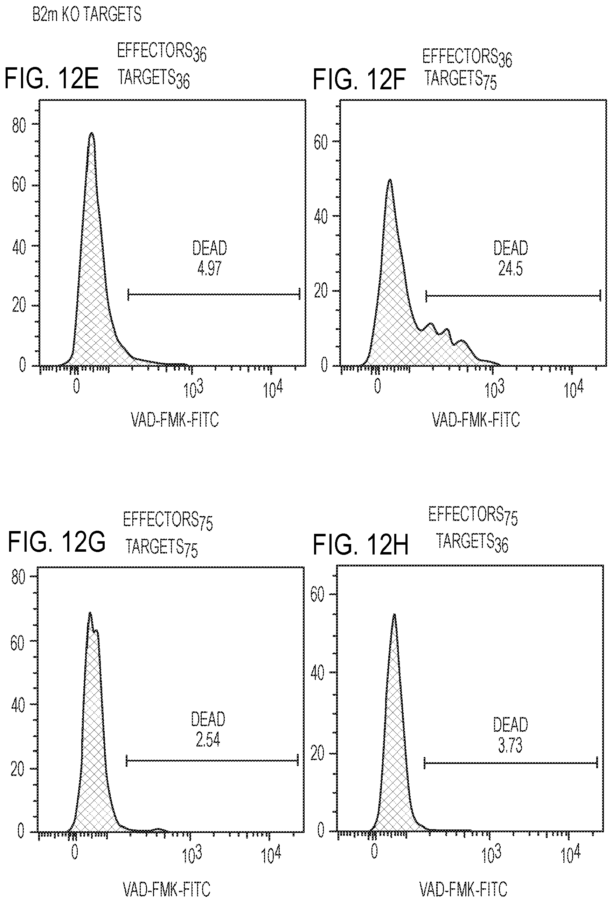

Figs. 12A-12H. Reduced allogenicity of B2M knockout T cells. The allogenicity of B2M knockout T cells was determined in a allogenic cytotoxicity assay using T cells from two donors (donor 36 and donor 75) and mismatched dendritic cells from another donor. Cytotoxicity was measured by VAD-FMK-FITC signal. A) Wild-type T cells; effector and donor cells from donor 36. B) Wild-type T cells; effector cells from donor 36, target cells from donor 75. C) Wild-type T cells; effector and donor cells from donor 75. D) Wild-type T cells; effector cells from donor 75, target cells from donor 36. E) B2M knockout T cells; effector and donor cells from donor 36. F) B2M knockout T cells; effector cells from donor 36, target cells from donor 75. G) B2M knockout T cells; effector and donor cells from donor 75. H) B2M knockout T cells; effector cells from donor 75, target cells from donor 36.

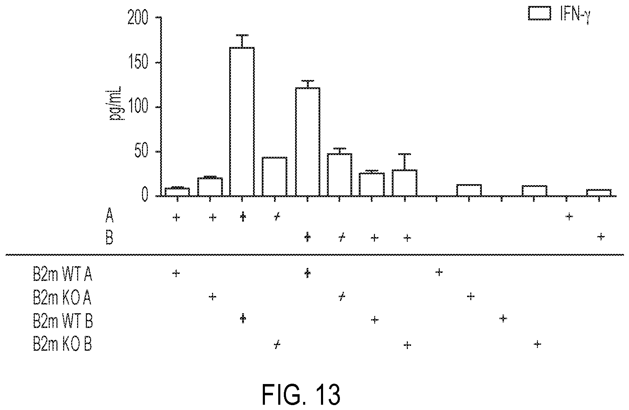

Fig. 13. Reduced allogenicity of B2M knockout T cells. Cytotoxicity determined by IFN-γ secretion as measured by ELISA.

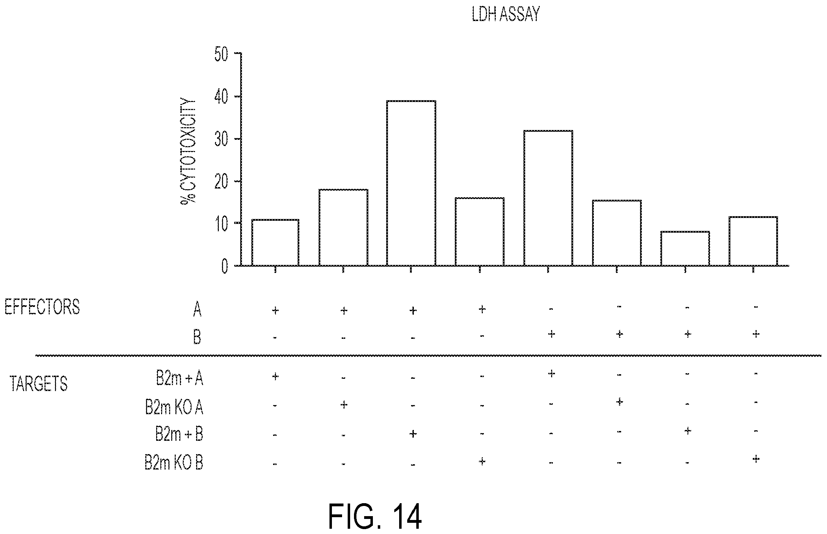

Fig. 14. Reduced allogenicity of B2M knockout T cells. Cytotoxicity determined by LDH secretion.

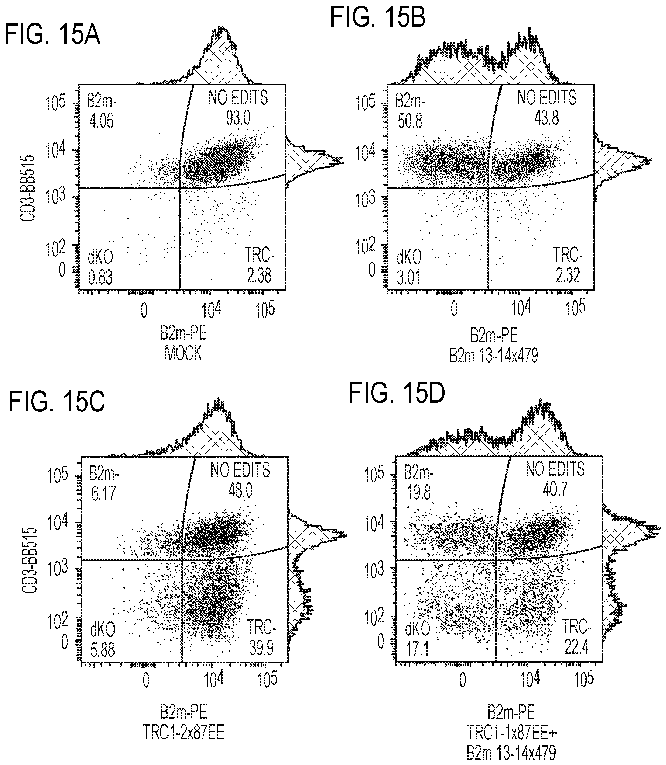

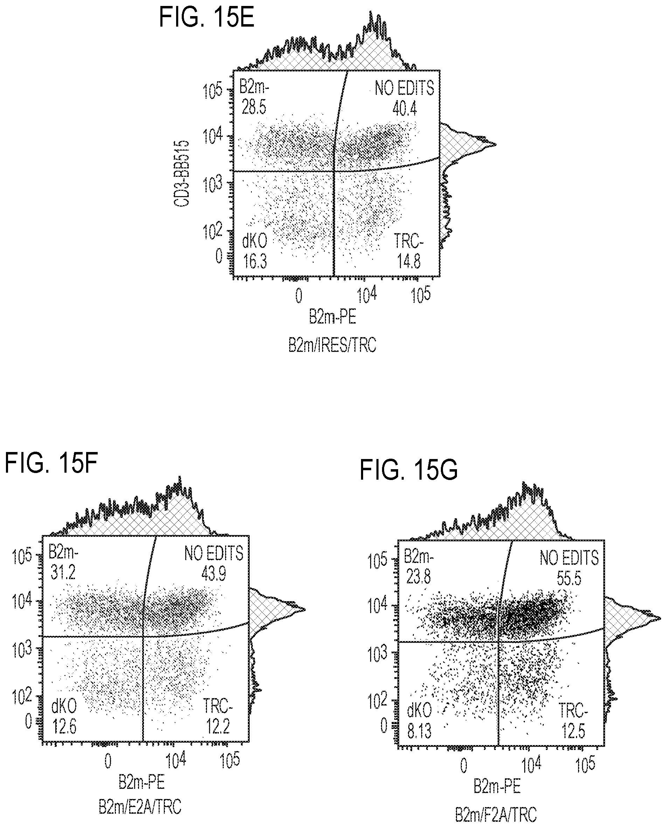

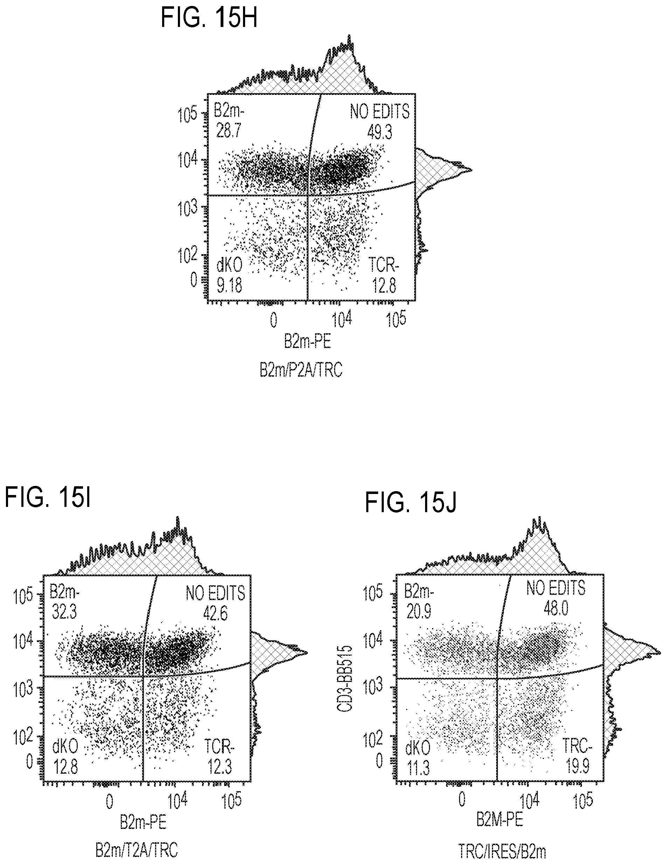

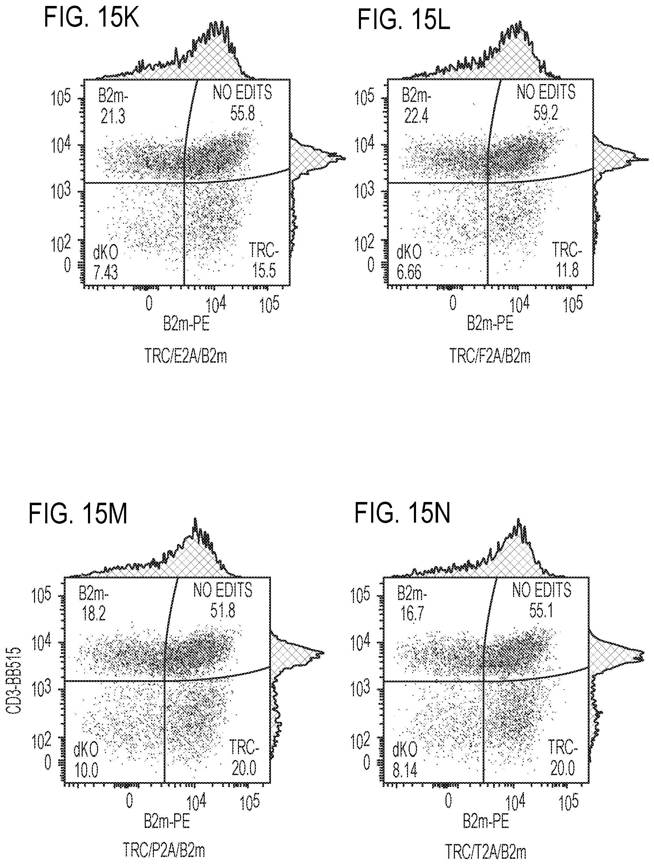

Figs. 15A-15N. Simultaneous knockout of TRC and B2M using bicistronic mRNA. Donor human T cells were electroporated with 1µg of TRC1-2x.87EE mRNA or B2M13-14x.479 RNA. An additional sample of cells was electroporated with 1 µg each of both individual nuclease mRNAs. As controls, human T cells were electroporated in the absence of mRNAs. At 3 and 7 days post-electroporation, cells that were electroporated with bicistronic mRNAs were stained with an antibody against CD3 (to determine TRC knockdown) and an antibody against B2M and analyzed by flow cytometry. A) Mock-treated. B) B2M 13-14x.479. C) TRC 1-2x.87 EE. D) TRC 1-2x.87 EE and B2M 13-14x.479. E) B2M-IRES-TRC. F) B2M-E2A-TRC. G) B2M-F2A-TRC. H) B2M-P2A-TRC. I) B2M-T2A-TRC. J) TRC-IRES-B2M. K) TRC-E2A-B2M. L) TRC-F2A-B2M. M) TRC-P2A-B2M. N) TRC-T2A-B2M.

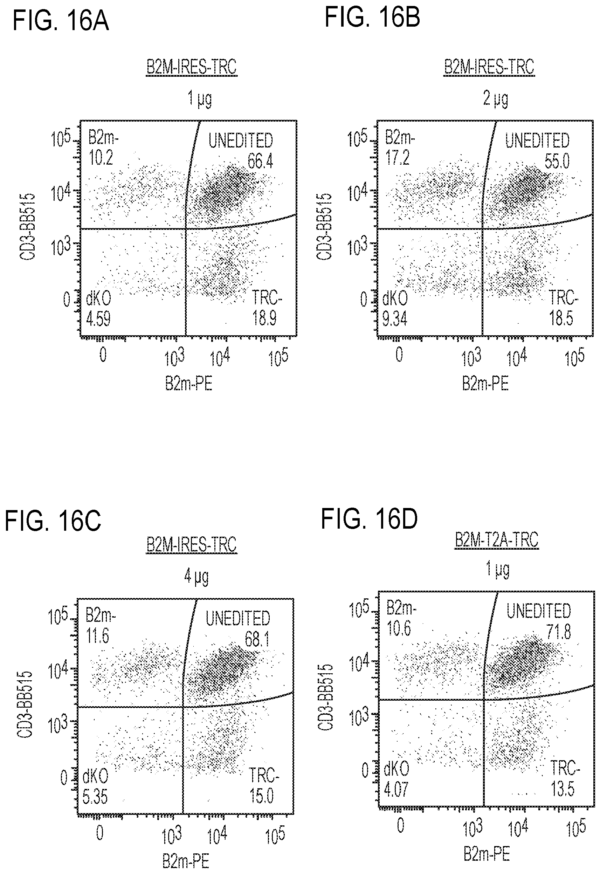

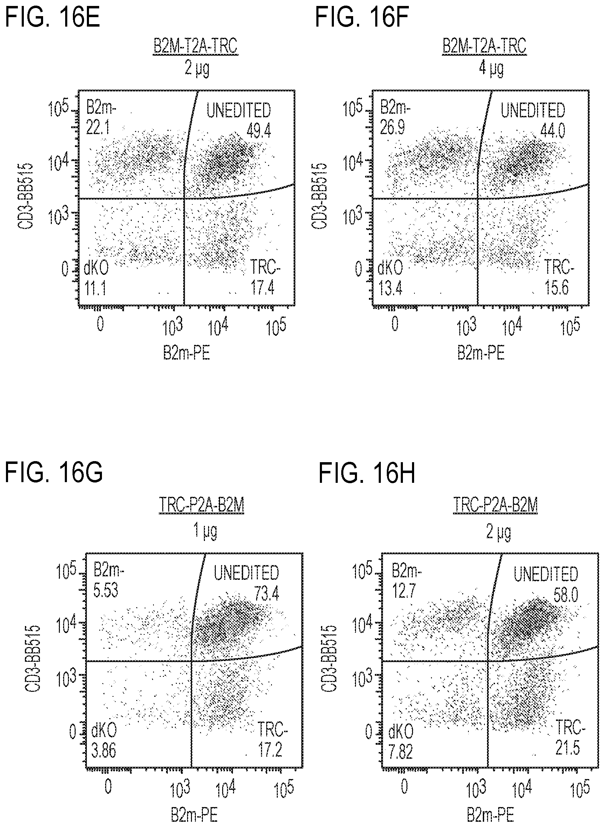

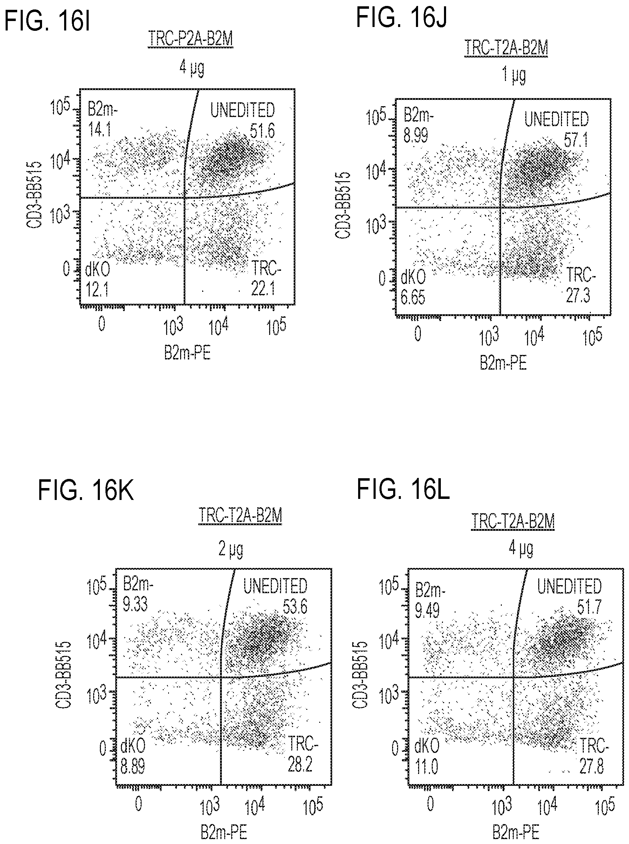

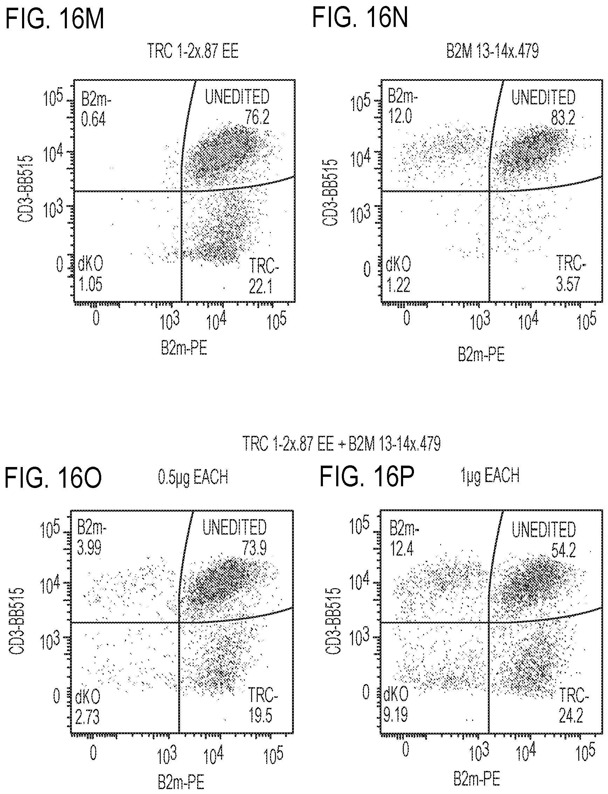

Figs. 16A-16P. Titration of bicistronic mRNA in T cells. B2M-IRES-TRC, B2M-T2A-TRC, TRC-P2A-B2M, or TRC-T2A-B2M mRNAs were introduced into donor human T cells at increasing concentrations, and the percent knockdown of cell-surface CD3 (indicated TRC knockdown) and B2M was determined. For comparison, donor human T cells were electroporated with 1µg of TRC1-2x.87EE or 1µg of B2M13-14x.479. In addition, donor human T cells were electroporated with both nucleases encoded on separate RNA molecules, using doses of 0.5µg of each nuclease or 1µg of each nuclease. As controls, human T cells were electroporated with no RNA. At 7 days post-electroporation, cells were enumerated and viability was assessed using trypan blue. Cells were stained with an antibody against CD3 (to determine TRC knockdown) and an antibody against B2M and analyzed by flow cytometry, as well as Ghost Dye 780 to exclude dead cells from analysis. A) B2MIRES-TRC 1 µg. B) B2M-IRES-TRC 2 µg. C) B2M-IRES-TRC 4 µg. D) B2M-T2A-TRC 1 µg. E) B2M-T2A-TRC 2 µg. F) B2M-T2A-TRC 4 µg. G) TRC-P2A-B2M 1 µg. H) TRC-P2A-B2M 2 µg. I) TRC-P2A-B2M 4 µg. J) TRC-T2A-B2M 1 µg. K) TRC-T2A-B2M 2 µg. L) TRC-T2A-B2M 4 µg. M) TRC 1-2x.87 EE. N) B2M 13-14x.479. O) TRC 1-2x.87 EE 0.5 µg and B2M 13-14x.479 0.5 µg. P) TRC 1-2x.87 EE 1.0 µg and B2M 13-14x.479 1.0 µg.

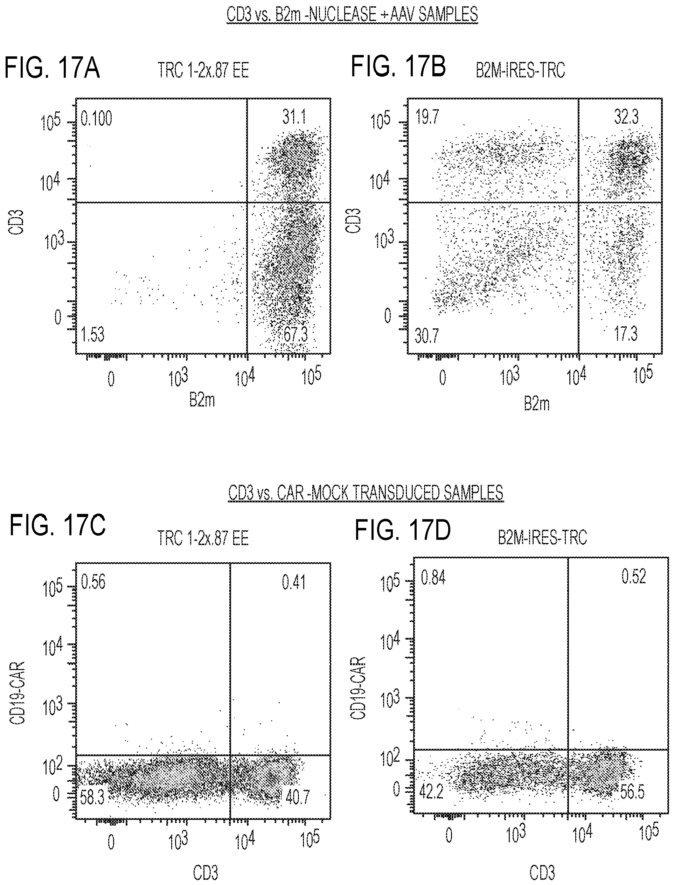

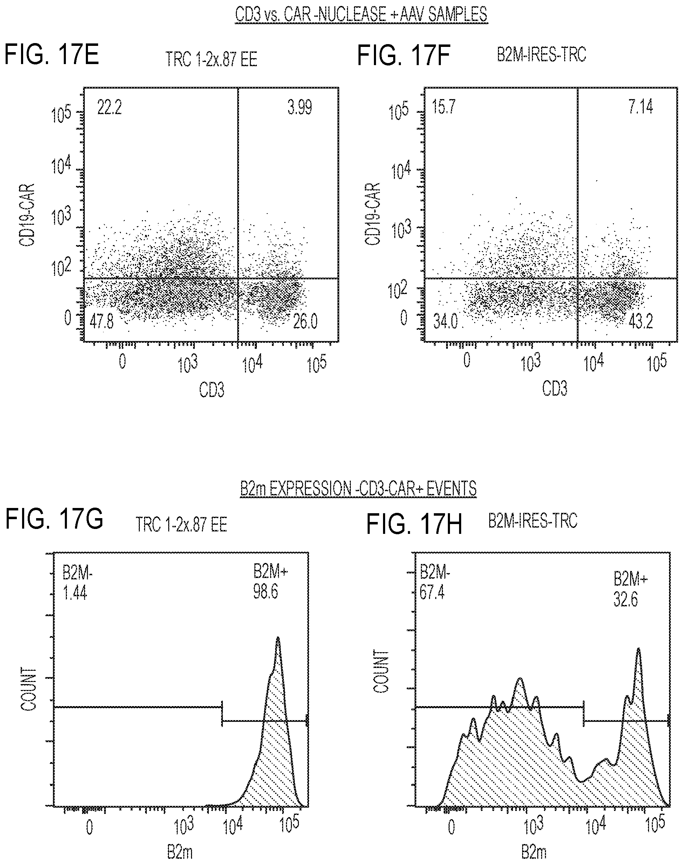

Figs. 17A-17H. Production of anti-CD19 CAR T cells using bicistronic mRNA and AAV. Bicistronic B2M-IRES-TRC was used in conjunction with an AAV vector to introduce an exogenous nucleic acid sequence, encoding a chimeric antigen receptor, into the genome of human T cells at the TRC 1-2 recognition sequence via homologous recombination, while simultaneously knocking out cell-surface expression of both the T cell receptor and B2M. The AAV vector comprised a nucleic acid comprising the anti-CD 19 CAR coding sequence previously described, which was flanked by homology arms. Expression of the CAR cassette was driven by a JeT promoter. As controls, cells were electroporated with 1µg of TRC1-2x87EE RNA prior to AAV transduction. In addition, B2M-IRES-TRC and TRC 1-2x.87 EE electroporated cells were mock transduced. At 3 and 6 days post-electroporation/transduction, edited cells were stained with an antibody against CD3 (to determine TRC knockdown) and an antibody against B2M, as well as a biotinylated recombinant CD19-Fc fusion protein to detect the CAR. Streptavidin-PE was used as the secondary detection reagent for CAR staining. CD3, B2m, and CAR levels were assessed by flow cytometry. A) Staining for CD3 (X axis) and B2M (Y axis) after nucleofection with TRC 1-2x.87 EE. B) Staining for CD3 (X axis) and B2M (Y axis) after nucleofection with B2M-IRES-TRC. C) Staining for CD3 (X axis) and CAR (Y axis) after nucleofection with TRC 1-2x.87 EE and mock transduction. D) Staining for CD3 (X axis) and CAR (Y axis) after nucleofection with B2M-IRES-TRC and mock transduction. E) Staining for CD3 (X axis) and CAR (Y axis) after nucleofection with TRC 1-2x.87 EE and transduction with AAV. F) Staining for CD3 (X axis) and CAR (Y axis) after nucleofection with B2M-IRES-TRC and transduction with AAV. G) Staining for B2M in cells nucleofected with TRC 1-2x.87 EE and transduced with AAV. H) Staining for B2M in cells nucleofected with B2MIRES-TRC and transduced with AAV.

### BRIEF DESCRIPTION OF THE SEQUENCES

SEQ ID NO: 1 sets forth the nucleic acid sequence of the human beta-2 microglobulin gene.
SEQ ID NO:2 sets forth the nucleic acid sequence of the B2M 13-14 recognition sequence (sense).
SEQ ID NO:3 sets forth the nucleic acid sequence of the B2M 13-14 recognition sequence (anti-sense).
SEQ ID NO:4 sets forth the nucleic acid sequence of the B2M 5-6 recognition sequence (sense).
SEQ ID NO:5 sets forth the nucleic acid sequence of the B2M 5-6 recognition sequence (anti-sense).
SEQ ID NO:6 sets forth the nucleic acid sequence of the B2M 7-8 recognition sequence (sense).
SEQ ID NO:7 sets forth the nucleic acid sequence of the B2M 7-8 recognition sequence (anti-sense).
SEQ ID NO:8 sets forth the nucleic acid sequence of the B2M 11-12 recognition sequence (sense).
SEQ ID NO:9 sets forth the nucleic acid sequence of the B2M 11-12 recognition sequence (anti-sense).
SEQ ID NO: 10 sets forth the amino acid sequence of the I-CreI meganuclease.
SEQ ID NO: 11 sets forth the amino acid sequence of the LAGLIDADG motif.
SEQ ID NO: 12 sets forth the amino acid sequence of the B2M 13-14x.479 meganuclease.
SEQ ID NO: 13 sets forth the amino acid sequence of the B2M 13-14x.287 meganuclease.
SEQ ID NO: 14 sets forth the amino acid sequence of the B2M 13-14x.377 meganuclease.
SEQ ID NO:15 sets forth the amino acid sequence of the B2M 13-14x.169 meganuclease.
SEQ ID NO:16 sets forth the amino acid sequence of the B2M 13-14x.202 meganuclease.
SEQ ID NO: 17 sets forth the amino acid sequence of the B2M 13-14x.93 meganuclease.
SEQ ID NO:18 sets forth the amino acid sequence of the B2M 13-14x.93 QE meganuclease.
SEQ ID NO:19 sets forth the amino acid sequence of the B2M 13-14x.93 EQ meganuclease.
SEQ ID NO:20 sets forth the amino acid sequence of the B2M 13-14x.93 EE meganuclease.
SEQ ID NO:21 sets forth the amino acid sequence of the B2M 13-14x.93 QQY66 meganuclease.
SEQ ID NO:22 sets forth the amino acid sequence of the B2M 13-14x.93 QQK66 meganuclease.
SEQ ID NO:23 sets forth the amino acid sequence of the B2M 13-14x.93 QQR66 meganuclease.
SEQ ID NO:24 sets forth the amino acid sequence of the B2M 13-14x.93 EEY66 meganuclease.
SEQ ID NO:25 sets forth the amino acid sequence of the B2M 13-14x.93 EEK66 meganuclease.
SEQ ID NO:26 sets forth the amino acid sequence of the B2M 13-14x.93 EER66 meganuclease.
SEQ ID NO:27 sets forth the amino acid sequence of the B2M 13-14x.93 EQY66 meganuclease.
SEQ ID NO:28 sets forth the amino acid sequence of the B2M 13-14x.93 EQK66 meganuclease.
SEQ ID NO:29 sets forth the amino acid sequence of the B2M 13-14x.93 EQR66 meganuclease.
SEQ ID NO:30 sets forth the amino acid sequence of the B2M 13-14x.3 meganuclease.
SEQ ID NO:31 sets forth the amino acid sequence of the B2M 13-14x.10 meganuclease.
SEQ ID NO:32 sets forth the amino acid sequence of the B2M 13-14x.14 meganuclease.
SEQ ID NO:33 sets forth the amino acid sequence of the B2M 13-14x.22 meganuclease.
SEQ ID NO:34 sets forth the amino acid sequence of the B2M 13-14x.67 meganuclease.
SEQ ID NO:35 sets forth the amino acid sequence of the B2M 13-14x.84 meganuclease.
SEQ ID NO:36 sets forth the amino acid sequence of the B2M 13-14x.85 meganuclease.
SEQ ID NO:37 sets forth the amino acid sequence of the B2M 13-14x.96 meganuclease.
SEQ ID NO:38 sets forth the amino acid sequence of the B2M 13-14x.97 meganuclease.
SEQ ID NO:39 sets forth the amino acid sequence of the B2M 13-14x.102 meganuclease.
SEQ ID NO:40 sets forth the amino acid sequence of the B2M 13-14x.105 meganuclease.
SEQ ID NO:41 sets forth the amino acid sequence of the B2M 13-14x.106 meganuclease.
SEQ ID NO:42 sets forth the amino acid sequence of the B2M 13-14x.1 15 meganuclease.
SEQ ID NO:43 sets forth the amino acid sequence of the B2M 13-14x.139 meganuclease.
SEQ ID NO:44 sets forth the amino acid sequence of the B2M 13-14x.141 meganuclease.
SEQ ID NO:45 sets forth the amino acid sequence of the B2M 13-14x.146 meganuclease.
SEQ ID NO:46 sets forth the amino acid sequence of the B2M 13-14x.162 meganuclease.
SEQ ID NO:47 sets forth the amino acid sequence of the B2M 13-14x.165 meganuclease.
SEQ ID NO:48 sets forth the amino acid sequence of the B2M 13-14x.178 meganuclease.
SEQ ID NO:49 sets forth the amino acid sequence of the B2M 13-14x.182 meganuclease.
SEQ ID NO:50 sets forth the amino acid sequence of the B2M 13-14x.198 meganuclease.
SEQ ID NO:51 sets forth the amino acid sequence of the B2M 13-14x.199 meganuclease.
SEQ ID NO:52 sets forth the amino acid sequence of the B2M 13-14x.207 meganuclease.
SEQ ID NO:53 sets forth the amino acid sequence of the B2M 13-14x.222 meganuclease.
SEQ ID NO:54 sets forth the amino acid sequence of the B2M 13-14x.245 meganuclease.
SEQ ID NO:55 sets forth the amino acid sequence of the B2M 13-14x.255 meganuclease.
SEQ ID NO:56 sets forth the amino acid sequence of the B2M 13-14x.259 meganuclease.
SEQ ID NO:57 sets forth the amino acid sequence of the B2M 13-14x.275 meganuclease.
SEQ ID NO:58 sets forth the amino acid sequence of the B2M 13-14x.280 meganuclease.
SEQ ID NO:59 sets forth the amino acid sequence of the B2M 13-14x.281 meganuclease.
SEQ ID NO:60 sets forth the amino acid sequence of the B2M 13-14x.283 meganuclease.
SEQ ID NO:61 sets forth the amino acid sequence of the B2M 13-14x.285 meganuclease.
SEQ ID NO:62 sets forth the amino acid sequence of the B2M 13-14x.286 meganuclease.
SEQ ID NO:63 sets forth the amino acid sequence of the B2M 13-14x.295 meganuclease.
SEQ ID NO:64 sets forth the amino acid sequence of the B2M 13-14x.301 meganuclease.
SEQ ID NO:65 sets forth the amino acid sequence of the B2M 13-14x.306 meganuclease.
SEQ ID NO:66 sets forth the amino acid sequence of the B2M 13-14x.317 meganuclease.
SEQ ID NO:67 sets forth the amino acid sequence of the B2M 13-14x.325 meganuclease.
SEQ ID NO:68 sets forth the amino acid sequence of the B2M 13-14x.335 meganuclease.
SEQ ID NO:69 sets forth the amino acid sequence of the B2M 13-14x.338 meganuclease.
SEQ ID NO:70 sets forth the amino acid sequence of the B2M 13-14x.347 meganuclease.
SEQ ID NO:71 sets forth the amino acid sequence of the B2M 13-14x.361 meganuclease.
SEQ ID NO:72 sets forth the amino acid sequence of the B2M 13-14x.362 meganuclease.
SEQ ID NO:73 sets forth the amino acid sequence of the B2M 13-14x.365 meganuclease.
SEQ ID NO:74 sets forth the amino acid sequence of the B2M 13-14x.369 meganuclease.
SEQ ID NO:75 sets forth the amino acid sequence of the B2M 13-14x.371 meganuclease.
SEQ ID NO:76 sets forth the amino acid sequence of the B2M 13-14x.372 meganuclease.
SEQ ID NO:77 sets forth the amino acid sequence of the B2M 13-14x.375 meganuclease.
SEQ ID NO:78 sets forth the amino acid sequence of the B2M 13-14x.378 meganuclease.
SEQ ID NO:79 sets forth the amino acid sequence of the B2M 13-14x.385 meganuclease.
SEQ ID NO:80 sets forth the amino acid sequence of the B2M 13-14x.392 meganuclease.
SEQ ID NO:81 sets forth the amino acid sequence of the B2M 13-14x.432 meganuclease.
SEQ ID NO:82 sets forth the amino acid sequence of the B2M 13-14x.433 meganuclease.
SEQ ID NO:83 sets forth the amino acid sequence of the B2M 13-14x.440 meganuclease.
SEQ ID NO:84 sets forth the amino acid sequence of the B2M 13-14x.449 meganuclease.
SEQ ID NO:85 sets forth the amino acid sequence of the B2M 13-14x.456 meganuclease.
SEQ ID NO:86 sets forth the amino acid sequence of the B2M 13-14x.457 meganuclease.
SEQ ID NO:87 sets forth the amino acid sequence of the B2M 13-14x.459 meganuclease.
SEQ ID NO:88 sets forth the amino acid sequence of the B2M 13-14x.464 meganuclease.
SEQ ID NO:89 sets forth the amino acid sequence of the B2M 13-14x.465 meganuclease.
SEQ ID NO:90 sets forth the amino acid sequence of the B2M 13-14x.470 meganuclease.
SEQ ID NO:91 sets forth the amino acid sequence of the B2M 13-14x.471 meganuclease.
SEQ ID NO:92 sets forth the amino acid sequence of the B2M 13-14x.540 meganuclease.
SEQ ID NO:93 sets forth the amino acid sequence of the B2M 13-14x.543 meganuclease.
SEQ ID NO:94 sets forth the amino acid sequence of the B2M 13-14x.551 meganuclease.
SEQ ID NO:95 sets forth the amino acid sequence of the B2M 13-14x.554 meganuclease.
SEQ ID NO:96 sets forth the amino acid sequence of the B2M 13-14x.556 meganuclease.
SEQ ID NO:97 sets forth the amino acid sequence of the B2M 13-14x.76 meganuclease.
SEQ ID NO:98 sets forth the amino acid sequence of the B2M 13-14x.82 meganuclease.
SEQ ID NO:99 sets forth the amino acid sequence of the B2M 13-14x.31 meganuclease.
SEQ ID NO:100 sets forth the amino acid sequence of the B2M 13-14x.32 meganuclease.
SEQ ID NO:101 sets forth the amino acid sequence of the B2M 5-6x. 14 meganuclease.
SEQ ID NO:102 sets forth the amino acid sequence of the B2M 5-6x.5 meganuclease.
SEQ ID NO:103 sets forth the amino acid sequence of the B2M 5-6x.6 meganuclease.
SEQ ID NO:104 sets forth the amino acid sequence of the B2M 5-6x. 13 meganuclease.
SEQ ID NO:105 sets forth the amino acid sequence of the B2M 5-6x.22 meganuclease.
SEQ ID NO:106 sets forth the amino acid sequence of the B2M 5-6x.31 meganuclease.
SEQ ID NO:107 sets forth the amino acid sequence of the B2M 5-6x.69 meganuclease.
SEQ ID NO:108 sets forth the amino acid sequence of the B2M 5-6x.73 meganuclease.
SEQ ID NO:109 sets forth the amino acid sequence of the B2M 5-6x.85 meganuclease.
SEQ ID NO:110 sets forth the amino acid sequence of the B2M 5-6x.86 meganuclease.
SEQ ID NO:111 sets forth the amino acid sequence of the B2M 5-6x.91 meganuclease.
SEQ ID NO:112 sets forth the amino acid sequence of the B2M 5-6x.28 meganuclease.
SEQ ID NO:113 sets forth the amino acid sequence of the B2M 5-6x.3 meganuclease.
SEQ ID NO:114 sets forth the amino acid sequence of the B2M 7-8x.88 meganuclease.
SEQ ID NO:115 sets forth the amino acid sequence of the B2M 7-8x.7 meganuclease.
SEQ ID NO:116 sets forth the amino acid sequence of the B2M 7-8x.23 meganuclease.
SEQ ID NO:117 sets forth the amino acid sequence of the B2M 7-8x.30 meganuclease.
SEQ ID NO:118 sets forth the amino acid sequence of the B2M 7-8x.53 meganuclease.
SEQ ID NO:119 sets forth the amino acid sequence of the B2M 7-8x.2 meganuclease.
SEQ ID NO:120 sets forth the amino acid sequence of the B2M 7-8x.3 meganuclease.
SEQ ID NO:121 sets forth the amino acid sequence of the B2M 7-8x.6 meganuclease.
SEQ ID NO:122 sets forth the amino acid sequence of the B2M 7-8x.25 meganuclease.
SEQ ID NO:123 sets forth the amino acid sequence of the B2M 7-8x.78 meganuclease.
SEQ ID NO:124 sets forth the amino acid sequence of the B2M 7-8x.85 meganuclease.
SEQ ID NO:125 sets forth the amino acid sequence of the B2M 11-12x.45 meganuclease.
SEQ ID NO:126 sets forth the amino acid sequence of the B2M 11-12x.2 meganuclease.
SEQ ID NO:127 sets forth the nucleic acid sequence of the human T cell receptor alpha constant region gene.
SEQ ID NO:128 sets forth the nucleic acid sequence of the TRC 1-2 recognition sequence (sense).
SEQ ID NO:129 sets forth the nucleic acid sequence of the TRC 3-4 recognition sequence (sense).
SEQ ID NO:130 sets forth the nucleic acid sequence of the TRC 7-8 recognition sequence (sense).
SEQ ID NO:131 sets forth the amino acid sequence of the TRC 1-2x.87 EE meganuclease.
SEQ ID NO:132 sets forth the B2M13 half-site binding subunit of the B2M 13-14x.479 meganuclease.
SEQ ID NO:133 sets forth the B2M13 half-site binding subunit of the B2M 13-14x.287 meganuclease.
SEQ ID NO:134 sets forth the B2M13 half-site binding subunit of the B2M 13-14x.377 meganuclease.
SEQ ID NO:135 sets forth the B2M13 half-site binding subunit of the B2M 13-14x.169 meganuclease.
SEQ ID NO:136 sets forth the B2M13 half-site binding subunit of the B2M 13-14x.202 meganuclease.
SEQ ID NO:137 sets forth the B2M13 half-site binding subunit of the B2M 13-14x.93 meganuclease.
SEQ ID NO:138 sets forth the B2M13 half-site binding subunit of the B2M 13-14x.93 QE meganuclease.
SEQ ID NO:139 sets forth the B2M13 half-site binding subunit of the B2M 13-14x.93 EQ meganuclease.
SEQ ID NO:140 sets forth the B2M13 half-site binding subunit of the B2M 13-14x.93 EE meganuclease.
SEQ ID NO:141 sets forth the B2M13 half-site binding subunit of the B2M 13-14x.93 QQY66 meganuclease.
SEQ ID NO:142 sets forth the B2M13 half-site binding subunit of the B2M 13-14x.93 QQK66 meganuclease.
SEQ ID NO:143 sets forth the B2M13 half-site binding subunit of the B2M 13-14x.93 QQR66 meganuclease.
SEQ ID NO:144 sets forth the B2M13 half-site binding subunit of the B2M 13-14x.93 EEY66 meganuclease.
SEQ ID NO: 145 sets forth the B2M13 half-site binding subunit of the B2M 13-14x.93 EEK66 meganuclease.
SEQ ID NO: 146 sets forth the B2M13 half-site binding subunit of the B2M 13-14x.93 EER66 meganuclease.
SEQ ID NO: 147 sets forth the B2M13 half-site binding subunit of the B2M 13-14x.93 EQY66 meganuclease.
SEQ ID NO: 148 sets forth the B2M13 half-site binding subunit of the B2M 13-14x.93 EQK66 meganuclease.
SEQ ID NO: 149 sets forth the B2M13 half-site binding subunit of the B2M 13-14x.93 EQR66 meganuclease.
SEQ ID NO: 150 sets forth the B2M13 half-site binding subunit of the B2M 13-14x.3 meganuclease.
SEQ ID NO: 151 sets forth the B2M13 half-site binding subunit of the B2M 13-14x.10 meganuclease.
SEQ ID NO: 152 sets forth the B2M13 half-site binding subunit of the B2M 13-14x.14 meganuclease.
SEQ ID NO: 153 sets forth the B2M13 half-site binding subunit of the B2M 13-14x.22 meganuclease.
SEQ ID NO: 154 sets forth the B2M13 half-site binding subunit of the B2M 13-14x.67 meganuclease.
SEQ ID NO: 155 sets forth the B2M13 half-site binding subunit of the B2M 13-14x.84 meganuclease.
SEQ ID NO: 156 sets forth the B2M13 half-site binding subunit of the B2M 13-14x.85 meganuclease.
SEQ ID NO: 157 sets forth the B2M13 half-site binding subunit of the B2M 13-14x.96 meganuclease.
SEQ ID NO: 158 sets forth the B2M13 half-site binding subunit of the B2M 13-14x.97 meganuclease.
SEQ ID NO: 159 sets forth the B2M13 half-site binding subunit of the B2M 13-14x.102 meganuclease.
SEQ ID NO: 160 sets forth the B2M13 half-site binding subunit of the B2M 13-14x.105 meganuclease.
SEQ ID NO:161 sets forth the B2M13 half-site binding subunit of the B2M 13-14x.106 meganuclease.
SEQ ID NO:162 sets forth the B2M13 half-site binding subunit of the B2M 13-14x.115 meganuclease.
SEQ ID NO:163 sets forth the B2M13 half-site binding subunit of the B2M 13-14x.139 meganuclease.
SEQ ID NO:164 sets forth the B2M13 half-site binding subunit of the B2M 13-14x.141 meganuclease.
SEQ ID NO:165 sets forth the B2M13 half-site binding subunit of the B2M 13-14x.146 meganuclease.
SEQ ID NO:166 sets forth the B2M13 half-site binding subunit of the B2M 13-14x.162 meganuclease.
SEQ ID NO:167 sets forth the B2M13 half-site binding subunit of the B2M 13-14x.165 meganuclease.
SEQ ID NO:168 sets forth the B2M13 half-site binding subunit of the B2M 13-14x.178 meganuclease.
SEQ ID NO:169 sets forth the B2M13 half-site binding subunit of the B2M 13-14x.182 meganuclease.
SEQ ID NO:170 sets forth the B2M13 half-site binding subunit of the B2M 13-14x.198 meganuclease.
SEQ ID NO:171 sets forth the B2M13 half-site binding subunit of the B2M 13-14x.199 meganuclease.
SEQ ID NO:172 sets forth the B2M13 half-site binding subunit of the B2M 13-14x.207 meganuclease.
SEQ ID NO:173 sets forth the B2M13 half-site binding subunit of the B2M 13-14x.222 meganuclease.
SEQ ID NO:174 sets forth the B2M13 half-site binding subunit of the B2M 13-14x.245 meganuclease.
SEQ ID NO:175 sets forth the B2M13 half-site binding subunit of the B2M 13-14x.255 meganuclease.
SEQ ID NO:176 sets forth the B2M13 half-site binding subunit of the B2M 13-14x.259 meganuclease.
SEQ ID NO:177 sets forth the B2M13 half-site binding subunit of the B2M 13-14x.275 meganuclease.
SEQ ID NO:178 sets forth the B2M13 half-site binding subunit of the B2M 13-14x.280 meganuclease.
SEQ ID NO:179 sets forth the B2M13 half-site binding subunit of the B2M 13-14x.281 meganuclease.
SEQ ID NO:180 sets forth the B2M13 half-site binding subunit of the B2M 13-14x.283 meganuclease.
SEQ ID NO:181 sets forth the B2M13 half-site binding subunit of the B2M 13-14x.285 meganuclease.
SEQ ID NO:182 sets forth the B2M13 half-site binding subunit of the B2M 13-14x.286 meganuclease.
SEQ ID NO:183 sets forth the B2M13 half-site binding subunit of the B2M 13-14x.295 meganuclease.
SEQ ID NO:184 sets forth the B2M13 half-site binding subunit of the B2M 13-14x.301 meganuclease.
SEQ ID NO:185 sets forth the B2M13 half-site binding subunit of the B2M 13-14x.306 meganuclease.
SEQ ID NO:186 sets forth the B2M13 half-site binding subunit of the B2M 13-14x.317 meganuclease.
SEQ ID NO:187 sets forth the B2M13 half-site binding subunit of the B2M 13-14x.325 meganuclease.
SEQ ID NO:188 sets forth the B2M13 half-site binding subunit of the B2M 13-14x.335 meganuclease.
SEQ ID NO:189 sets forth the B2M13 half-site binding subunit of the B2M 13-14x.338 meganuclease.
SEQ ID NO:190 sets forth the B2M13 half-site binding subunit of the B2M 13-14x.347 meganuclease.
SEQ ID NO:191 sets forth the B2M13 half-site binding subunit of the B2M 13-14x.361 meganuclease.
SEQ ID NO:192 sets forth the B2M13 half-site binding subunit of the B2M 13-14x.362 meganuclease.
SEQ ID NO: 193 sets forth the B2M13 half-site binding subunit of the B2M 13-14x.365 meganuclease.
SEQ ID NO: 194 sets forth the B2M13 half-site binding subunit of the B2M 13-14x.369 meganuclease.
SEQ ID NO: 195 sets forth the B2M13 half-site binding subunit of the B2M 13-14x.371 meganuclease.
SEQ ID NO: 196 sets forth the B2M13 half-site binding subunit of the B2M 13-14x.372 meganuclease.
SEQ ID NO: 197 sets forth the B2M13 half-site binding subunit of the B2M 13-14x.375 meganuclease.
SEQ ID NO: 198 sets forth the B2M13 half-site binding subunit of the B2M 13-14x.378 meganuclease.
SEQ ID NO: 199 sets forth the B2M13 half-site binding subunit of the B2M 13-14x.385 meganuclease.
SEQ ID NO:200 sets forth the B2M13 half-site binding subunit of the B2M 13-14x.392 meganuclease.
SEQ ID NO:201 sets forth the B2M13 half-site binding subunit of the B2M 13-14x.432 meganuclease.
SEQ ID NO:202 sets forth the B2M13 half-site binding subunit of the B2M 13-14x.433 meganuclease.
SEQ ID NO:203 sets forth the B2M13 half-site binding subunit of the B2M 13-14x.440 meganuclease.
SEQ ID NO:204 sets forth the B2M13 half-site binding subunit of the B2M 13-14x.449 meganuclease.
SEQ ID NO:205 sets forth the B2M13 half-site binding subunit of the B2M 13-14x.456 meganuclease.
SEQ ID NO:206 sets forth the B2M13 half-site binding subunit of the B2M 13-14x.457 meganuclease.
SEQ ID NO:207 sets forth the B2M13 half-site binding subunit of the B2M 13-14x.459 meganuclease.
SEQ ID NO:208 sets forth the B2M13 half-site binding subunit of the B2M 13-14x.464 meganuclease.
SEQ ID NO:209 sets forth the B2M13 half-site binding subunit of the B2M 13-14x.465 meganuclease.
SEQ ID NO:210 sets forth the B2M13 half-site binding subunit of the B2M 13-14x.470 meganuclease.
SEQ ID NO:211 sets forth the B2M13 half-site binding subunit of the B2M 13-14x.471 meganuclease.
SEQ ID NO:212 sets forth the B2M13 half-site binding subunit of the B2M 13-14x.540 meganuclease.
SEQ ID NO:213 sets forth the B2M13 half-site binding subunit of the B2M 13-14x.543 meganuclease.
SEQ ID NO:214 sets forth the B2M13 half-site binding subunit of the B2M 13-14x.551 meganuclease.
SEQ ID NO:215 sets forth the B2M13 half-site binding subunit of the B2M 13-14x.554 meganuclease.
SEQ ID NO:216 sets forth the B2M13 half-site binding subunit of the B2M 13-14x.556 meganuclease.
SEQ ID NO:217 sets forth the B2M13 half-site binding subunit of the B2M 13-14x.76 meganuclease.
SEQ ID NO:218 sets forth the B2M13 half-site binding subunit of the B2M 13-14x.82 meganuclease.
SEQ ID NO:219 sets forth the B2M13 half-site binding subunit of the B2M 13-14x.31 meganuclease.
SEQ ID NO:220 sets forth the B2M13 half-site binding subunit of the B2M 13-14x.32 meganuclease.
SEQ ID NO:221 sets forth the B2M14 half-site binding subunit of the B2M 13-14x.479 meganuclease.
SEQ ID NO:222 sets forth the B2M14 half-site binding subunit of the B2M 13-14x.287 meganuclease.
SEQ ID NO:223 sets forth the B2M14 half-site binding subunit of the B2M 13-14x.377 meganuclease.
SEQ ID NO:224 sets forth the B2M14 half-site binding subunit of the B2M 13-14x.169 meganuclease.
SEQ ID NO:225 sets forth the B2M14 half-site binding subunit of the B2M 13-14x.202 meganuclease.
SEQ ID NO:226 sets forth the B2M14 half-site binding subunit of the B2M 13-14x.93 meganuclease.
SEQ ID NO:227 sets forth the B2M14 half-site binding subunit of the B2M 13-14x.93 QE meganuclease.
SEQ ID NO:228 sets forth the B2M14 half-site binding subunit of the B2M 13-14x.93 EQ meganuclease.
SEQ ID NO:229 sets forth the B2M14 half-site binding subunit of the B2M 13-14x.93 EE meganuclease.
SEQ ID NO:230 sets forth the B2M14 half-site binding subunit of the B2M 13-14x.93 QQY66 meganuclease.
SEQ ID NO:231 sets forth the B2M14 half-site binding subunit of the B2M 13-14x.93 QQK66 meganuclease.
SEQ ID NO:232 sets forth the B2M14 half-site binding subunit of the B2M 13-14x.93 QQR66 meganuclease.
SEQ ID NO:233 sets forth the B2M14 half-site binding subunit of the B2M 13-14x.93 EEY66 meganuclease.
SEQ ID NO:234 sets forth the B2M14 half-site binding subunit of the B2M 13-14x.93 EEK66 meganuclease.
SEQ ID NO:235 sets forth the B2M14 half-site binding subunit of the B2M 13-14x.93 EER66 meganuclease.
SEQ ID NO:236 sets forth the B2M14 half-site binding subunit of the B2M 13-14x.93 EQY66 meganuclease.
SEQ ID NO:237 sets forth the B2M14 half-site binding subunit of the B2M 13-14x.93 EQK66 meganuclease.
SEQ ID NO:238 sets forth the B2M14 half-site binding subunit of the B2M 13-14x.93 EQR66 meganuclease.
SEQ ID NO:239 sets forth the B2M14 half-site binding subunit of the B2M 13-14x.3 meganuclease.
SEQ ID NO:240 sets forth the B2M14 half-site binding subunit of the B2M 13-14x.10 meganuclease.
SEQ ID NO:241 sets forth the B2M14 half-site binding subunit of the B2M 13-14x.14 meganuclease.
SEQ ID NO:242 sets forth the B2M14 half-site binding subunit of the B2M 13-14x.22 meganuclease.
SEQ ID NO:243 sets forth the B2M14 half-site binding subunit of the B2M 13-14x.67 meganuclease.
SEQ ID NO:244 sets forth the B2M14 half-site binding subunit of the B2M 13-14x.84 meganuclease.
SEQ ID NO:245 sets forth the B2M14 half-site binding subunit of the B2M 13-14x.85 meganuclease.
SEQ ID NO:246 sets forth the B2M14 half-site binding subunit of the B2M 13-14x.96 meganuclease.
SEQ ID NO:247 sets forth the B2M14 half-site binding subunit of the B2M 13-14x.97 meganuclease.
SEQ ID NO:248 sets forth the B2M14 half-site binding subunit of the B2M 13-14x.102 meganuclease.
SEQ ID NO:249 sets forth the B2M14 half-site binding subunit of the B2M 13-14x.105 meganuclease.
SEQ ID NO:250 sets forth the B2M14 half-site binding subunit of the B2M 13-14x.106 meganuclease.
SEQ ID NO:251 sets forth the B2M14 half-site binding subunit of the B2M 13-14x.115 meganuclease.
SEQ ID NO:252 sets forth the B2M14 half-site binding subunit of the B2M 13-14x.139 meganuclease.
SEQ ID NO:253 sets forth the B2M14 half-site binding subunit of the B2M 13-14x.141 meganuclease.
SEQ ID NO:254 sets forth the B2M14 half-site binding subunit of the B2M 13-14x.146 meganuclease.
SEQ ID NO:255 sets forth the B2M14 half-site binding subunit of the B2M 13-14x.162 meganuclease.
SEQ ID NO:256 sets forth the B2M14 half-site binding subunit of the B2M 13-14x.165 meganuclease.
SEQ ID NO:257 sets forth the B2M14 half-site binding subunit of the B2M 13-14x.178 meganuclease.
SEQ ID NO:258 sets forth the B2M14 half-site binding subunit of the B2M 13-14x.182 meganuclease.
SEQ ID NO:259 sets forth the B2M14 half-site binding subunit of the B2M 13-14x.198 meganuclease.
SEQ ID NO:260 sets forth the B2M14 half-site binding subunit of the B2M 13-14x.199 meganuclease.
SEQ ID NO:261 sets forth the B2M14 half-site binding subunit of the B2M 13-14x.207 meganuclease.
SEQ ID NO:262 sets forth the B2M14 half-site binding subunit of the B2M 13-14x.222 meganuclease.
SEQ ID NO:263 sets forth the B2M14 half-site binding subunit of the B2M 13-14x.245 meganuclease.
SEQ ID NO:264 sets forth the B2M14 half-site binding subunit of the B2M 13-14x.255 meganuclease.
SEQ ID NO:265 sets forth the B2M14 half-site binding subunit of the B2M 13-14x.259 meganuclease.
SEQ ID NO:266 sets forth the B2M14 half-site binding subunit of the B2M 13-14x.275 meganuclease.
SEQ ID NO:267 sets forth the B2M14 half-site binding subunit of the B2M 13-14x.280 meganuclease.
SEQ ID NO:268 sets forth the B2M14 half-site binding subunit of the B2M 13-14x.281 meganuclease.
SEQ ID NO:269 sets forth the B2M14 half-site binding subunit of the B2M 13-14x.283 meganuclease.
SEQ ID NO:270 sets forth the B2M14 half-site binding subunit of the B2M 13-14x.285 meganuclease.
SEQ ID NO:271 sets forth the B2M14 half-site binding subunit of the B2M 13-14x.286 meganuclease.
SEQ ID NO:272 sets forth the B2M14 half-site binding subunit of the B2M 13-14x.295 meganuclease.
SEQ ID NO:273 sets forth the B2M14 half-site binding subunit of the B2M 13-14x.301 meganuclease.
SEQ ID NO:274 sets forth the B2M14 half-site binding subunit of the B2M 13-14x.306 meganuclease.
SEQ ID NO:275 sets forth the B2M14 half-site binding subunit of the B2M 13-14x.317 meganuclease.
SEQ ID NO:276 sets forth the B2M14 half-site binding subunit of the B2M 13-14x.325 meganuclease.
SEQ ID NO:277 sets forth the B2M14 half-site binding subunit of the B2M 13-14x.335 meganuclease.
SEQ ID NO:278 sets forth the B2M14 half-site binding subunit of the B2M 13-14x.338 meganuclease.
SEQ ID NO:279 sets forth the B2M14 half-site binding subunit of the B2M 13-14x.347 meganuclease.
SEQ ID NO:280 sets forth the B2M14 half-site binding subunit of the B2M 13-14x.361 meganuclease.
SEQ ID NO:281 sets forth the B2M14 half-site binding subunit of the B2M 13-14x.362 meganuclease.
SEQ ID NO:282 sets forth the B2M14 half-site binding subunit of the B2M 13-14x.365 meganuclease.
SEQ ID NO:283 sets forth the B2M14 half-site binding subunit of the B2M 13-14x.369 meganuclease.
SEQ ID NO:284 sets forth the B2M14 half-site binding subunit of the B2M 13-14x.371 meganuclease.
SEQ ID NO:285 sets forth the B2M14 half-site binding subunit of the B2M 13-14x.372 meganuclease.
SEQ ID NO:286 sets forth the B2M14 half-site binding subunit of the B2M 13-14x.375 meganuclease.
SEQ ID NO:287 sets forth the B2M14 half-site binding subunit of the B2M 13-14x.378 meganuclease.
SEQ ID NO:288 sets forth the B2M14 half-site binding subunit of the B2M 13-14x.385 meganuclease.
SEQ ID NO:289 sets forth the B2M14 half-site binding subunit of the B2M 13-14x.392 meganuclease.
SEQ ID NO:290 sets forth the B2M14 half-site binding subunit of the B2M 13-14x.432 meganuclease.
SEQ ID NO:291 sets forth the B2M14 half-site binding subunit of the B2M 13-14x.433 meganuclease.
SEQ ID NO:292 sets forth the B2M14 half-site binding subunit of the B2M 13-14x.440 meganuclease.
SEQ ID NO:293 sets forth the B2M14 half-site binding subunit of the B2M 13-14x.449 meganuclease.
SEQ ID NO:294 sets forth the B2M14 half-site binding subunit of the B2M 13-14x.456 meganuclease.
SEQ ID NO:295 sets forth the B2M14 half-site binding subunit of the B2M 13-14x.457 meganuclease.
SEQ ID NO:296 sets forth the B2M14 half-site binding subunit of the B2M 13-14x.459 meganuclease.
SEQ ID NO:297 sets forth the B2M14 half-site binding subunit of the B2M 13-14x.464 meganuclease.
SEQ ID NO:298 sets forth the B2M14 half-site binding subunit of the B2M 13-14x.465 meganuclease.
SEQ ID NO:299 sets forth the B2M14 half-site binding subunit of the B2M 13-14x.470 meganuclease.
SEQ ID NO:300 sets forth the B2M14 half-site binding subunit of the B2M 13-14x.471 meganuclease.
SEQ ID NO:301 sets forth the B2M14 half-site binding subunit of the B2M 13-14x.540 meganuclease.
SEQ ID NO:302 sets forth the B2M14 half-site binding subunit of the B2M 13-14x.543 meganuclease.
SEQ ID NO:303 sets forth the B2M14 half-site binding subunit of the B2M 13-14x.551 meganuclease.
SEQ ID NO:304 sets forth the B2M14 half-site binding subunit of the B2M 13-14x.554 meganuclease.
SEQ ID NO:305 sets forth the B2M14 half-site binding subunit of the B2M 13-14x.556 meganuclease.
SEQ ID NO:306 sets forth the B2M14 half-site binding subunit of the B2M 13-14x.76 meganuclease.
SEQ ID NO:307 sets forth the B2M14 half-site binding subunit of the B2M 13-14x.82 meganuclease.
SEQ ID NO:308 sets forth the B2M14 half-site binding subunit of the B2M 13-14x.31 meganuclease.
SEQ ID NO:309 sets forth the B2M14 half-site binding subunit of the B2M 13-14x.32 meganuclease.
SEQ ID NO:310 sets forth the B2M5 half-site binding subunit of the B2M 5-6x.14 meganuclease.
SEQ ID NO:311 sets forth the B2M5 half-site binding subunit of the B2M 5-6x.5 meganuclease.
SEQ ID NO:312 sets forth the B2M5 half-site binding subunit of the B2M 5-6x.6 meganuclease.
SEQ ID NO:313 sets forth the B2M5 half-site binding subunit of the B2M 5-6x.13 meganuclease.
SEQ ID NO:314 sets forth the B2M5 half-site binding subunit of the B2M 5-6x.22 meganuclease.
SEQ ID NO:315 sets forth the B2M5 half-site binding subunit of the B2M 5-6x.31 meganuclease.
SEQ ID NO:316 sets forth the B2M5 half-site binding subunit of the B2M 5-6x.69 meganuclease.
SEQ ID NO:317 sets forth the B2M5 half-site binding subunit of the B2M 5-6x.73 meganuclease.
SEQ ID NO:318 sets forth the B2M5 half-site binding subunit of the B2M 5-6x.85 meganuclease.
SEQ ID NO:319 sets forth the B2M5 half-site binding subunit of the B2M 5-6x.86 meganuclease.
SEQ ID NO:320 sets forth the B2M5 half-site binding subunit of the B2M 5-6x.91 meganuclease.
SEQ ID NO:321 sets forth the B2M5 half-site binding subunit of the B2M 5-6x.28 meganuclease.
SEQ ID NO:322 sets forth the B2M5 half-site binding subunit of the B2M 5-6x.3 meganuclease.
SEQ ID NO:323 sets forth the B2M6 half-site binding subunit of the B2M 5-6x.14 meganuclease.
SEQ ID NO:324 sets forth the B2M6 half-site binding subunit of the B2M 5-6x.5 meganuclease.
SEQ ID NO:325 sets forth the B2M6 half-site binding subunit of the B2M 5-6x.6 meganuclease.
SEQ ID NO:326 sets forth the B2M6 half-site binding subunit of the B2M 5-6x.13 meganuclease.
SEQ ID NO:327 sets forth the B2M6 half-site binding subunit of the B2M 5-6x.22 meganuclease.
SEQ ID NO:328 sets forth the B2M6 half-site binding subunit of the B2M 5-6x.31 meganuclease.
SEQ ID NO:329 sets forth the B2M6 half-site binding subunit of the B2M 5-6x.69 meganuclease.
SEQ ID NO:330 sets forth the B2M6 half-site binding subunit of the B2M 5-6x.73 meganuclease.
SEQ ID NO:331 sets forth the B2M6 half-site binding subunit of the B2M 5-6x.85 meganuclease.
SEQ ID NO:332 sets forth the B2M6 half-site binding subunit of the B2M 5-6x.86 meganuclease.
SEQ ID NO:333 sets forth the B2M6 half-site binding subunit of the B2M 5-6x.91 meganuclease.
SEQ ID NO:334 sets forth the B2M6 half-site binding subunit of the B2M 5-6x.28 meganuclease.
SEQ ID NO:335 sets forth the B2M6 half-site binding subunit of the B2M 5-6x.3 meganuclease.
SEQ ID NO:336 sets forth the B2M7 half-site binding subunit of the B2M 7-8x.88 meganuclease.
SEQ ID NO:337 sets forth the B2M7 half-site binding subunit of the B2M 7-8x.7 meganuclease.
SEQ ID NO:338 sets forth the B2M7 half-site binding subunit of the B2M 7-8x.23 meganuclease.
SEQ ID NO:339 sets forth the B2M7 half-site binding subunit of the B2M 7-8x.30 meganuclease.
SEQ ID NO:340 sets forth the B2M7 half-site binding subunit of the B2M 7-8x.53 meganuclease.
SEQ ID NO:341 sets forth the B2M7 half-site binding subunit of the B2M 7-8x.2 meganuclease.
SEQ ID NO:342 sets forth the B2M7 half-site binding subunit of the B2M 7-8x.3 meganuclease.
SEQ ID NO:343 sets forth the B2M7 half-site binding subunit of the B2M 7-8x.6 meganuclease.
SEQ ID NO:344 sets forth the B2M7 half-site binding subunit of the B2M 7-8x.25 meganuclease.
SEQ ID NO:345 sets forth the B2M7 half-site binding subunit of the B2M 7-8x.78 meganuclease.
SEQ ID NO:346 sets forth the B2M7 half-site binding subunit of the B2M 7-8x.85 meganuclease.
SEQ ID NO:347 sets forth the B2M8 half-site binding subunit of the B2M 7-8x.88 meganuclease.
SEQ ID NO:348 sets forth the B2M8 half-site binding subunit of the B2M 7-8x.7 meganuclease.
SEQ ID NO:349 sets forth the B2M8 half-site binding subunit of the B2M 7-8x.23 meganuclease.
SEQ ID NO:350 sets forth the B2M8 half-site binding subunit of the B2M 7-8x.30 meganuclease.
SEQ ID NO:351 sets forth the B2M8 half-site binding subunit of the B2M 7-8x.53 meganuclease.
SEQ ID NO:352 sets forth the B2M8 half-site binding subunit of the B2M 7-8x.2 meganuclease.
SEQ ID NO:353 sets forth the B2M8 half-site binding subunit of the B2M 7-8x.3 meganuclease.
SEQ ID NO:354 sets forth the B2M8 half-site binding subunit of the B2M 7-8x.6 meganuclease.
SEQ ID NO:355 sets forth the B2M8 half-site binding subunit of the B2M 7-8x.25 meganuclease.
SEQ ID NO:356 sets forth the B2M8 half-site binding subunit of the B2M 7-8x.78 meganuclease.
SEQ ID NO:357 sets forth the B2M8 half-site binding subunit of the B2M 7-8x.85 meganuclease.
SEQ ID NO:358 sets forth the B2M11 half-site binding subunit of the B2M 11-12x.45 meganuclease.
SEQ ID NO:359 sets forth the B2M11 half-site binding subunit of the B2M 11-12x.2 meganuclease.
SEQ ID NO:360 sets forth the B2M12 half-site binding subunit of the B2M 11-12x.45 meganuclease.
SEQ ID NO:361 sets forth the B2M12 half-site binding subunit of the B2M 11-12x.2 meganuclease.
SEQ ID NO:362 sets forth the nucleic acid sequence of an IRES element.
SEQ ID NO:363 sets forth the nucleic acid sequence of a T2A element.
SEQ ID NO:364 sets forth the nucleic acid sequence of a P2A element.
SEQ ID NO:365 sets forth the nucleic acid sequence of a E2A element.
SEQ ID NO:366 sets forth the nucleic acid sequence of a F2A element.
SEQ ID NO:367 sets forth the nucleic acid sequence of a TRC-IRES-B2M bicistronic mRNA.
SEQ ID NO:368 sets forth the nucleic acid sequence of a TRC-T2A-B2M bicistronic mRNA.
SEQ ID NO:369 sets forth the nucleic acid sequence of a TRC-P2A-B2M bicistronic mRNA.
SEQ ID NO:370 sets forth the nucleic acid sequence of a TRC-E2A-B2M bicistronic mRNA.
SEQ ID NO:371 sets forth the nucleic acid sequence of a TRC-F2A-B2M bicistronic mRNA.
SEQ ID NO:372 sets forth the nucleic acid sequence of a B2M-IRES-TRC bicistronic mRNA.
SEQ ID NO:373 sets forth the nucleic acid sequence of a B2M-T2A-TRC bicistronic mRNA.
SEQ ID NO:374 sets forth the nucleic acid sequence of a B2M-P2A-TRC bicistronic mRNA.
SEQ ID NO:375 sets forth the nucleic acid sequence of a B2M-E2A-TRC bicistronic mRNA.
SEQ ID NO:376 sets forth the nucleic acid sequence of a B2M-F2A-TRC bicistronic mRNA.

### DETAILED DESCRIPTION OF THE INVENTION

### 1.1 References and Definitions

The patent and scientific literature referred to herein establishes knowledge that is available to those of skill in the art. The issued US patents, allowed applications, published foreign applications, and references, including GenBank database sequences, which are cited herein are hereby incorporated by reference to the same extent as if each was specifically and individually indicated to be incorporated by reference.

The present disclosure is embodied in different forms and should not be construed as limited to the embodiments set forth herein. Rather, these embodiments are provided so that this disclosure will be thorough and complete, and will fully convey the scope of the disclosure to those skilled in the art. For example, features illustrated with respect to one embodiment can be incorporated into other embodiments, and features illustrated with respect to a particular embodiment can be deleted from that embodiment. In addition, numerous variations and additions to the embodiments suggested herein will be apparent to those skilled in the art in light of the instant disclosure, which do not depart from the instant disclosure.

Unless otherwise defined, all technical and scientific terms used herein have the same meaning as commonly understood by one of ordinary skill in the art to which this disclosure belongs. The terminology used in the description of the disclosure herein is for the purpose of describing particular embodiments only and is not intended to be limiting of the disclosure.

All publications, patent applications, patents, and other references mentioned herein are incorporated by reference herein in their entirety.

As used herein, "a," "an," or "the" can mean one or more than one. For example, "a" cell can mean a single cell or a multiplicity of cells.

As used herein, unless specifically indicated otherwise, the word "or" is used in the inclusive sense of "and/or" and not the exclusive sense of "either/or."

As used herein, the term "meganuclease" refers to an endonuclease that binds double-stranded DNA at a recognition sequence that is greater than 12 base pairs. Preferably, the recognition sequence for a meganuclease of the disclosure is 22 base pairs. A meganuclease is an endonuclease that is derived from I-CreI, and can refer to an engineered variant of I-Crel that has been modified relative to natural I-CreI with respect to, for example, DNA-binding specificity, DNA cleavage activity, DNA-binding affinity, or dimerization properties. Methods for producing such modified variants of I-Crel are known in the art *(e.g.* WO 2007/047859). A meganuclease as used herein binds to double-stranded DNA as a heterodimer. A meganuclease may also be a "single-chain meganuclease" in which a pair of DNA-binding domains are joined into a single polypeptide using a peptide linker. The term "homing endonuclease" is synonymous with the term "meganuclease." Meganucleases of the disclosure are substantially non-toxic when expressed in cells, particularly in human T cells, such that cells can be transfected and maintained at 37°C without observing deleterious effects on cell viability or significant reductions in meganuclease cleavage activity when measured using the methods described herein.

As used herein, the term "single-chain meganuclease" refers to a polypeptide comprising a pair of nuclease subunits joined by a linker. A single-chain meganuclease has the organization: N-terminal subunit - Linker - C-terminal subunit. The two meganuclease subunits will generally be non-identical in amino acid sequence and will recognize non-identical DNA sequences. Thus, single-chain meganucleases typically cleave pseudo-palindromic or non-palindromic recognition sequences. A single-chain meganuclease is referred to as a "single-chain heterodimer" or "single-chain heterodimeric meganuclease" although it is not, in fact, dimeric. For clarity, unless otherwise specified, the term "meganuclease" can refer to a dimeric or single-chain meganuclease.

As used herein, the term "linker" refers to an exogenous peptide sequence used to join two meganuclease subunits into a single polypeptide. A linker may have a sequence that is found in natural proteins, or is an artificial sequence that is not found in any natural protein. A linker is flexible and lacking in secondary structure or may have a propensity to form a specific three-dimensional structure under physiological conditions. A linker can include, without limitation, those encompassed by U.S. Patent No. 8,445,251. In some embodiments, a linker may have an amino acid sequence comprising residues 154-195 of any one of SEQ ID NOs: 12-126.

As used herein, the term "TALEN" refers to an endonuclease comprising a DNA-binding domain comprising 16-22 TAL domain repeats fused to any portion of the FokI nuclease domain.

As used herein, the term "Compact TALEN" refers to an endonuclease comprising a DNA-binding domain with 16-22 TAL domain repeats fused in any orientation to any portion of the I-TevI homing endonuclease.

As used herein, the term "zinc finger nuclease" or "ZFN" refers to a chimeric endonuclease comprising a zinc finger DNA-binding domain fused to the nuclease domain of the FokI restriction enzyme. The zinc finger domain can be redesigned through rational or experimental means to produce a protein which binds to a pre-determined DNA sequence ~18 basepairs in length, comprising a pair of nine basepair half-sites separated by 2-10 basepairs. Cleavage by a zinc finger nuclease can create a blunt end or a 5' overhand of variable length (frequently four basepairs).

As used herein, the term "CRISPR" refers to a caspase-based endonuclease comprising a caspase, such as Cas9, and a guide RNA that directs DNA cleavage of the caspase by hybridizing to a recognition site in the genomic DNA.

As used herein, the term "megaTAL" refers to a single-chain nuclease comprising a transcription activator-like effector (TALE) DNA binding domain with an engineered, sequence-specific homing endonuclease.

As used herein, with respect to a protein, the term "recombinant" means having an altered amino acid sequence as a result of the application of genetic engineering techniques to nucleic acids which encode the protein, and cells or organisms which express the protein. With respect to a nucleic acid, the term "recombinant" means having an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In accordance with this definition, a protein having an amino acid sequence identical to a naturally-occurring protein, but produced by cloning and expression in a heterologous host, is not considered recombinant.

As used herein, the term "wild-type" refers to the most common naturally occurring allele (*i.e.*, polynucleotide sequence) in the allele population of the same type of gene, wherein a polypeptide encoded by the wild-type allele has its original functions. The term "wild-type" also refers a polypeptide encoded by a wild-type allele. Wild-type alleles *(i.e.,* polynucleotides) and polypeptides are distinguishable from mutant or variant alleles and polypeptides, which comprise one or more mutations and/or substitutions relative to the wild-type sequence(s). Whereas a wild-type allele or polypeptide can confer a normal phenotype in an organism, a mutant or variant allele or polypeptide can, in some instances, confer an altered phenotype. Wild-type nucleases are distinguishable from recombinant or non-naturally-occurring nucleases.

As used herein with respect to recombinant proteins, the term "modification" means any insertion, deletion or substitution of an amino acid residue in the recombinant sequence relative to a reference sequence *(e.g.,* a wild-type or a native sequence).

As used herein, the term "recognition sequence" refers to a DNA sequence that is bound and cleaved by an endonuclease. In the case of a meganuclease, a recognition sequence comprises a pair of inverted, 9 base pair "half sites" which are separated by four basepairs. In the case of a single-chain meganuclease, the N-terminal domain of the protein contacts a first half-site and the C-terminal domain of the protein contacts a second half-site. Cleavage by a meganuclease produces four base pair 3' "overhangs". "Overhangs", or "sticky ends" are short, single-stranded DNA segments that can be produced by endonuclease cleavage of a double-stranded DNA sequence. In the case of meganucleases and single-chain meganucleases derived from I-CreI, the overhang comprises bases 10-13 of the 22 base pair recognition sequence. In the case of a Compact TALEN, the recognition sequence comprises a first CNNNGN sequence that is recognized by the I-TevI domain, followed by a nonspecific spacer 4-16 basepairs in length, followed by a second sequence 16-22 bp in length that is recognized by the TAL-effector domain (this sequence typically has a 5' T base). Cleavage by a Compact TALEN produces two base pair 3' overhangs. In the case of a CRISPR, the recognition sequence is the sequence, typically 16-24 basepairs, to which the guide RNA binds to direct Cas9 cleavage. Cleavage by a CRISPR produced blunt ends.

As used herein, the term "target site" or "target sequence" refers to a region of the chromosomal DNA of a cell comprising a recognition sequence for a nuclease.

As used herein, the term "DNA-binding affinity" or "binding affinity" means the tendency of a meganuclease to non-covalently associate with a reference DNA molecule *(e.g.,* a recognition sequence or an arbitrary sequence). Binding affinity is measured by a dissociation constant, K_{d}. As used herein, a nuclease has "altered" binding affinity if the K_{d} of the nuclease for a reference recognition sequence is increased or decreased by a statistically significant (p<0.05) amount relative to a reference nuclease.

As used herein, the term "homologous recombination" or "HR" refers to the natural, cellular process in which a double-stranded DNA-break is repaired using a homologous DNA sequence as the repair template (see, *e.g.* Cahill et al. (2006), Front. Biosci. 11:1958-1976). The homologous DNA sequence is an endogenous chromosomal sequence or an exogenous nucleic acid that was delivered to the cell.

As used herein, the term "non-homologous end-joining" or "NHEJ" refers to the natural, cellular process in which a double-stranded DNA-break is repaired by the direct joining of two non-homologous DNA segments (see, *e.g.* Cahill et al. (2006), Front. Biosci. 11:1958-1976). DNA repair by non-homologous end-joining is error-prone and frequently results in the untemplated addition or deletion of DNA sequences at the site of repair. In some instances, cleavage at a target recognition sequence results in NHEJ at a target recognition site. Nuclease-induced cleavage of a target site in the coding sequence of a gene followed by DNA repair by NHEJ can introduce mutations into the coding sequence, such as frameshift mutations, that disrupt gene function. Thus, engineered nucleases can be used to effectively knock-out a gene in a population of cells.

As used herein, a "chimeric antigen receptor" or "CAR" refers to an engineered receptor that grafts specificity for an antigen onto an immune effector cell *(e.g.,* a human T cell). A chimeric antigen receptor typically comprises an extracellular ligand-binding domain or moiety and an intracellular domain that comprises one or more stimulatory domains. In some embodiments, the extracellular ligand-binding domain or moiety can be in the form of single-chain variable fragments (scFvs) derived from a monoclonal antibody, which provide specificity for a particular epitope or antigen *(e.g.,* an epitope or antigen preferentially present on the surface of a cancer cell or other disease-causing cell or particle). The scFvs can be attached via a linker sequence. The extracellular ligand-binding domain can be specific for any antigen or epitope of interest. In a particular embodiment, the ligand-binding domain is specific for CD19. In other embodiments, the scFvs can be humanized or fully human. The extracellular domain of a chimeric antigen receptor can also comprise an autoantigen (see, Payne et al. (2016) Science, Vol. 353 (6295): 179-184), which can be recognized by autoantigen-specific B cell receptors on B lymphocytes, thus directing T cells to specifically target and kill autoreactive B lymphocytes in antibody-mediated autoimmune diseases. Such CARs can be referred to as chimeric autoantibody receptors (CAARs), and their use is encompassed by the disclosure.

In some non-limiting embodiments, the extracellular ligand-binding domain of the CAR can have specificity for a tumor-associated surface antigen, such as CD19, CD123, CD22, CS1, CD20, ErbB2 (HER2/neu), FLT3R, carcinoembryonic antigen (CEA), epithelial cell adhesion molecule (EpCAM), epidermal growth factor receptor (EGFR), EGFR variant III (EGFRvlll), CD30, CD40, CD44, CD44v6, disialoganglioside GD2, ductal-epithelial mucine, gp36, TAG-72, glycosphingolipids, glioma-associated antigen, B-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostase specific antigen (PSA), PAP, NY-ESO-1, LAGA-la, p53, prostein, PSMA, surviving and telomerase, prostate-carcinoma tumor antigen-1 (PCTA-1), MAGE, ELF2M, neutrophil elastase, ephrin B2, insulin growth factor (IGFl)-l, IGF-II, IGFI receptor, mesothelin, a major histocompatibility complex (MHC) molecule presenting a tumor-specific peptide epitope, 5T4, ROR1, Nkp30, NKG2D, tumor stromal antigens, the extra domain A (EDA) and extra domain B (EDB) of fibronectin and the Al domain of tenascin-C (TnC Al) and fibroblast associated protein (fap); a lineage-specific or tissue specific antigen such as CD3, CD4, CD8, CD24, CD25, CD33, CD34, CD133, CD138, CTLA-4, B7- 1 (CD80), B7-2 (CD86), endoglin, a major histocompatibility complex (MHC) molecule, BCMA (CD269, TNFRSF 17), or a virus-specific surface antigen such as an HIV-specific antigen (such as HIV gpl20); an EBV-specific antigen, a CMV-specific antigen, a HPV-specific antigen, a Lasse Virus-specific antigen, an Influenza Virus-specific antigen, as well as any derivate or variant of these surface markers. In a particular embodiment of the invention, the ligand-binding domain is specific for CD19.

The intracellular stimulatory domain can include one or more cytoplasmic signaling domains which transmit an activation signal to the immune effector cell following antigen binding. The intracellular stimulatory domain can be any intracellular stimulatory domain of interest. Such cytoplasmic signaling domains can include, without limitation, CD3-zeta. The intracellular stimulatory domain can also include one or more intracellular co-stimulatory domains which transmit a proliferative and/or cell-survival signal after ligand binding. The intracellular co-stimulatory domain can be any intracellular co-stimulatory domain of interest. Such intracellular co-stimulatory domains can include, without limitation, a CD28 domain, a 4-1BB domain, an OX40 domain, or a combination thereof. A chimeric antigen receptor can further include additional structural elements, including a transmembrane domain which is attached to the extracellular ligand-binding domain via a hinge or spacer sequence.

As used herein, an "exogenous T cell receptor" or "exogenous TCR" refers to a TCR whose sequence is introduced into the genome of an immune effector cell *(e.g.,* a human T cell) that may or may not endogenously express the TCR. Expression of an exogenous TCR on an immune effector cell can confer specificity for a specific epitope or antigen (e.g., an epitope or antigen preferentially present on the surface of a cancer cell or other diseasecausing cell or particle). Such exogenous T cell receptors can comprise alpha and beta chains or, alternatively, may comprise gamma and delta chains. Exogenous TCRs useful in the disclosure may have specificity to any antigen or epitope of interest.

As used herein, the term "reduced" refers to any reduction in the expression of an endogenous polypeptide (*e.g*., beta-2 microglobulin, an endogenous T cell receptor, etc.) at the cell surface of a genetically-modified cell or when compared to a control cell. The term "reduced" can also refer to a reduction in the percentage of cells in a population of cells that express an endogenous polypeptide at the cell surface when compared to a population of control cells. In either case, such a reduction is up to 5%, 10%, 20%, 30%, 40%, 50%, 60%, 70%, 80%, 90%, 95%, or up to 100%. Accordingly, the term "reduced" encompasses both a partial knockdown and a complete knockdown of an endogenous polypeptide.

As used herein with respect to both amino acid sequences and nucleic acid sequences, the terms "percent identity," "sequence identity," "percentage similarity," "sequence similarity," and the like, refer to a measure of the degree of similarity of two sequences based upon an alignment of the sequences which maximizes similarity between aligned amino acid residues or nucleotides, and which is a function of the number of identical or similar residues or nucleotides, the number of total residues or nucleotides, and the presence and length of gaps in the sequence alignment. A variety of algorithms and computer programs are available for determining sequence similarity using standard parameters. As used herein, sequence similarity is measured using the BLASTp program for amino acid sequences and the BLASTn program for nucleic acid sequences, both of which are available through the National Center for Biotechnology Information (www.ncbi.nlm.nih.gov), and are described in, for example, Altschul et al. (1990), J. Mol. Biol. 215:403-410; Gish and States (1993), Nature Genet. 3:266-272; Madden et al. (1996), Meth. Enzymol.266:131-141; Altschul et al. (1997), Nucleic Acids Res. 25:33 89-3402); Zhang et al. (2000), J. Comput. Biol. 7(1-2):203-14. As used herein, percent similarity of two amino acid sequences is the score based upon the following parameters for the BLASTp algorithm: word size=3; gap opening penalty=-11; gap extension penalty=-1; and scoring matrix=BLOSUM62. As used herein, percent similarity of two nucleic acid sequences is the score based upon the following parameters for the BLASTn algorithm: word size=11; gap opening penalty=-5; gap extension penalty=-2; match reward=1; and mismatch penalty=-3.

As used herein with respect to modifications of two proteins or amino acid sequences, the term "corresponding to" is used to indicate that a specified modification in the first protein is a substitution of the same amino acid residue as in the modification in the second protein, and that the amino acid position of the modification in the first proteins corresponds to or aligns with the amino acid position of the modification in the second protein when the two proteins are subjected to standard sequence alignments (e.g., using the BLASTp program). Thus, the modification of residue "X" to amino acid "A" in the first protein will correspond to the modification of residue "Y" to amino acid "A" in the second protein if residues X and Y correspond to each other in a sequence alignment, and despite the fact that X and Y are different numbers.

As used herein, the term "recognition half-site," "recognition sequence half-site," or simply "half-site" means a nucleic acid sequence in a double-stranded DNA molecule which is recognized by a monomer of a homodimeric or heterodimeric meganuclease, or by one subunit of a single-chain meganuclease.

As used herein, the term "hypervariable region" refers to a localized sequence within a meganuclease monomer or subunit that comprises amino acids with relatively high variability. A hypervariable region can comprise about 50-60 contiguous residues, about 53-57 contiguous residues, or preferably about 56 residues. In some embodiments, the residues of a hypervariable region may correspond to positions 24-79 or positions 215-270 of any one of SEQ ID NOs: 12-126. A hypervariable region can comprise one or more residues that contact DNA bases in a recognition sequence and can be modified to alter base preference of the monomer or subunit. A hypervariable region can also comprise one or more residues that bind to the DNA backbone when the meganuclease associates with a double-stranded DNA recognition sequence. Such residues can be modified to alter the binding affinity of the meganuclease for the DNA backbone and the target recognition sequence. In different embodiments of the disclosure, a hypervariable region may comprise between about 1-21 residues that exhibit variability and can be modified to influence base preference and/or DNA-binding affinity. In some embodiments, variable residues within a hypervariable region correspond to one or more of positions 24, 26, 28, 29, 30, 32, 33, 38, 40, 42, 44, 46, 48, 66, 68, 69, 70, 72, 73, 75, and 77 of any one of SEQ ID NOs: 12-126. In other embodiments, variable residues within a hypervariable region correspond to one or more of positions 215, 217, 219, 220, 221, 223, 224, 229, 231, 233, 235, 237, 239, 248, 257, 259, 260, 261, 263, 264, 266, and 268 of any one of SEQ ID NOs: 12-126.

As used herein, the terms "human beta-2 microglobulin gene," "B2M gene," and the like, are used interchangeably and refer to the human gene identified by NCBI Gene ID NO. 567 (Accession No. NG_012920.1), which is set forth in SEQ ID NO: 1, as well as naturally-occurring variants of the human beta-2 microglobulin gene which encode a functional B2M polypeptide.

As used herein, the terms "T cell receptor alpha constant region gene," "TCR alpha constant region gene," and the like, are used interchangeably and refer to the human gene identified by NCBI Gene ID NO. 28755, which is set forth in SEQ ID NO:127, as well as naturally-occurring variants of the T cell receptor alpha constant region gene which encode a functional polypeptide.

The terms "recombinant DNA construct," "recombinant construct," "expression cassette," "expression construct," "chimeric construct," "construct," and "recombinant DNA fragment" are used interchangeably herein and are nucleic acid fragments. A recombinant construct comprises an artificial combination of nucleic acid fragments, including, without limitation, regulatory and coding sequences that are not found together in nature. For example, a recombinant DNA construct may comprise regulatory sequences and coding sequences that are derived from different sources, or regulatory sequences and coding sequences derived from the same source and arranged in a manner different than that found in nature. Such a construct is used by itself or used in conjunction with a vector.

As used herein, a "vector" or "recombinant DNA vector" is a construct that includes a replication system and sequences that are capable of transcription and translation of a polypeptide-encoding sequence in a given host cell. If a vector is used then the choice of vector is dependent upon the method that will be used to transform host cells as is well known to those skilled in the art. Vectors can include, without limitation, plasmid vectors and recombinant AAV vectors, or any other vector known in that art suitable for delivering a gene encoding a meganuclease of the disclosure to a target cell. The skilled artisan is well aware of the genetic elements that must be present on the vector in order to successfully transform, select and propagate host cells comprising any of the isolated nucleotides or nucleic acid sequences of the disclosure.

As used herein, a "vector" can also refer to a viral vector. Viral vectors can include, without limitation, retroviral vectors, lentiviral vectors, adenoviral vectors, and adeno-associated viral vectors (AAV).

As used herein, a "polycistronic" mRNA refers to a single messenger RNA which comprises two or more coding sequences *(i.e.,* cistrons) and encodes more than one protein. A polycistronic mRNA can comprise any element known in the art to allow for the translation of two or more genes from the same mRNA molecule including, but not limited to, an IRES element, a T2A element, a P2A element, an E2A element, and an F2A element.

As used herein, a "human T cell" or "T cell" refers to a T cell isolated from a human donor. Human T cells, and cells derived therefrom, include isolated T cells that have not been passaged in culture, T cells that have been passaged and maintained under cell culture conditions without immortalization, and T cells that have been immortalized and is maintained under cell culture conditions indefinitely.

As used herein, a "control" or "control cell" refers to a cell that provides a reference point for measuring changes in genotype or phenotype of a genetically-modified cell. A control cell may comprise, for example: (a) a wild-type cell, *i.e.,* of the same genotype as the starting material for the genetic alteration which resulted in the genetically-modified cell; (b) a cell of the same genotype as the genetically-modified cell but which has been transformed with a null construct *(i.e.,* with a construct which has no known effect on the trait of interest); or, (c) a cell genetically identical to the genetically-modified cell but which is not exposed to conditions, stimuli, or further genetic modifications that would induce expression of altered genotype or phenotype.

As used herein, the recitation of a numerical range for a variable is intended to convey that the disclosure is practiced with the variable equal to any of the values within that range. Thus, for a variable which is inherently discrete, the variable is equal to any integer value within the numerical range, including the end-points of the range. Similarly, for a variable which is inherently continuous, the variable is equal to any real value within the numerical range, including the end-points of the range. As an example, and without limitation, a variable which is described as having values between 0 and 2 can take the values 0, 1 or 2 if the variable is inherently discrete, and can take the values 0.0, 0.1, 0.01, 0.001, or any other real values ≧0 and ≦2 if the variable is inherently continuous.

### 2.1 Principle of the Invention

The present disclosure is based, in part, on the hypothesis that engineered nucleases is utilized to recognize and cleave recognition sequences found within the human beta-2 microglobulin gene (SEQ ID NO:1), such that NHEJ at the cleavage site disrupts expression of the beta-2 microglobulin polypeptide at the cell-surface, thus interfering with assembly and activation of endogenous MHC class I receptors encoded by HLA genes. Moreover, according to the disclosure, an exogenous polynucleotide sequence is inserted into the beta-2 microglobulin gene at the nuclease cleavage site, for example by homologous recombination. In some embodiments, the polynucleotide sequence comprises a sequence of interest that is concurrently expressed in the cell. Moreover, the engineered nucleases of the disclosure is used to knockout cell-surface expression of beta-2 microglobulin in eukaryotic cells that are genetically-modified to exhibit one or more additional knockouts (e.g., knockout of an endogenous T cell receptor) and/or genetically-modified to express one or more polypeptides of interest *(e.g.,* a chimeric antigen receptor or exogenous T cell receptor). Thus, in a preferred embodiment, the present disclosure allows for the production of genetically-modified eukaryotic cell, such as a T cell, that exhibits knockout of both beta-2 microglobulin and an endogenous T cell receptor at the cell surface, while concurrently expressing a chimeric antigen receptor or exogenous T cell receptor. Such cells can exhibit reduced alloreactivity and/or reduced allogenicity when administered to a subject.

### 2.2 Nucleases for Recognizing and Cleaving Recognition Sequences Within the Human

### Beta-2 Microglobulin Gene

It is known in the art that it is possible to use a site-specific nuclease to make a DNA break in the genome of a living cell, and that such a DNA break can result in permanent modification of the genome via mutagenic NHEJ repair or via homologous recombination with a transgenic DNA sequence. NHEJ can produce mutagenesis at the cleavage site, resulting in inactivation of the allele. NHEJ-associated mutagenesis may inactivate an allele via generation of early stop codons, frameshift mutations producing aberrant non-functional proteins, or could trigger mechanisms such as nonsense-mediated mRNA decay. The use of nucleases to induce mutagenesis via NHEJ can be used to target a specific mutation or a sequence present in a wild-type allele. The use of nucleases to induce a double-strand break in a target locus is known to stimulate homologous recombination, particularly of transgenic DNA sequences flanked by sequences that are homologous to the genomic target. In this manner, exogenous nucleic acid sequences can be inserted into a target locus. Such exogenous nucleic acids can encode, for example, a chimeric antigen receptor, an exogenous TCR, or any sequence or polypeptide of interest.

In different embodiments, a variety of different types of nuclease are useful for practicing the disclosure. In one embodiment, the disclosure is practiced using recombinant meganucleases. In another embodiment, the disclosure is practiced using a CRISPR nuclease or CRISPR Nickase. Methods for making CRISPRs and CRISPR Nickases that recognize pre-determined DNA sites are known in the art, for example Ran, et al. (2013) Nat Protoc. 8:2281-308. In another embodiment, the disclosure is practiced using TALENs or Compact TALENs. Methods for making TALE domains that bind to pre-determined DNA sites are known in the art, for example Reyon et al. (2012) Nat Biotechnol. 30:460-5. In a further embodiment, the disclosure is practiced using megaTALs.

In preferred embodiments, the nucleases used to practice the disclosure are single-chain meganucleases. A single-chain meganuclease comprises an N-terminal subunit and a C-terminal subunit joined by a linker peptide. Each of the two domains recognizes half of the recognition sequence *(i.e.,* a recognition half-site) and the site of DNA cleavage is at the middle of the recognition sequence near the interface of the two subunits. DNA strand breaks are offset by four base pairs such that DNA cleavage by a meganuclease generates a pair of four base pair, 3' single-strand overhangs.

In some examples, recombinant meganucleases of the disclosure have been engineered to recognize and cleave the B2M 13-14 recognition sequence (SEQ ID NO:2). Such recombinant meganucleases are collectively referred to herein as "B2M 13-14 meganucleases." Exemplary B2M 13-14 meganucleases are provided in SEQ ID NOs:12-100.

In other examples, recombinant meganucleases of the disclosure have been engineered to recognize and cleave the B2M 5-6 recognition sequence (SEQ ID NO:4). Such recombinant meganucleases are collectively referred to herein as "B2M 5-6 meganucleases." Exemplary B2M 5-6 meganucleases are provided in SEQ ID NOs: 101-113.

In additional examples, recombinant meganucleases of the disclosure have been engineered to recognize and cleave the B2M 7-8 recognition sequence (SEQ ID NO:6). Such recombinant meganucleases are collectively referred to herein as "B2M 7-8 meganucleases." Exemplary B2M 7-8 meganucleases are provided in SEQ ID NOs: 114-124.

In further examples, recombinant meganucleases of the disclosure have been engineered to recognize and cleave the B2M 11-12 recognition sequence (SEQ ID NO:8). Such recombinant meganucleases are collectively referred to herein as "B2M 11-12 meganucleases." Exemplary B2M 11-12 meganucleases are provided in SEQ ID NOs:125 and 126.

Recombinant meganucleases of the disclosure comprise a first subunit, comprising a first hypervariable (HVR1) region, and a second subunit, comprising a second hypervariable (HVR2) region. Further, the first subunit binds to a first recognition half-site in the recognition sequence (e.g., the B2M13, B2M5, B2M7, or B2M11 half-site), and the second subunit binds to a second recognition half-site in the recognition sequence (e.g., the B2M14, B2M6, B2M8, or B2M12 half-site). In embodiments where the recombinant meganuclease is a single-chain meganuclease, the first and second subunits is oriented such that the first subunit, which comprises the HVR1 region and binds the first half-site, is positioned as the N-terminal subunit, and the second subunit, which comprises the HVR2 region and binds the second half-site, is positioned as the C-terminal subunit. In alternative embodiments, the first and second subunits is oriented such that the first subunit, which comprises the HVR1 region and binds the first half-site, is positioned as the C-terminal subunit, and the second subunit, which comprises the HVR2 region and binds the second half-site, is positioned as the N-terminal subunit. Exemplary B2M 13-14 meganucleases of the disclosure are provided in Table 1. Exemplary B2M 5-6 meganucleases of the disclosure are provided in Table 2. Exemplary B2M 7-8 meganucleases of the disclosure are provided in Table 3. Exemplary B2M 11-12 meganucleases of the disclosure are provided in Table 4.

**Table 1. Exemplary recombinant meganucleases engineered to recognize and cleave the B2M 13-14 recognition sequence (SEQ ID NO:2)**

| **Meganuclease** | **AA SEQ ID** | **B2M13 Subunit Residues** | **B2M13 Subunit SEQ ID** | ***B2M13 Subunit %** | **B2M14 Subunit Residues** | **B2M14 Subunit SEQ ID** | ***B2M14 Subunit %** |
|---|---|---|---|---|---|---|---|
| B2M 13-14x.479 | 12 | 198-344 | 132 | 100 | 7-153 | 221 | 100 |
| B2M 13-14x.287 | 13 | 198-344 | 133 | 95.92 | 7-153 | 222 | 99.32 |
| B2M 13-14x.377 | 14 | 198-344 | 134 | 94.56 | 7-153 | 223 | 97.96 |
| B2M 13.14x.169 | 15 | 198-344 | 135 | 95.24 | 7-153 | 224 | 99.32 |
| B2M 13-14x.202 | 16 | 198-344 | 136 | 95.24 | 7-153 | 225 | 97.96 |
| B2M 13-14x.93 | 17 | 198-344 | 137 | 94.56 | 7-153 | 226 | 95.92 |
| B2M 13-14x.93 QE | 18 | 198-344 | 138 | 95.24 | 7-153 | 227 | 95.92 |
| B2M 13-14x.93 EQ | 19 | 198-344 | 139 | 94.56 | 7-153 | 228 | 96.6 |
| B2M 13-14x.93 EE | 20 | 198-344 | 140 | 95.24 | 7-153 | 229 | 96.6 |
| B2M 13-14x.93 QQY66 | 21 | 198-344 | 141 | 94.56 | 7-153 | 230 | 95.92 |
| B2M 13-14x.93 QQK66 | 22 | 198-344 | 142 | 94.56 | 7-153 | 231 | 95.24 |
| B2M 13-14x.93 QQR66 | 23 | 198-344 | 143 | 94.56 | 7-153 | 232 | 95.24 |
| B2M 13-14x.93 EEY66 | 24 | 198-344 | 144 | 95.24 | 7-153 | 233 | 96.6 |
| B2M 13-14x.93 EEK66 | 25 | 198-344 | 145 | 95.24 | 7-153 | 234 | 95.92 |
| B2M 13-14x.93 EER66 | 26 | 198-344 | 146 | 95.24 | 7-153 | 235 | 95.92 |
| B2M 13-14x.93 EQY66 | 27 | 198-344 | 147 | 94.56 | 7-153 | 236 | 95.92 |
| B2M 13-14x.93 EQK66 | 28 | 198-344 | 148 | 94.56 | 7-153 | 237 | 95.92 |
| B2M 13-14x.93 EQR66 | 29 | 198-344 | 149 | 94.56 | 7-153 | 238 | 95.92 |
| B2M 13-14x.3 | 30 | 198-344 | 150 | 91.84 | 7-153 | 239 | 92.52 |
| B2M 13-14x.10 | 31 | 198-344 | 151 | 94.56 | 7-153 | 240 | 93.2 |
| B2M 13-14x.14 | 32 | 198-344 | 152 | 91.84 | 7-153 | 241 | 95.24 |
| B2M 13-14x.22 | 33 | 198-344 | 153 | 93.88 | 7-153 | 242 | 93.2 |
| B2M 13-14x.67 | 34 | 198-344 | 154 | 94.56 | 7-153 | 243 | 93.2 |
| B2M 13-14x.84 | 35 | 198-344 | 155 | 93.88 | 7-153 | 244 | 94.56 |
| B2M 13-14x.85 | 36 | 198-344 | 156 | 94.56 | 7-153 | 245 | 93.2 |
| B2M 13-14x.96 | 37 | 198-344 | 157 | 95.24 | 7-153 | 246 | 96.6 |
| B2M 13-14x.97 | 38 | 198-344 | 158 | 95.24 | 7-153 | 247 | 98.64 |
| B2M 13-14x.102 | 39 | 198-344 | 159 | 95.24 | 7-153 | 248 | 98.64 |
| B2M 13.14x.105 | 40 | 198-344 | 160 | 95.24 | 7-153 | 249 | 98.64 |
| B2M 13-14x.106 | 41 | 198-344 | 161 | 95.24 | 7-153 | 250 | 97.96 |
| B2M 13-14x.115 | 42 | 198-344 | 162 | 95.24 | 7-153 | 251 | 98.64 |
| B2M 13-14x.139 | 43 | 198-344 | 163 | 95.24 | 7-153 | 252 | 95.92 |
| B2M 13-14x.141 | 44 | 198-344 | 164 | 95.24 | 7-153 | 253 | 97.96 |
| B2M 13-14x.146 | 45 | 198-344 | 165 | 95.24 | 7-153 | 254 | 96.6 |
| B2M 13-14x.162 | 46 | 198-344 | 166 | 95.24 | 7-153 | 255 | 97.96 |
| B2M 13.14x.165 | 47 | 198-344 | 167 | 95.24 | 7-153 | 256 | 99.32 |
| B2M 13-14x.178 | 48 | 198-344 | 168 | 95.24 | 7-153 | 257 | 99.32 |
| B2M 13-14x.182 | 49 | 198-344 | 169 | 95.24 | 7-153 | 258 | 98.64 |
| B2M 13-14x.198 | 50 | 198-344 | 170 | 95.24 | 7-153 | 259 | 98.64 |
| B2M 13-14x.199 | 51 | 198-344 | 171 | 95.24 | 7-153 | 260 | 97.96 |
| B2M 13-14x.207 | 52 | 198-344 | 172 | 95.24 | 7-153 | 261 | 96.6 |
| B2M 13-14x.222 | 53 | 198-344 | 173 | 95.24 | 7-153 | 262 | 98.64 |
| B2M 13-14x.245 | 54 | 198-344 | 174 | 95.24 | 7-153 | 263 | 99.32 |
| B2M 13-14x.255 | 55 | 198-344 | 175 | 95.24 | 7-153 | 264 | 99.32 |
| B2M 13-14x.259 | 56 | 198-344 | 176 | 95.24 | 7-153 | 265 | 97.96 |
| B2M 13-14x.275 | 57 | 198-344 | 177 | 95.24 | 7-153 | 266 | 100 |
| B2M 13-14x.280 | 58 | 198-344 | 178 | 95.92 | 7-153 | 267 | 99.32 |
| B2M 13-14x.281 | 59 | 198-344 | 179 | 94.56 | 7-153 | 268 | 99.32 |
| B2M 13-14x.283 | 60 | 198-344 | 180 | 94.56 | 7-153 | 269 | 99.32 |
| B2M 13-14x.285 | 61 | 198-344 | 181 | 95.24 | 7-153 | 270 | 99.32 |
| B2M 13-14x.286 | 62 | 198-344 | 182 | 94.56 | 7-153 | 271 | 99.32 |
| B2M 13-14x.295 | 63 | 198-344 | 183 | 96.6 | 7-153 | 272 | 99.32 |
| B2M 13-14x.301 | 64 | 198-344 | 184 | 95.24 | 7-153 | 273 | 99.32 |
| B2M 13-14x.306 | 65 | 198-344 | 185 | 95.24 | 7-153 | 274 | 99.32 |
| B2M 13-14x.317 | 66 | 198-344 | 186 | 94.56 | 7-153 | 275 | 99.32 |
| B2M 13-14x.325 | 67 | 198-344 | 187 | 95.24 | 7-153 | 276 | 99.32 |
| B2M 13-14x.335 | 68 | 198-344 | 188 | 94.56 | 7-153 | 277 | 99.32 |
| B2M 13-14x.338 | 69 | 198-344 | 189 | 95.24 | 7-153 | 278 | 99.32 |
| B2M 13-14x.347 | 70 | 198-344 | 190 | 95.24 | 7-153 | 279 | 99.32 |
| B2M 13-14x.361 | 71 | 198-344 | 191 | 95.24 | 7-153 | 280 | 99.32 |
| B2M 13-14x.362 | 72 | 198-344 | 192 | 94.56 | 7-153 | 281 | 99.32 |
| B2M 13-14x.365 | 73 | 198-344 | 193 | 95.24 | 7-153 | 282 | 99.32 |
| B2M 13-14x.369 | 74 | 198-344 | 194 | 95.24 | 7-153 | 283 | 99.32 |
| B2M 13-14x.371 | 75 | 198-344 | 195 | 94.56 | 7-153 | 284 | 99.32 |
| B2M 13-14x.372 | 76 | 198-344 | 196 | 95.24 | 7-153 | 285 | 99.32 |
| B2M 13-14x.375 | 77 | 198-344 | 197 | 95.92 | 7-153 | 286 | 97.96 |
| B2M 13-14x.378 | 78 | 198-344 | 198 | 95.92 | 7-153 | 287 | 97.96 |
| B2M 13-14x.385 | 79 | 198-344 | 199 | 95.92 | 7-153 | 288 | 97.96 |
| B2M 13-14x.392 | 80 | 198-344 | 200 | 94.56 | 7-153 | 289 | 97.96 |
| B2M 13-14x.432 | 81 | 198-344 | 201 | 96.6 | 7-153 | 290 | 97.96 |
| B2M 13-14x.433 | 82 | 198-344 | 202 | 94.56 | 7-153 | 291 | 97.96 |
| B2M 13-14x.440 | 83 | 198-344 | 203 | 94.56 | 7-153 | 292 | 97.96 |
| B2M 13-14x.449 | 84 | 198-344 | 204 | 94.56 | 7-153 | 293 | 97.96 |
| B2M 13-14x.456 | 85 | 198-344 | 205 | 94.56 | 7-153 | 294 | 97.96 |
| B2M 13-14x.457 | 86 | 198-344 | 206 | 95.92 | 7-153 | 295 | 97.96 |
| B2M 13-14x.459 | 87 | 198-344 | 207 | 95.24 | 7-153 | 296 | 97.96 |
| B2M 13-14x.464 | 88 | 198-344 | 208 | 96.6 | 7-153 | 297 | 97.96 |
| B2M 13-14x.465 | 89 | 198-344 | 209 | 96.6 | 7-153 | 298 | 97.96 |
| B2M 13-14x.470 | 90 | 198-344 | 210 | 94.56 | 7-153 | 299 | 100 |
| B2M 13-14x.471 | 91 | 198-344 | 211 | 96.6 | 7-153 | 300 | 100 |
| B2M 13-14x.540 | 92 | 198-344 | 212 | 95.92 | 7-153 | 301 | 100 |
| B2M 13-14x.543 | 93 | 198-344 | 213 | 94.56 | 7-153 | 302 | 100 |
| B2M 13-14x.551 | 94 | 198-344 | 214 | 94.56 | 7-153 | 303 | 100 |
| B2M 13-14x.554 | 95 | 198-344 | 215 | 95.92 | 7-153 | 304 | 100 |
| B2M 13-14x.556 | 96 | 198-344 | 216 | 94.56 | 7-153 | 305 | 100 |
| B2M 13-14x.76 | 97 | 7-153 | 217 | 91.16 | 198-344 | 306 | 91.84 |
| B2M 13-14x.82 | 98 | 7-153 | 218 | 93.88 | 198-344 | 307 | 92.52 |
| B2M 13-14x.31 | 99 | 7-153 | 219 | 89.8 | 198-344 | 308 | 91.84 |
| B2M 13-14x.32 | 100 | 7-153 | 220 | 93.88 | 198-344 | 309 | 94.56 |

| | | | | | | | |
|---|---|---|---|---|---|---|---|
| *"B2M13 Subunit %" and "B2M14 Subunit %" represent the amino acid sequence identity between the B2M13-binding and B2M14-binding subunit regions of each meganuclease and the B2M13-binding and B2M14-binding subunit regions, respectively, of the B2M 13-14x.479 meganuclease. | | | | | | | |

**Table 2. Exemplary recombinant meganucleases engineered to recognize and cleave the B2M 5-6 recognition sequence (SEQ ID NO:4)**

| **Meganuclease** | **AA SEQ ID** | **B2M5 Subunit Residues** | **B2M5 Subunit SEQ ID** | ***B2M5 Subunit %** | **B2M6 Subunit Residues** | **B2M6 Subunit SEQ ID** | ***B2M6 Subunit %** |
|---|---|---|---|---|---|---|---|
| B2M 5-6x.14 | 101 | 7-153 | 310 | 100 | 198-344 | 323 | 100 |
| B2M 5-6x.5 | 102 | 7-153 | 311 | 94.56 | 198-344 | 324 | 90.48 |
| B2M 5-6x.6 | 103 | 7-153 | 312 | 100 | 198-344 | 325 | 92.52 |
| B2M 5-6x.13 | 104 | 7-153 | 313 | 93.2 | 198-344 | 326 | 93.88 |
| B2M 5-6x.22 | 105 | 7-153 | 314 | 91.16 | 198-344 | 327 | 93.2 |
| B2M 5-6x.31 | 106 | 7-153 | 315 | 92.52 | 198-344 | 328 | 92.52 |
| B2M 5-6x.69 | 107 | 7-153 | 316 | 93.2 | 198-344 | 329 | 93.88 |
| B2M 5-6x.73 | 108 | 7-153 | 317 | 93.2 | 198-344 | 330 | 93.2 |
| B2M 5-6x.85 | 109 | 7-153 | 318 | 91.16 | 198-344 | 331 | 92.52 |
| B2M 5-6x.86 | 110 | 7-153 | 319 | 100 | 198-344 | 332 | 91.16 |
| B2M 5-6x.91 | 111 | 7-153 | 320 | 92.52 | 198-344 | 333 | 93.88 |
| B2M 5-6x.28 | 112 | 198-344 | 321 | 91.16 | 7-153 | 334 | 93.2 |
| B2M 5-6x.3 | 113 | 198-344 | 322 | 91.16 | 7-153 | 335 | 92.52 |

| | | | | | | | |
|---|---|---|---|---|---|---|---|
| ^{∗}"B2M5 Subunit %" and "B2M6 Subunit %" represent the amino acid sequence identity between the B2M5-binding and B2M6-binding subunit regions of each meganuclease and the B2M5-binding and B2M6-binding subunit regions, respectively, of the B2M 5-6x.14 meganuclease. | | | | | | | |

**Table 3. Exemplary recombinant meganucleases engineered to recognize and cleave the B2M 7-8 recognition sequence (SEQ ID NO:6)**

| **Meganuclease** | **AA SEQ ID** | **B2M7 Subunit Residues** | **B2M7 Subunit SEQ ID** | ***B2M7 Subunit %** | **B2M8 Subunit Residues** | **B2M8 Subunit SEQ ID** | ***B2M8 Subunit %** |
|---|---|---|---|---|---|---|---|
| B2M 7-8x.88 | 114 | 7-153 | 336 | 100 | 198-344 | 347 | 100 |
| B2M 7-8x.7 | 115 | 7-153 | 337 | 95.92 | 198-344 | 348 | 92.52 |
| B2M 7-8x.23 | 116 | 7-153 | 338 | 94.56 | 198-344 | 349 | 93.88 |
| B2M 7-8x.30 | 117 | 7-153 | 339 | 90.48 | 198-344 | 350 | 90.48 |
| B2M 7-8x.53 | 118 | 7-153 | 340 | 95.24 | 198-344 | 351 | 93.2 |
| B2M 7-8x.2 | 119 | 198-344 | 341 | 99.32 | 7-153 | 352 | 100 |
| B2M 7-8x.3 | 120 | 198-344 | 342 | 96.6 | 7-153 | 353 | 93.88 |
| B2M 7-8x.6 | 121 | 198-344 | 343 | 93.88 | 7-153 | 354 | 93.2 |
| B2M 7-8x.25 | 122 | 198-344 | 344 | 93.2 | 7-153 | 355 | 93.2 |
| B2M 7-8x.78 | 123 | 198-344 | 345 | 95.92 | 7-153 | 356 | 96.6 |
| B2M 7-8x.85 | 124 | 198-344 | 346 | 96.6 | 7-153 | 357 | 93.88 |

| | | | | | | | |
|---|---|---|---|---|---|---|---|
| ^{∗}"B2M7 Subunit %" and "B2M8 Subunit %" represent the amino acid sequence identity between the B2M7-binding and B2M8-binding subunit regions of each meganuclease and the B2M7-binding and B2M8-binding subunit regions, respectively, of the B2M 7-8x.88 meganuclease. | | | | | | | |

**Table 4. Exemplary recombinant meganucleases engineered to recognize and cleave the B2M 11-12 recognition sequence (SEQ ID NO:8)**

| **Meganuclease** | **AA SEQ ID** | **B2M11 Subunit Residues** | **B2M11 Subunit SEQ ID** | ***B2M11 Subunit %** | **B2M12 Subunit Residues** | **B2M12 Subunit SEQ ID** | ***B2M12 Subunit %** |
|---|---|---|---|---|---|---|---|
| B2M 11-12x.45 | 125 | 7-153 | 358 | 100 | 198-344 | 360 | 100 |
| B2M 11-12x.2 | 126 | 198-344 | 359 | 99 | 7-153 | 361 | 99 |

| | | | | | | | |
|---|---|---|---|---|---|---|---|
| ^{∗}"B2M11 Subunit %" and "B2M12 Subunit %" represent the amino acid sequence identity between the B2M11-binding and B2M12-binding subunit regions of each meganuclease and the B2M11-binding and B2M12-binding subunit regions, respectively, of the B2M 11-12x.45 meganuclease. | | | | | | | |

### 2.3 Methods for Producing Genetically-Modified Cells

The disclosure provides methods for producing genetically-modified cells using engineered nucleases that recognize and cleave recognition sequences found within the human beta-2 microglobulin gene (SEQ ID NO: 1). Cleavage at such recognition sequences can allow for NHEJ at the cleavage site and disrupted expression of the beta-2 microglobulin polypeptide, thus interfering with assembly and activation of endogenous MHC class I receptors encoded by HLA genes. Additionally, cleavage at such recognition sequences can further allow for homologous recombination of exogenous nucleic acid sequences directly into the beta-2 microglobulin gene.

In some aspects, the disclosure further provides methods for producing genetically-modified eukaryotic cells that have reduced cell-surface expression of an endogenous T cell receptor. Such methods utilize an endonuclease engineered to recognize and cleave a recognition sequence located in a gene encoding a component of an endogenous T cell receptor. Such a gene can include, without limitation, the gene encoding the human T cell receptor alpha constant region gene (SEQ ID NO: 127). Endonucleases useful in the method can include, without limitation, recombinant meganucleases, CRISPRs, TALENs, compact TALENs, zinc finger nucleases (ZFNs), megaTALs. In some embodiments, the endonuclease is a recombinant meganuclease, and the meganuclease recognition sequence comprises any one of SEQ ID NOs: 128-130. Recombinant meganucleases useful for recognizing and cleaving a recognition sequence in the human T cell receptor alpha constant region gene can include, without limitation, those disclosed in U.S. Application Nos. 62/237,382 and 62/237,394. Cleavage at TCR recognition sequences can allow for NHEJ at the cleavage site and disrupted expression of the endogenous T cell receptor. Additionally, cleavage at TCR recognition sequences can further allow for homologous recombination of exogenous nucleic acid sequences directly into targeted gene.

Engineered nucleases of the disclosure is delivered into a cell in the form of protein or, preferably, as a nucleic acid encoding the engineered nuclease. Such nucleic acid is DNA (e.g., circular or linearized plasmid DNA or PCR products) or RNA. For embodiments in which the engineered nuclease coding sequence is delivered in DNA form, it should be operably linked to a promoter to facilitate transcription of the meganuclease gene. Mammalian promoters suitable for the disclosure include constitutive promoters such as the cytomegalovirus early (CMV) promoter (Thomsen et al. (1984), Proc Natl Acad Sci USA. 81(3):659-63) or the SV40 early promoter (Benoist and Chambon (1981), Nature. 290(5804):304-10) as well as inducible promoters such as the tetracycline-inducible promoter (Dingermann et al. (1992), Mol Cell Biol. 12(9):4038-45).

In some embodiments, mRNA encoding the engineered nuclease is delivered to the cell because this reduces the likelihood that the gene encoding the engineered nuclease will integrate into the genome of the cell. Such mRNA encoding an engineered nuclease is produced using methods known in the art such as *in vitro* transcription. In some embodiments, the mRNA is capped using 7-methyl-guanosine. In some embodiments, the mRNA is polyadenylated.

In particular embodiments, an mRNA encoding an engineered nuclease of the disclosure is a polycistronic mRNA encoding two or more nucleases which are simultaneously expressed in the cell. A polycistronic mRNA can encode two or more nucleases of the disclosure which target different recognition sequences in the same target gene. Alternatively, a polycistronic mRNA can encode one or more nucleases of the disclosure and a second nuclease targeting a separate recognition sequence positioned in the same gene, or a second recognition sequence positioned in a second gene such that cleavage sites are produced in both genes. A polycistronic mRNA can comprise any element known in the art to allow for the translation of two genes (*i.e*., cistrons) from the same mRNA molecule including, but not limited to, an IRES element (*e.g.,* SEQ ID NO:362), a T2A element (*e.g.,* SEQ ID NO:363), a P2A element (*e.g.,* SEQ ID NO:364), an E2A element (*e.g.,* SEQ ID NO:365), and an F2A element (*e.g.,* SEQ ID NO:366). disclosure

Purified nuclease proteins can be delivered into cells to cleave genomic DNA, which allows for homologous recombination or non-homologous end-joining at the cleavage site with a sequence of interest, by a variety of different mechanisms known in the art.

In some embodiments, engineered nuclease proteins, or DNA/mRNA encoding engineered nucleases, are coupled to a cell penetrating peptide or targeting ligand to facilitate cellular uptake. Examples of cell penetrating peptides known in the art include poly-arginine (Jearawiriyapaisarn, et al. (2008) Mol Ther. 16:1624-9), TAT peptide from the HIV virus (Hudecz et al. (2005), Med. Res. Rev. 25: 679-736), MPG (Simeoni, et al. (2003) Nucleic Acids Res. 31:2717-2724), Pep-1 (Deshayes et al. (2004) Biochemistry 43: 7698-7706, and HSV-1 VP-22 (Deshayes et al. (2005) Cell Mol Life Sci. 62:1839-49. In an alternative embodiment, engineered nucleases, or DNA/mRNA encoding engineered nucleases, are coupled covalently or non-covalently to an antibody that recognizes a specific cell-surface receptor expressed on target cells such that the nuclease protein/DNA/mRNA binds to and is internalized by the target cells. Alternatively, engineered nuclease protein/DNA/mRNA is coupled covalently or non-covalently to the natural ligand (or a portion of the natural ligand) for such a cell-surface receptor. (McCall, et al. (2014) Tissue Barriers. 2(4):e944449; Dinda, et al. (2013) Curr Pharm Biotechnol. 14:1264-74; Kang, et al. (2014) Curr Pharm Biotechnol. 15(3):220-30; Qian et al. (2014) Expert Opin Drug Metab Toxicol. 10(11):1491-508).

In some embodiments, engineered nuclease proteins, or DNA/mRNA encoding engineered nucleases, are coupled covalently or, preferably, non-covalently to a nanoparticle or encapsulated within such a nanoparticle using methods known in the art (Sharma, et al. (2014) Biomed Res Int. 2014). A nanoparticle is a nanoscale delivery system whose length scale is <1 µm, preferably <100 nm. Such nanoparticles is designed using a core composed of metal, lipid, polymer, or biological macromolecule, and multiple copies of the recombinant meganuclease proteins, mRNA, or DNA is attached to or encapsulated with the nanoparticle core. This increases the copy number of the protein/mRNA/DNA that is delivered to each cell and, so, increases the intracellular expression of each engineered nuclease to maximize the likelihood that the target recognition sequences will be cut. The surface of such nanoparticles is further modified with polymers or lipids (e.g., chitosan, cationic polymers, or cationic lipids) to form a core-shell nanoparticle whose surface confers additional functionalities to enhance cellular delivery and uptake of the payload (Jian et al. (2012) Biomaterials. 33(30): 7621-30). Nanoparticles may additionally be advantageously coupled to targeting molecules to direct the nanoparticle to the appropriate cell type and/or increase the likelihood of cellular uptake. Examples of such targeting molecules include antibodies specific for cell-surface receptors and the natural ligands (or portions of the natural ligands) for cell surface receptors.

In some embodiments, the engineered nucleases or DNA/mRNA encoding the engineered nucleases, are encapsulated within liposomes or complexed using cationic lipids (see, *e.g.,* Lipofectamine^{™}, Life Technologies Corp., Carlsbad, CA; Zuris et al. (2015) Nat Biotechnol. 33: 73-80; Mishra et al. (2011) J Drug Deliv. 2011:863734). The liposome and lipoplex formulations can protect the payload from degradation, and facilitate cellular uptake and delivery efficiency through fusion with and/or disruption of the cellular membranes of the cells.

In some embodiments, engineered nuclease proteins, or DNA/mRNA encoding engineered nucleases, are encapsulated within polymeric scaffolds (e.g., PLGA) or complexed using cationic polymers *(e.g.,* PEI, PLL) (Tamboli et al. (2011) Ther Deliv. 2(4): 523-536).

In some embodiments, engineered nuclease proteins, or DNA/mRNA encoding engineered nucleases, are combined with amphiphilic molecules that self-assemble into micelles (Tong et al. (2007) J Gene Med. 9(11): 956-66). Polymeric micelles may include a micellar shell formed with a hydrophilic polymer (e.g., polyethyleneglycol) that can prevent aggregation, mask charge interactions, and reduce nonspecific interactions outside of the cell.

In some embodiments, engineered nuclease proteins, or DNA/mRNA encoding engineered nucleases, are formulated into an emulsion or a nanoemulsion *(i.e.,* having an average particle diameter of < 1nm) for delivery to the cell. The term "emulsion" refers to, without limitation, any oil-in-water, water-in-oil, water-in-oil-in-water, or oil-in-water-in-oil dispersions or droplets, including lipid structures that can form as a result of hydrophobic forces that drive apolar residues (e.g., long hydrocarbon chains) away from water and polar head groups toward water, when a water immiscible phase is mixed with an aqueous phase. These other lipid structures include, but are not limited to, unilamellar, paucilamellar, and multilamellar lipid vesicles, micelles, and lamellar phases. Emulsions are composed of an aqueous phase and a lipophilic phase (typically containing an oil and an organic solvent). Emulsions also frequently contain one or more surfactants. Nanoemulsion formulations are well known, *e.g*., as described in US Patent Application Nos. 2002/0045667 and 2004/0043041, and US Pat. Nos. 6,015,832, 6,506,803, 6,635,676, and 6,559,189, each of which is incorporated herein by reference in its entirety.

In some embodiments, engineered nuclease proteins, or DNA/mRNA encoding engineered nucleases, are covalently attached to, or non-covalently associated with, multifunctional polymer conjugates, DNA dendrimers, and polymeric dendrimers (Mastorakos et al. (2015) Nanoscale. 7(9): 3845-56; Cheng et al. (2008) J Pharm Sci. 97(1): 123-43). The dendrimer generation can control the payload capacity and size, and can provide a high payload capacity. Moreover, display of multiple surface groups is leveraged to improve stability and reduce nonspecific interactions.

In some embodiments, genes encoding an engineered nuclease are introduced into a cell using a viral vector. Such vectors are known in the art and include retroviral vectors, lentiviral vectors, adenoviral vectors, and adeno-associated virus (AAV) vectors (reviewed in Vannucci, et al. (2013 New Microbiol. 36:1-22). Recombinant AAV vectors useful in the disclosure can have any serotype that allows for transduction of the virus into the cell and insertion of the nuclease gene into the cell genome. In particular embodiments, recombinant AAV vectors have a serotype of AAV2 or AAV6. Recombinant AAV vectors can also be self-complementary such that they do not require second-strand DNA synthesis in the host cell (McCarty, et al. (2001) Gene Ther. 8:1248-54).

If the engineered nuclease genes are delivered in DNA form (*e.g.* plasmid) and/or via a viral vector (*e.g*. AAV) they must be operably linked to a promoter. In some embodiments, this is a viral promoter such as endogenous promoters from the viral vector (*e.g.* the LTR of a lentiviral vector) or the well-known cytomegalovirus- or SV40 virus-early promoters. In a preferred embodiment, nuclease genes are operably linked to a promoter that drives gene expression preferentially in the target cell *(e.g.,* a human T cell).

The disclosure further provides for the introduction of an exogenous nucleic acid into the cell, such that the exogenous nucleic acid sequence is inserted into the beta-2 microglobulin gene at a nuclease cleavage site. In some embodiments, the exogenous nucleic acid comprises a 5' homology arm and a 3' homology arm to promote recombination of the nucleic acid sequence into the cell genome at the nuclease cleavage site.

Exogenous nucleic acids of the disclosure is introduced into the cell by any of the means previously discussed. In a particular embodiment, exogenous nucleic acids are introduced by way of a viral vector, preferably a recombinant AAV vector. Recombinant AAV vectors useful for introducing an exogenous nucleic acid can have any serotype that allows for transduction of the virus into the cell and insertion of the exogenous nucleic acid sequence into the cell genome. In particular embodiments, the recombinant AAV vectors have a serotype of AAV2 or AAV6. The recombinant AAV vectors can also be self-complementary such that they do not require second-strand DNA synthesis in the host cell.

In another particular embodiment, an exogenous nucleic acid is introduced into the cell using a single-stranded DNA template. The single-stranded DNA can comprise the exogenous nucleic acid and, in preferred embodiments, can comprise 5' and 3' homology arms to promote insertion of the nucleic acid sequence into the nuclease cleavage site by homologous recombination. The single-stranded DNA can further comprise a 5' AAV inverted terminal repeat (ITR) sequence 5' upstream of the 5' homology arm, and a 3' AAV ITR sequence 3' downstream of the 3' homology arm.

### 2.4 Pharmaceutical Compositions

In some embodiments, the disclosure provides a pharmaceutical composition comprising a genetically-modified cell, or a population of genetically-modified cells, of the disclosure and a pharmaceutical carrier. Such pharmaceutical compositions is prepared in accordance with known techniques. See, *e.g.,* Remington, The Science And Practice of Pharmacy (21st ed. 2005). In the manufacture of a pharmaceutical formulation according to the disclosure, cells are typically admixed with a pharmaceutically acceptable carrier and the resulting composition is administered to a subject. The carrier must, of course, be acceptable in the sense of being compatible with any other ingredients in the formulation and must not be deleterious to the subject. In some embodiments, pharmaceutical compositions of the disclosure can further comprise one or more additional agents useful in the treatment of a disease in the subject. In additional embodiments, where the genetically-modified cell is a genetically-modified human T cell (or a cell derived therefrom), pharmaceutical compositions of the disclosure can further include biological molecules, such as cytokines (*e.g.,* IL-2, IL-7, IL-15, and/or EL-21), which promote *in vivo* cell proliferation and engraftment. Pharmaceutical compositions comprising genetically-modified cells of the disclosure is administered in the same composition as an additional agent or biological molecule or, alternatively, is co-administered in separate compositions.

Pharmaceutical compositions of the disclosure is useful for treating any disease state that is targeted by T cell adoptive immunotherapy. In a particular embodiment, the pharmaceutical compositions of the disclosure are useful in the treatment of cancer. Such cancers can include, without limitation, carcinoma, lymphoma, sarcoma, blastomas, leukemia, cancers of B-cell origin, breast cancer, gastric cancer, neuroblastoma, osteosarcoma, lung cancer, melanoma, prostate cancer, colon cancer, renal cell carcinoma, ovarian cancer, rhabdomyo sarcoma, leukemia, and Hodgkin's lymphoma. In certain embodiments, cancers of B-cell origin include, without limitation, B-lineage acute lymphoblastic leukemia, B-cell chronic lymphocytic leukemia, and B-cell non-Hodgkin's lymphoma.

### 2.5 Methods for Producing Recombinant AAV Vectors

In some embodiments, the disclosure provides recombinant AAV vectors for use in the methods of the disclosure. Recombinant AAV vectors are typically produced in mammalian cell lines such as HEK-293. Because the viral *cap* and *rep* genes are removed from the vector to prevent its self-replication to make room for the therapeutic gene(s) to be delivered (*e.g.* the endonuclease gene), it is necessary to provide these *in trans* in the packaging cell line. In addition, it is necessary to provide the "helper" (*e.g*. adenoviral) components necessary to support replication (Cots D, Bosch A, Chillon M (2013) Curr. Gene Ther. 13(5): 370-81). Frequently, recombinant AAV vectors are produced using a triple-transfection in which a cell line is transfected with a first plasmid encoding the "helper" components, a second plasmid comprising the *cap* and *rep* genes, and a third plasmid comprising the viral ITRs containing the intervening DNA sequence to be packaged into the virus. Viral particles comprising a genome (ITRs and intervening gene(s) of interest) encased in a capsid are then isolated from cells by freeze-thaw cycles, sonication, detergent, or other means known in the art. Particles are then purified using cesium-chloride density gradient centrifugation or affinity chromatography and subsequently delivered to the gene(s) of interest to cells, tissues, or an organism such as a human patient.

Because recombinant AAV particles are typically produced (manufactured) in cells, precautions must be taken in practicing the current disclosure to ensure that the site-specific endonuclease is NOT expressed in the packaging cells. Because the viral genomes of the disclosure comprise a recognition sequence for the endonuclease, any endonuclease expressed in the packaging cell line will be capable of cleaving the viral genome before it is packaged into viral particles. This will result in reduced packaging efficiency and/or the packaging of fragmented genomes. Several approaches are used to prevent endonuclease expression in the packaging cells, including:
1. The endonuclease is placed under the control of a tissue-specific promoter that is not active in the packaging cells. For example, if a viral vector is developed for delivery of (an) endonuclease gene(s) to muscle tissue, a muscle-specific promoter is used. Examples of muscle-specific promoters include C5-12 (Liu, et al. (2004) Hum Gene Ther. 15:783-92), the muscle-specific creatine kinase (MCK) promoter (Yuasa, et al. (2002) Gene Ther. 9:1576-88), or the smooth muscle 22 (SM22) promoter (Haase, et al. (2013) BMC Biotechnol. 13:49-54). Examples of CNS (neuron)-specific promoters include the NSE, Synapsin, and MeCP2 promoters (Lentz, et al. (2012) Neurobiol Dis. 48:179-88). Examples of liver-specific promoters include albumin promoters ( such as Palb), human α1-antitrypsin (such as Pa1AT), and hemopexin (such as Phpx) (Kramer, MG et al., (2003) Mol. Therapy 7:375-85). Examples of eye-specific promoters include opsin, and corneal epithelium-specific K12 promoters (Martin KRG, Klein RL, and Quigley HA (2002) Methods (28): 267-75) (Tong Y, et al., (2007) J Gene Med, 9:956-66). These promoters, or other tissue-specific promoters known in the art, are not highly-active in HEK-293 cells and, thus, will not expected to yield significant levels of endonuclease gene expression in packaging cells when incorporated into viral vectors of the present disclosure. Similarly, the viral vectors of the present disclosure contemplate the use of other cell lines with the use of incompatible tissue specific promoters *(i.e.,* the well-known HeLa cell line (human epithelial cell) and using the liver-specific hemopexin promoter). Other examples of tissue specific promoters include: synovial sarcomas PDZD4 (cerebellum), C6 (liver), ASB5 (muscle), PPP1R12B (heart), SLC5A12 (kidney), cholesterol regulation APOM (liver), ADPRHL1 (heart), and monogenic malformation syndromes TP73L (muscle). (Jacox E, et al., (2010) PLoS One v.5(8):e12274).
2. Alternatively, the vector is packaged in cells from a different species in which the endonuclease is not likely to be expressed. For example, viral particles is produced in microbial, insect, or plant cells using mammalian promoters, such as the well-known cytomegalovirus- or SV40 virus-early promoters, which are not active in the non-mammalian packaging cells. In a preferred embodiment, viral particles are produced in insect cells using the baculovirus system as described by Gao, et al. (Gao, H., et al. (2007) J. Biotechnol. 131(2):138-43). An endonuclease under the control of a mammalian promoter is unlikely to be expressed in these cells (Airenne, KJ, et al. (2013) Mol. Ther. 21(4):739-49). Moreover, insect cells utilize different mRNA splicing motifs than mammalian cells. Thus, it is possible to incorporate a mammalian intron, such as the human growth hormone (HGH) intron or the SV40 large T antigen intron, into the coding sequence of an endonuclease. Because these introns are not spliced efficiently from pre-mRNA transcripts in insect cells, insect cells will not express a functional endonuclease and will package the full-length genome. In contrast, mammalian cells to which the resulting recombinant AAV particles are delivered will properly splice the pre-mRNA and will express functional endonuclease protein. Haifeng Chen has reported the use of the HGH and SV40 large T antigen introns to attenuate expression of the toxic proteins barnase and diphtheria toxin fragment A in insect packaging cells, enabling the production of recombinant AAV vectors carrying these toxin genes (Chen, H (2012) Mol Ther Nucleic Acids. 1(11): e57).
3. The endonuclease gene is operably linked to an inducible promoter such that a small-molecule inducer is required for endonuclease expression. Examples of inducible promoters include the Tet-On system (Clontech; Chen H., et al., (2015) BMC Biotechnol. 15(1):4)) and the RheoSwitch system (Intrexon; Sowa G., et al., (2011) Spine, 36(10): E623-8). Both systems, as well as similar systems known in the art, rely on ligand-inducible transcription factors (variants of the Tet Repressor and Ecdysone receptor, respectively) that activate transcription in response to a small-molecule activator (Doxycycline or Ecdysone, respectively). Practicing the current disclosure using such ligand-inducible transcription activators includes: 1) placing the endonuclease gene under the control of a promoter that responds to the corresponding transcription factor, the endonuclease gene having (a) binding site(s) for the transcription factor; and 2) including the gene encoding the transcription factor in the packaged viral genome The latter step is necessary because the endonuclease will not be expressed in the target cells or tissues following recombinant AAV delivery if the transcription activator is not also provided to the same cells. The transcription activator then induces endonuclease gene expression only in cells or tissues that are treated with the cognate small-molecule activator. This approach is advantageous because it enables endonuclease gene expression to be regulated in a spatio-temporal manner by selecting when and to which tissues the small-molecule inducer is delivered. However, the requirement to include the inducer in the viral genome, which has significantly limited carrying capacity, creates a drawback to this approach.
4. In another preferred embodiment, recombinant AAV particles are produced in a mammalian cell line that expresses a transcription repressor that prevents expression of the endonuclease. Transcription repressors are known in the art and include the Tet-Repressor, the Lac-Repressor, the Cro repressor, and the Lambdarepressor. Many nuclear hormone receptors such as the ecdysone receptor also act as transcription repressors in the absence of their cognate hormone ligand. To practice the current disclosure, packaging cells are transfected/transduced with a vector encoding a transcription repressor and the endonuclease gene in the viral genome (packaging vector) is operably linked to a promoter that is modified to comprise binding sites for the repressor such that the repressor silences the promoter. The gene encoding the transcription repressor is placed in a variety of positions. It is encoded on a separate vector; it is incorporated into the packaging vector outside of the ITR sequences; it is incorporated into the cap/rep vector or the adenoviral helper vector; or, most preferably, it is stably integrated into the genome of the packaging cell such that it is expressed constitutively. Methods to modify common mammalian promoters to incorporate transcription repressor sites are known in the art. For example, Chang and Roninson modified the strong, constitutive CMV and RSV promoters to comprise operators for the Lac repressor and showed that gene expression from the modified promoters was greatly attenuated in cells expressing the repressor (Chang BD, and Roninson IB (1996) Gene 183:137-42). The use of a non-human transcription repressor ensures that transcription of the endonuclease gene will be repressed only in the packaging cells expressing the repressor and not in target cells or tissues transduced with the resulting recombinant AAV vector.

### 2.6 Engineered Nuclease Variants

Embodiments of the disclosure encompass the engineered nucleases, and particularly the recombinant meganucleases, described herein, and variants thereof. Further embodiments of the disclosure encompass isolated polynucleotides comprising a nucleic acid sequence encoding the recombinant meganucleases described herein, and variants of such polynucleotides.

As used herein, "variants" is intended to mean substantially similar sequences. A "variant" polypeptide is intended to mean a polypeptide derived from the "native" polypeptide by deletion or addition of one or more amino acids at one or more internal sites in the native protein and/or substitution of one or more amino acids at one or more sites in the native polypeptide. As used herein, a "native" polynucleotide or polypeptide comprises a parental sequence from which variants are derived. Variant polypeptides encompassed by the embodiments are biologically active. That is, they continue to possess the desired biological activity of the native protein; *i.e.,* the ability to recognize and cleave recognition sequences found in the human beta-2 microglobulin gene (SEQ ID NO:1), including, for example, the B2M 13-14 recognition sequence (SEQ ID NO:2), the B2M 5-6 recognition sequence (SEQ ID NO:4), the B2M 7-8 recognition sequence (SEQ ID NO:6), and the B2M 11-12 recognition sequence (SEQ ID NO:8). Such variants may result, for example, from human manipulation. Biologically active variants of a native polypeptide of the embodiments (e.g., SEQ ID NOs: 12-126), or biologically active variants of the recognition half-site binding subunits described herein *(e.g.,* SEQ ID NOs: 132-361), will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, or about 99%, sequence identity to the amino acid sequence of the native polypeptide or native subunit, as determined by sequence alignment programs and parameters described elsewhere herein. A biologically active variant of a polypeptide or subunit of the embodiments may differ from that polypeptide or subunit by as few as about 1-40 amino acid residues, as few as about 1-20, as few as about 1-10, as few as about 5, as few as 4, 3, 2, or even 1 amino acid residue.

The polypeptides of the embodiments is altered in various ways including amino acid substitutions, deletions, truncations, and insertions. Methods for such manipulations are generally known in the art. For example, amino acid sequence variants are prepared by mutations in the DNA. Methods for mutagenesis and polynucleotide alterations are well known in the art. See, for example, Kunkel (1985) Proc. Natl. Acad. Sci. USA 82:488-492; Kunkel et al. (1987) Methods in Enzymol. 154:367-382; U.S. Pat. No. 4,873,192; Walker and Gaastra, eds. (1983) Techniques in Molecular Biology (MacMillan Publishing Company, New York) and the references cited therein. Guidance as to appropriate amino acid substitutions that do not affect biological activity of the protein of interest is found in the model of Dayhoff et al. (1978) Atlas of Protein Sequence and Structure (Natl. Biomed. Res. Found., Washington, D.C.), herein incorporated by reference. Conservative substitutions, such as exchanging one amino acid with another having similar properties, is optimal.

A substantial number of amino acid modifications to the DNA recognition domain of the wild-type I-CreI meganuclease have previously been identified *(e.g.,* U.S. 8,021,867) which, singly or in combination, result in recombinant meganucleases with specificities altered at individual bases within the DNA recognition sequence half-site, such that the resulting rationally-designed meganucleases have half-site specificities different from the wild-type enzyme. Table 5 provides potential substitutions that can be made in a recombinant meganuclease monomer or subunit to enhance specificity based on the base present at each half-site position (-1 through -9) of a recognition half-site. Such substitutions are incorporated into variants of the meganucleases disclosed herein.

**Table 5**

| | Favored Sense-Strand Base | | | | | | | | | | |
|---|---|---|---|---|---|---|---|---|---|---|---|
| Posn. | A | C | G | T | A/T | A/C | A/G | C/T | G/T | A/G/T | A/C/G/T |
| -1 | Y75 | **R70*** | K70 | Q70^{∗} | | | | **T46*** | | | G70 |
| | L75^{∗} | H75^{∗} | E70^{∗} | C70 | | | | | | | A70 |
| | C75^{∗} | R75^{∗} | E75^{∗} | L70 | | | | | | | S70 |
| | Y139^{∗} | H46^{∗} | E46^{∗} | Y75^{∗} | | | | | | | G46^{∗} |
| | C46^{∗} | K46^{∗} | D46^{∗} | Q75^{∗} | | | | | | | |
| | A46^{∗} | R46^{∗} | | H75^{∗} | | | | | | | |
| | | | | H139 | | | | | | | |
| | | | | Q46^{∗} | | | | | | | |
| | | | | H46^{∗} | | | | | | | |
| -2 | Q70 | E70 | H70 | **Q44*** | C44^{∗} | | | | | | |
| | T44^{∗} | D70 | D44^{∗} | | | | | | | | |
| | A44^{∗} | K44^{∗} | E44^{∗} | | | | | | | | |
| | V44^{∗} | R44^{∗} | | | | | | | | | |
| | 144^{∗} | | | | | | | | | | |
| | L44^{∗} | | | | | | | | | | |
| | N44^{∗} | | | | | | | | | | |
| -3 | Q68 | E68 | **R68** | M68 | | H68 | | Y68 | K68 | | |
| | C24^{∗} | F68 | | C68 | | | | | | | |
| | **I24*** | K24^{∗} | | L68 | | | | | | | |
| | | R24^{∗} | | F68 | | | | | | | |
| -4 | A26^{∗} | E77 | R77 | | | | | S77 | | | S26^{∗} |
| | Q77 | K26^{∗} | E26^{∗} | | | | | **Q26*** | | | |
| -5 | | E42 | R42 | | | **K28*** | C28^{∗} | | | | M66 |
| | | | | | | | Q42 | | | | K66 |
| -6 | Q40 | E40 | R40 | C40 | A40 | | | | | | **S40** |
| | C28^{∗} | R28^{∗} | | 140 | A79 | | | | | | S28^{∗} |
| | | | | V40 | A28^{∗} | | | | | | |
| | | | | C79 | H28^{∗} | | | | | | |
| | | | | 179 | | | | | | | |
| | | | | V79 | | | | | | | |
| | | | | Q28* | | | | | | | |
| -7 | **N30*** | E38 | K38 | 138 | | | C38 | | | | H38 |
| | **Q38** | K30^{∗} | R38 | L38 | | | | | | | N38 |
| | | R30^{∗} | E30^{∗} | | | | | | | | Q30* |
| -8 | F33 | E33 | F33 | L33 | | R32^{∗} | R33 | | | | |
| | Y33 | D33 | H33 | V33 | | | | | | | |
| | | | | 133 | | | | | | | |
| | | | | F33 | | | | | | | |
| | | | | C33 | | | | | | | |
| -9 | | E32 | R32 | L32 | | | | D32 | | | **S32** |
| | | | K32 | V32 | | | | 132 | | | N32 |
| | | | | A32 | | | | | | | H32 |
| | | | | C32 | | | | | | | Q32 |
| | | | | | | | | | | | T32 |

For polynucleotides, a "variant" comprises a deletion and/or addition of one or more nucleotides at one or more sites within the native polynucleotide. One of skill in the art will recognize that variants of the nucleic acids of the embodiments will be constructed such that the open reading frame is maintained. For polynucleotides, conservative variants include those sequences that, because of the degeneracy of the genetic code, encode the amino acid sequence of one of the polypeptides of the embodiments. Variant polynucleotides include synthetically derived polynucleotides, such as those generated, for example, by using sitedirected mutagenesis but which still encode a recombinant meganuclease of the embodiments. Generally, variants of a particular polynucleotide of the embodiments will have at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, about 99% or more sequence identity to that particular polynucleotide as determined by sequence alignment programs and parameters described elsewhere herein. Variants of a particular polynucleotide of the embodiments *(i.e.,* the reference polynucleotide) can also be evaluated by comparison of the percent sequence identity between the polypeptide encoded by a variant polynucleotide and the polypeptide encoded by the reference polynucleotide.

The deletions, insertions, and substitutions of the protein sequences encompassed herein are not expected to produce radical changes in the characteristics of the polypeptide. However, when it is difficult to predict the exact effect of the substitution, deletion, or insertion in advance of doing so, one skilled in the art will appreciate that the effect will be evaluated by screening the polypeptide for its ability to preferentially recognize and cleave recognition sequences found within the human beta-2 microglobulin gene (SEQ ID NO: 1).

### EXAMPLES

This disclosure is further illustrated by the following examples, which should not be construed as limiting. Those skilled in the art will recognize, or be able to ascertain, using no more than routine experimentation, numerous equivalents to the specific substances and procedures described herein. Such equivalents are intended to be encompassed in the scope of the items that follow the examples below.

### EXAMPLE 1

### Characterization of Meganucleases That Recognize and Cleave B2M Recognition Sequences

### 1. Meganucleases that recognize and cleave the B2M 13-14 recognition sequence

Recombinant meganucleases (SEQ ID NOs: 12-100), collectively referred to herein as "B2M 13-14 meganucleases," were engineered to recognize and cleave the B2M 13-14 recognition sequence (SEQ ID NO:2), which is present in the human beta-2 microglobulin gene (SEQ ID NO: 1). Each B2M 13-14 recombinant meganuclease comprises an N-terminal nuclease-localization signal derived from SV40, a first meganuclease subunit, a linker sequence, and a second meganuclease subunit. A first subunit in each B2M 13-14 meganuclease binds to the B2M13 recognition half-site of SEQ ID NO:2, while a second subunit binds to the

### B2M14 recognition half-site (see Fig. 1).

B2M13-binding subunits and B2M14-binding subunits each comprise a 56 base pair hypervariable region, referred to as HVR1 and HVR2, respectively. B2M13-binding subunits are highly conserved or, in many cases, identical outside of the HVR1 region except at position 80 or position 271 (comprising a Q or E residue), and are highly conserved within the HVR1 region. Similarly, B2M14-binding subunits are also highly conserved or, in many cases, identical outside of the HVR2 region except at position 80 or position 271 (comprising a Q or E residue). Like the HVR1 region, the HVR2 region is also highly conserved.

The B2M13-binding regions of SEQ ID NOs: 12-100 are provided as SEQ ID NOs: 132-220, respectively. Each of SEQ ID NOs: 132-220 share at least 90% sequence identity to SEQ ID NO: 132, which is the B2M13-binding region of the meganuclease B2M 13-14x.479 (SEQ ID NO:12). B2M14-binding regions of SEQ ID NOs: 12-100 are provided as SEQ ID NOs:221-309, respectively. Each of SEQ ID NOs:221-309 share at least 90% sequence identity to SEQ ID NO:221, which is the B2M14-binding region of the meganuclease B2M 13-14x.479 (SEQ ID NO: 12).

### 2. Meganucleases that recognize and cleave the B2M 5-6 recognition sequence

Recombinant meganucleases (SEQ ID NOs: 101-113), collectively referred to herein as "B2M 5-6 meganucleases," were engineered to recognize and cleave the B2M 5-6 recognition sequence (SEQ ID NO:4), which is present in the human beta-2 microglobulin gene (SEQ ID NO:1). Each B2M 5-6 recombinant meganuclease comprises an N-terminal nuclease-localization signal derived from SV40, a first meganuclease subunit, a linker sequence, and a second meganuclease subunit. A first subunit in each B2M 5-6 meganuclease binds to the B2M5 recognition half-site of SEQ ID NO:4, while a second subunit binds to the B2M6 recognition half-site *(see* Fig. 1).

B2M5-binding subunits and B2M6-binding subunits each comprise a 56 base pair hypervariable region, referred to as HVR1 and HVR2, respectively. B2M5-binding subunits are highly conserved or, in many cases, identical outside of the HVR1 region except at position 80 or position 271 (comprising a Q or E residue), and are highly conserved within the HVR1 region. Similarly, B2M5-binding subunits are also highly conserved or, in many cases, identical outside of the HVR2 region except at position 80 or position 271 (comprising a Q or E residue), and are highly conserved within the HVR2 region.

The B2M5-binding regions of SEQ ID NOs: 101-113 are provided as SEQ ID NOs:310-322, respectively. Each of SEQ ID NOs:310-322 share at least 90% sequence identity to SEQ ID NO:310, which is the B2M5-binding region of the meganuclease B2M 5-6x.14 (SEQ ID NO:101). B2M6-binding regions of SEQ ID NOs: 101-113 are provided as SEQ ID NOs:323-335, respectively. Each of SEQ ID NOs:323-335 share at least 90% sequence identity to SEQ ID NO:323, which is the B2M6-binding region of the meganuclease B2M (SEQ ID NO:101).

### 3. Meganucleases that recognize and cleave the B2M 7-8 recognition sequence

Recombinant meganucleases (SEQ ID NOs: 114-124), collectively referred to herein as "B2M 7-8 meganucleases," were engineered to recognize and cleave the B2M 7-8 recognition sequence (SEQ ID NO:6), which is present in the human beta-2 microglobulin gene (SEQ ID NO:1). Each B2M 7-8 recombinant meganuclease comprises an N-terminal nuclease-localization signal derived from SV40, a first meganuclease subunit, a linker sequence, and a second meganuclease subunit. A first subunit in each B2M 7-8 meganuclease binds to the B2M7 recognition half-site of SEQ ID NO:6, while a second subunit binds to the B2M8 recognition half-site *(see* Fig. 1).

B2M7-binding subunits and B2M8-binding subunits each comprise a 56 base pair hypervariable region, referred to as HVR1 and HVR2, respectively. B2M7-binding subunits are highly conserved or, in many cases, identical outside of the HVR1 region except at position 80 or position 271 (comprising a Q or E residue), and are highly conserved within the HVR1 region. Similarly, B2M8-binding subunits are also highly conserved or, in many cases, identical outside of the HVR2 region except at position 80 or position 271 (comprising a Q or E residue), and are highly conserved within the HVR2 region.

The B2M7-binding regions of SEQ ID NOs: 114-124 are provided as SEQ ID NOs:336-346, respectively. Each of SEQ ID NOs:336-346 share at least 90% sequence identity to SEQ ID NO:336, which is the B2M7-binding region of the meganuclease B2M 7-8x.88 (SEQ ID NO:114). B2M8-binding regions of SEQ ID NOs: 114-124 are provided as SEQ ID NOs:347-357, respectively. Each of SEQ ID NOs:347-357 share at least 90% sequence identity to SEQ ID NO:347, which is the B2M8-binding region of the meganuclease B2M 7-8x.88 (SEQ ID NO:114).

### 4. Meganucleases that recognize and cleave the B2M 11-12 recognition sequence

Recombinant meganucleases (SEQ ID NOs:125 and 126), collectively referred to herein as "B2M 11-12 meganucleases," were engineered to recognize and cleave the B2M 11-12 recognition sequence (SEQ ID NO:8), which is present in the human beta-2 microglobulin gene (SEQ ID NO:1). Each B2M 11-12 recombinant meganuclease comprises an N-terminal nuclease-localization signal derived from SV40, a first meganuclease subunit, a linker sequence, and a second meganuclease subunit. A first subunit in each B2M 11-12 meganuclease binds to the B2M11 recognition half-site of SEQ ID NO:8, while a second subunit binds to the B2M12 recognition half-site *(see* Fig. 1).

B2M11-binding subunits and B2M12-binding subunits each comprise a 56 base pair hypervariable region, referred to as HVR1 and HVR2, respectively. B2M11-binding subunits are highly conserved or, in many cases, identical outside of the HVR1 region except at position 80 or position 271 (comprising a Q or E residue), and are highly conserved within the HVR1 region. Similarly, B2M12-binding subunits are also highly conserved or, in many cases, identical outside of the HVR2 region except at position 80 or position 271 (comprising a Q or E residue), and are highly conserved within the HVR2 region.

The B2M11-binding regions of SEQ ID NOs:125 and 126 are provided as SEQ ID NOs:358 and 359, respectively. SEQ ID NOs:358 and 359 share 99% sequence identity. B2M12-binding regions of SEQ ID NOs:125 and 126 are provided as SEQ ID NOs:360 and 361, respectively. SEQ ID NOs:360 and 361 share 99% sequence identity.

### 5. Cleavage of B2M recognition sequences in a CHO cell reporter assay

To determine whether B2M 13-14, B2M 5-6, B2M 7-8, and B2M 11-12 meganucleases could recognize and cleave their respective recognition sequences (SEQ ID NOs:2, 4, 6, and 8, respectively), each recombinant meganuclease was evaluated using the CHO cell reporter assay previously described *(see* WO/2012/167192 and Figs. 8A-8D). To perform the assays, CHO cell reporter lines were produced which carried a non-functional Green Fluorescent Protein (GFP) gene expression cassette integrated into the genome of the cells. The GFP gene in each cell line was interrupted by a pair of recognition sequences such that intracellular cleavage of either recognition sequence by a meganuclease would stimulate a homologous recombination event resulting in a functional GFP gene.

In CHO reporter cell lines developed for this study, one recognition sequence inserted into the GFP gene was the B2M 13-14 recognition sequence (SEQ ID NO:2), the B2M 5-6 recognition sequence (SEQ ID NO:4), the B2M 7-8 recognition sequence (SEQ ID NO:6), or the B2M 11-12 recognition sequence (SEQ ID NO:8). The second recognition sequence inserted into the GFP gene was a CHO-23/24 recognition sequence, which is recognized and cleaved by a control meganuclease called "CHO-23/24". CHO reporter cells comprising the B2M 13-14 recognition sequence and the CHO-23/24 recognition sequence are referred to herein as "B2M 13-14 cells." CHO reporter cells comprising the B2M 5-6 recognition sequence and the CHO-23/24 recognition sequence are referred to herein as "B2M 5-6 cells." CHO reporter cells comprising the B2M 7-8 recognition sequence and the CHO-23/24 recognition sequence are referred to herein as "B2M 7-8 cells." CHO reporter cells comprising the B2M 11-12 recognition sequence and the CHO-23/24 recognition sequence are referred to herein as "B2M 11-12 cells."

CHO reporter cells were transfected with plasmid DNA encoding their corresponding recombinant meganucleases (e.g., B2M 13-14 cells were transfected with plasmid DNA encoding B2M 13-14 meganucleases) or encoding the CHO-23/34 meganuclease. In each assay, 4e⁵ CHO reporter cells were transfected with 50 ng of plasmid DNA in a 96-well plate using Lipofectamine 2000 (ThermoFisher) according to the manufacturer's instructions. At 48 hours post-transfection, cells were evaluated by flow cytometry to determine the percentage of GFP-positive cells compared to an untransfected negative control *(e.g.,* B2M 13-14bs). As shown in Figs. 4A-4J, all B2M 13-14, B2M 5-6, B2M 7-8, and B2M 11-12 meganucleases tested were found to produce GFP-positive cells in cell lines comprising their corresponding recognition sequence at frequencies significantly exceeding the negative control.

These studies demonstrated that B2M 13-14 meganucleases, B2M 5-6 meganucleases, B2M 11-12 meganucleases, and B2M 13-14 meganucleases encompassed by the disclosure can efficiently target and cleave their respective recognition sequences in cells.

### EXAMPLE 2

### Suppression of Cell-Surface B2M Expression in T Cells

### 1. Suppression of B2M cell-surface expression in human T cells

This study demonstrated that a select number of B2M 13-14 meganucleases encompassed by the disclosure could cleave the B2M 13-14 recognition sequence in human T cells obtained from a donor, resulting in suppression of B2M cell-surface expression. To test whether B2M meganucleases could cleave the B2M 13-14 recognitions sequence in human T cells, donor cells were stimulated with anti-CD3 and anti-CD28 antibodies for 3 days, then electroporated with mRNA encoding a given B2M 13-14 meganuclease (1µg) using the Amaxa 4D-Nucleofector (Lonza) according to the manufacturer's instructions. As a positive control, cells were mock electroporated. In an additional control for electroporation efficiency, cells were electroporated with mRNA encoding GFP (1µg). At 3 days post-electroporation, cells were stained with an antibody recognizing β-2 microglobulin (BD Biosciences) and analyzed by flow cytometry. Flow plots are shown in Figs. 5A-5N and the data are summarized in Table 6.

Positive control cells and GFP-electroporated cells stained overwhelmingly positive for B2M expression with 0.18% and 0.23% of the cells staining negative, respectively (Figs. 5A and 5C, and Table 6). Unstained control cells were 99.99% negative for B2M staining (Fig. 5B and Table 6). Surprisingly, although all of the B2M 13-14 meganucleases tested were successful in the CHO reporter assay, B2M 13-14x.93 was the only meganuclease that showed any indication that it could generate indels in the B2M gene and reduce B2M cell-surface expression, with 2.18% of cells staining negative for B2M (Fig. 5N and Table 6). Cells electroporated with any of the other B2M 13-14 meganucleases tested showed B2M-negative cells roughly equivalent to the mock-electroporated control, ranging from 0.01%-0.14% negative (Figs. 5A-5M and Table 6). These results indicated that, for the B2M gene, successful cleavage of a B2M recognition sequence in reporter cells does not assure cleavage of the recognition sequence in T cells and, subsequently, reduced cell-surface expression of B2M.

**Table 6**

| **Meganuclease** | **% B2M Negative** | **% B2M Positive** |
|---|---|---|
| Positive Control | 0.18% | 99.82% |
| Unstained Control | 99.99% | 0.01% |
| GFP | 0.23% | 99.77% |
| B2M 13-14x.10 | 0.10% | 99.90% |
| B2M 13-14x.32 | 0.03% | 99.97% |
| B2M 13-14x.82 | 0.06% | 99.94% |
| B2M 13-14x.84 | 0.01% | 99.99% |
| B2M 13-14x.85 | 0.06% | 99.94% |
| B2M 13-14x.3 | 0.00% | 100.00% |
| B2M 13-14x.14 | 0.04% | 99.96% |
| B2M 13-14x.22 | 0.06% | 99.94% |
| B2M 13-14x.31 | 0.01% | 99.99% |
| B2M 13-14x.76 | 0.14% | 99.86% |
| B2M 13-14x.93 | 2.18% | 97.82% |

Since B2M 13-14x.93 was the only one of the B2M 13-14 meganucleases that demonstrated activity against the B2M recognition sequence in human T cells, this meganuclease was further modified to increase nuclease activity. The inventors have previously shown that mutations at amino acid position 80 and 66 of an I-CreI-derived meganuclease subunit (which also corresponds to positions 271 and 257, respectively, of a single-chain meganuclease) can dramatically impact nuclease activity, presumably due to non-specific interactions with the negatively-charged DNA backbone. Common substitutions include E or Q at amino acid position 80, and Y, K, or R at amino acid position 66. Amino acid position 80 can be changed in either the first meganuclease subunit and/or the second meganuclease subunit, generating the following possible combinations: E in both meganuclease subunits, Q in both meganuclease subunits, E in the first meganuclease subunit and Q in the second meganuclease subunit, or Q in the first meganuclease subunit and E in the second meganuclease subunit. Amino acid position 66 can be modified in either meganuclease subunit, but in this case is only modified in the first meganuclease subunit. The original B2M 13-14x.93 meganuclease had a Q at amino acid position 80 in both meganuclease subunits.

Table 7 shows B2M 13-14x.93 variants generated, with either E or Q indicating the amino acid at position 80 in the first and second meganuclease subunit, respectively followed by the amino acid substitution made at position 66 in the first meganuclease subunit. For example, B2M 13-14x.93 EQY66 indicates that amino acid 80 in the first meganuclease subunit is E, amino acid 80 (*i.e.,* 271) in the second meganuclease subunit is Q, and amino acid 66 in the first meganuclease subunit is Y.

To test these variants of B2M 13-14x.93, donor human T cells were stimulated with anti-CD3 and anti-CD28 antibodies for 3 days, then electroporated with mRNA encoding a given B2M 13-14 meganuclease (1µg) using the Amaxa 4D-Nucleofector (Lonza) according to the manufacturer's instructions. At 3 days post-electroporation, cells were stained with an antibody recognizing β-2 microglobulin (BD Biosciences) and analyzed by flow cytometry. Flow cytometry results are summarized in Table 7. All B2M 13-14x.93 variants were able to disrupt the B2M gene, with knockout efficiencies ranging from 1.58% to 37% (Table 7). The most active B2M 13-14x.93 variant was B2M 13-14x.93 QE, which resulted in 37% B2M-negative cells (Table 7). The next two most active B2M 13-14x.93 meganuclease variants both had a Q at position 80 in the second meganuclease subunit and a Y at position 66 (B2M 13-14x.93 QQY66 and B2M 13-14x.93 EQY66). Interestingly, most of the variants were not markedly different from the original B2M 13-14x.93 meganuclease (B2M 13-14x.93EQ, QQK66, QQR66, EEY66, EEK66, EER66, EQK66, and EQR66).

**Table 7**

| **Meganuclease** | **% B2M Negative** | **% B2M Positive** |
|---|---|---|
| B2M 13-14x.93EE | 18.3 | 81.7 |
| B2M 13-14x.93QE | 37 | 63 |
| B2M 13-14x.93EQ | 5.13 | 94.87 |
| B2M 13-14x.93QQY66 | 19.2 | 80.8 |
| B2M 13-14x.93QQK66 | 2.64 | 97.36 |
| B2M 13-14x.93QQR66 | 5.05 | 94.95 |
| B2M 13-14x.93EEY66 | 1.58 | 98.42 |
| B2M 13-14x.93EEK66 | 3.84 | 96.16 |
| B2M 13-14x.93EER66 | 7.07 | 92.93 |
| B2M 13-14x.93EQY66 | 21.4 | 78.6 |
| B2M 13-14x.93EQK66 | 6.81 | 93.19 |
| B2M 13-14x.93EQR66 | 5.12 | 94.88 |
| B2M 13-14x.93 | 3.95 | 96.05 |

While these substitutions at amino acid positions 80 and 66 resulted in B2M 13-14x.93 meganucleases that were more active against the B2M 13-14 recognition sequence than the original B2M 13-14x.93 meganuclease, further optimization was carried out to maximize the activity of the B2M 13-14 meganucleases. New B2M 13-14 meganucleases were engineered in which the first meganuclease subunit remained the same as in B2M 13-14x.93, but the second meganuclease subunit contained new amino acid substitutions at positions contacting the B2M 13-14 recognition sequence.

To test these new B2M 13-14 variants, donor human T cells were stimulated with anti-CD3 and anti-CD28 antibodies for 3 days, then electroporated with mRNA encoding a given B2M 13-14 meganuclease (1µg) using the Amaxa 4D-Nucleofector (Lonza) according to the manufacturer's instructions. B2M 13-14x.93 QE was included to allow for comparison to previous variants. At 6 days post-electroporation, cells were stained with an antibody recognizing β-2 microglobulin (BD Biosciences) as well as an antibody recognizing CD3 (BioLegend), a marker of T cells. Flow cytometry plots are shown in Figs. 6A-6J.

In this experiment, B2M 13-14x.93QE generated 21.2% B2M-negative cells, compared to 0.49% in the non-electroporated control cells (Figs. 6B and 6A, respectively). Several of the new variants, including B2M 13-14x.97, B2M 13-14x.199, B2M 13-14x.202, B2M 13-14x.169, and B2M 13-14x.275 were significantly more active than B2M 13-14x.93 QE, generating B2M knockout efficiencies as high as 58.4% (Fig. 6J).

A final group of B2M 13-14 meganucleases was generated and evaluated for their ability to eliminate cell-surface expression of B2M on human T cells. These nucleases were based on B2M 13-14x.169, one of the variants described above. Changes were made in the first meganuclease subunit to introduce alternative base contacts, while the second meganuclease subunit remained the same as in B2M 13-14x.169.

Donor human T cells were stimulated with anti-CD3 and anti-CD28 antibodies for 3 days, then electroporated with mRNA encoding a given B2M 13-14 meganuclease (1µg) using the Amaxa 4D-Nucleofector (Lonza) according to the manufacturer's instructions. B2M 13-14x.202 was included to allow for comparison to previous variants shown in Figs. 6A-6J. To look for the loss of B2M surface expression, cells were stained with an antibody recognizing β-2 microglobulin (BD Biosciences) as well as an antibody recognizing CD3 (BioLegend), a marker of T cells. Flow cytometry data for the entire panel of variants at day 3 post-electroporation are summarized in Table 8, and flow plots of the B2M 13-14 meganucleases that showed B2M knockout efficiency of >40% are shown in Figs. 7A-7H.

Similar to the previous experiment, B2M 13-14x.202 showed a B2M knockout efficiency of 60.9% (Table 8). Several of the B2M 13-14 variants tested showed knockout efficiencies greater than 40% (Figs. 7A-7H) and two variants, B2M 13-14x.287 and B2M 13-14x.479, surpassed the efficiency of B2M 13-14x.202 with negative staining populations of 75.7% and 67.2%, respectively.

**Table 8**

| **Meganuclease** | **% B2M Negative** | **% B2M Positive** |
|---|---|---|
| B2M 13-14x.281 | 53.6 | 46.4 |
| B2M 13-14x.283 | 49.3 | 50.7 |
| B2M 13-14x.285 | 22 | 78 |
| B2M 13-14x.286 | 44.6 | 55.4 |
| B2M 13-14x.287 | 75.7 | 24.3 |
| B2M 13-14x.288 | 18.8 | 81.2 |
| B2M 13-14x.317 | 3.91 | 96.09 |
| B2M 13-14x.325 | 14.4 | 85.6 |
| B2M 13-14x.338 | 4.68 | 95.32 |
| B2M 13-14x.362 | 37.8 | 62.2 |
| B2M 13-14x.365 | 27.4 | 72.6 |
| B2M 13-14x.371 | 22.6 | 77.4 |
| B2M 13-14x.377 | 75.9 | 24.1 |
| B2M 13-14x.378 | 3.54 | 96.46 |
| B2M 13-14x.381 | 44.1 | 55.9 |
| B2M 13-14x.448 | 17.1 | 82.9 |
| B2M 13-14x.456 | 15.7 | 84.3 |
| B2M 13-14x.457 | 19.7 | 80.3 |
| B2M 13-14x.464 | 8.24 | 91.76 |
| B2M 13-14x.465 | 30.8 | 69.2 |
| B2M 13-14x.479 | 67.2 | 32.8 |
| B2M 13-14x.556 | 11.8 | 88.2 |
| B2M 13-14x.551 | 40.3 | 59.7 |
| B2M 13-14x.202 | 60.9 | 39.1 |

The data presented above demonstrate the successful engineering of meganucleases designed to target a double strand break at the beta-2 microglobulin gene and the use of such meganucleases to generate mutations in the beta-2 microglobulin gene in human T cells, resulting in knockout of the gene. Surprisingly, only one of eleven B2M 13-14 meganucleases that were successful in the CHO reporter assay was actually able to eliminate expression of the B2M gene. Further, the only one of the initial B2M 13-14 meganuclease that caused a deletion in the B2M gene, B2M 13-14x.93, did so with very low frequency (2.18%, Table 6). B2M 13-14x.93 was taken through several rounds of redesign in order to optimize its activity and specificity, eventually resulting in several B2M 13-14 meganucleases that were capable of generating B2M knockouts with efficiencies in the 60-75% range (Table 8).

### EXAMPLE 3

### Double Knockout of Cell-Surface B2M and T Cell Receptor in T Cells

### 1. Double Knockout By Simultaneous Nucleofection

In some cases, it may be desirable to knockout both the beta-2 microglobulin gene and a native T cell receptor (TCR). The inventors have previously described meganucleases designed to cause a double strand break in the T cell receptor alpha constant gene (SEQ ID NO: 127) which, in turn, disrupts cell-surface expression of the endogenous TCR. One such meganuclease is referred to as TRC 1-2x.87 EE (SEQ ID NO: 131), which targets the recognition sequence set forth in SEQ ID NO: 128. Loss of the TCR can be observed by staining cells with an antibody against the CD3 protein, which is only expressed on the surface of cells if the TCR is expressed.

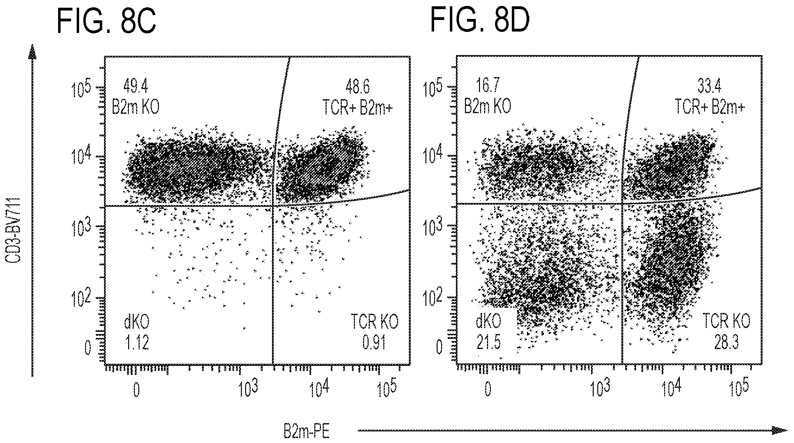

To test whether TRC 1-2x.87 EE and B2M 13-14 meganucleases could be used to generate a population of cells in which both the TRC gene and the B2M gene were knocked out, experiments were performed in which separate mRNAs encoding these meganucleases were delivered simultaneously to human T cells. In a first study, donor human T cells were stimulated with anti-CD3 and anti-CD28 antibodies for 2 days, then co-electroporated with mRNA encoding B2M 13-14x.202 (1µg) and mRNA encoding TRC 1-2x.87 EE (1µg) using the Amaxa 4D-Nucleofector (Lonza) according to the manufacturer's instructions. As controls, human T cells were mock electroporated or electroporated with mRNA encoding a single meganuclease, either B2M 13-14x.202 or TRC 1-2x.87 EE. At 6 days post-electroporation, cells were stained with an antibody against CD3 and an antibody against B2M and analyzed by flow cytometry (Figs. 8A-8D). Cells that were electroporated with TRC 1-2x.87 EE alone were 57.6% TCR negative (Fig. 8B), compared to 2.35% in the mock electroporated cells (Fig. 8A), and cells that were electroporated with B2M 13-14x.202 alone were 49.4% B2M negative (Fig. 8C) compared to 0.72% in mock electroporated cells (Fig. 8A). Cells that were co-electroporated with mRNA encoding B2M 13-14x.202 and mRNA encoding TRC 1-2x.87 EE show a clear population in which 21.5% of the cells were both negative for B2M and TCR expression (Fig. 8D), compared to 0.66% in mock electroporated cells (Fig. 8A). In cells that were co-electroporated with mRNA for both meganucleases, the single knockout efficiencies for TCR and B2M were 28.3% and 16.7%, respectively.

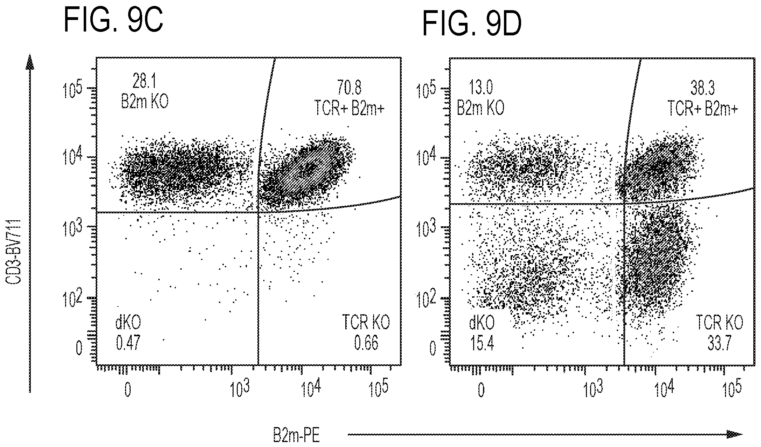

In a second study, donor human T cells were stimulated with anti-CD3 and anti-CD28 antibodies for 2 days, then co-electroporated with mRNA encoding B2M 13-14x.169 (1µg) and mRNA encoding TRC 1-2x.87 EE (1µg) using the Amaxa 4D-Nucleofector (Lonza) according to the manufacturer's instructions. As controls, human T cells were mock electroporated or electroporated with mRNA encoding a single meganuclease, either B2M 13-14x.169 or TRC 1-2x.87 EE. At 6 days post-electroporation, cells were stained with an antibody against CD3 and an antibody against B2M and analyzed by flow cytometry (Figs. 9A-9D). Cells that were electroporated with TRC 1-2x.87 EE alone were 57.6% TCR negative (Fig. 9B), compared to 2.35% in the mock electroporated cells (Fig. 9A), and cells that were electroporated with B2M 13-14x.169 alone were 28.1% B2M negative (Fig. 9C) compared to 0.72% in mock electroporated cells (Fig. 9A). Cells that were co-electroporated with mRNA encoding B2M 13-14x.169 and mRNA encoding TRC 1-2x.87 EE show a clear population in which 15.4% of the cells were both negative for B2M and TCR expression (Fig. 9D), compared to 0.66% in mock electroporated cells (Fig. 9A). In cells that were co-electroporated with mRNA for both meganucleases, the single knockout efficiencies for TCR and B2M were 33.7% and 13.0%, respectively.

### 2. Double Knockout By Sequential Nucleofection

While simultaneous electroporation of human T cells with mRNA encoding a B2M 13-14 meganuclease and mRNA encoding the TRC 1-2x.87EE meganuclease is effective in generating a B2M/TCR double-negative population, it may be useful to generate a double-knockout population using sequential electroporation of meganuclease mRNA.

To test this, donor human T cells were stimulated with anti-CD3 and anti-CD28 antibodies for 3 days, then electroporated with mRNA encoding B2M 13-14x.93 QE (1µg) using the Amaxa 4D-Nucleofector (Lonza) according to the manufacturer's instructions. 4 days post-electroporation, B2M-negative cells were enriched using a biotinylated anti-B2M antibody (BioLegend) and a human biotin selection cocktail kit (StemCell technologies), resulting in a population of cells that were 88.15% B2M negative (Fig. 10B) as shown by flow cytometry analysis after staining with an antibody against B2M. The B2M-negative enriched cells were re-stimulated with anti-CD3 and anti-CD28 antibodies for 3 days, then electroporated with mRNA encoding TRC 1-2x.87 EE (1µg) using the Amaxa 4D-Nucleofector (Lonza) according to the manufacturer's instructions. At 5 days post-electroporation, cells were stained with antibodies against B2M and TCR and analyzed by flow cytometry (Fig. 10C). 31.67% of these cells were negative for surface expression of both B2M and TCR, compared to 0.58% of the starting population (Fig. 10A), indicating that sequential electroporation of cells with mRNA encoding B2M 13-14 and TRC 1-2 meganucleases is also an effective method to generate a B2M/TCR double-negative population of human T cells.

It was then determined whether a highly purified population of B2M/TCR double-negative cells could be enriched. In this study, donor human peripheral blood mononuclear cells (PMBCs) were stimulated with anti-CD3 and anti-CD28 antibodies for 2 days, then electroporated with mRNA encoding B2M 13-14x.93 QE (1µg) using the Amaxa 4D-Nucleofector (Lonza) according to the manufacturer's instructions. B2M-negative cells were enriched as described above. Cells were then re-stimulated with anti-CD3 and anti-CD28 antibodies for 3 days and electroporated with mRNA encoding TRC 1-2x.87 EE. 6 days post-electroporation, CD3-negative cells were enriched using a CD3 positive selection kit (StemCell Technologies) followed by another enrichment for B2M-negative cells using a biotinylated anti-B2M antibody and a biotin selection kit (StemCell Technologies). Enriched cells were incubated 3 days in the presence of IL-2, IL-7 and IL-15, then stained with antibodies against B2M and CD3 and analyzed by flow cytometry (Figs. 11A-11C). Figs. 11A and 11B show the starting PBMCs stained either with anti-CD3 alone (Fig. 11A) or anti-CD3 and anti-B2M (Fig. 11B). Sequential electroporation with mRNA encoding B2M 13-14, then mRNA encoding TRC 1-2x.87 EE followed by enrichment for both CD3- and B2M-negative cells resulted in a population that was 98.5% B2M/TCR double-negative (Fig. 11C).

### 3. Production of an enriched and expanded population of B2M/TCR double knockout T cells

Co-electroporation of mRNA encoding a B2M 13-14 meganuclease and mRNA encoding TRC 1-2x.87EE may allow for the production and generation of a therapeutically relevant amount *(i.e.,* >10 million cells) of B2M/TCR double-negative human T cells. To generate >10 million B2M/TCR double-negative cells, human T cells will be stimulated with anti-CD3 and anti-CD28 antibodies for 3 days, then electroporated with mRNA encoding B2M 13-14x.479 and mRNA encoding TRC 1-2x.87EE using the Amaxa 4D-Nucleofector (Lonza). In typical experiments, 1 million cells are electroporated for each sample. To produce a therapeutically relevant amount of B2M/TCR double-negative cells, as many as 10 million cells will be electroporated. Following electroporation, cells will be incubated with media including IL-2 (30 ng/mL) and IL-7 (10 ng/mL) for 7 days. B2M/TCR double-negative cells will be enriched using a CD3 positive selection kit (StemCell Technologies) followed by enrichment for B2M-negative cells using a biotinylated anti-B2M antibody and a biotin selection kit (StemCell Technologies). Purity will be assessed by flow cytometry using antibodies against B2M and CD3. B2M/TCR double-negative cells will be incubated and expanded with media including IL-2 (30 ng/mL) and IL-7 (10 ng/mL) for an additional 7 days.

### EXAMPLE 4

### Reduced Allogenicity of B2M Knockout T Cells

### 1. Evaluation of B2M knockout T cells in cytotoxicity assay

The purpose of this study was to demonstrate whether B2M knockout T cells exhibit reduced allogenicity when compared to B2M-positive T cells.

Here, frozen PBMC vials from two mismatched donors were obtained from ImmunoSpot (C.T.L. - Catalog # CTL-CP1 lot 20060906 (Donor 36) and 20110525 (Donor 75)). Their HLA class I typing appears in Table 9.

**Table 9**

| **Donor 36** | | |
|---|---|---|
| **Gene** | **Allele 1** | **Allele2** |
| HLA A | 2 | 68 |
| HLAB | 7 | 44 |
| HLA C | w7 | w7 |

| **Donor** 75 | | |
|---|---|---|
| **Gene** | **Allele 1** | **Allele2** |
| HLA A | 33 | 68 |
| HLA B | 14 | 48 |
| HLA C | w8 | w8 |

The strategy was to prime T cells from each donor against allo-antigens using mismatched dendritic cells (DCs) (raised from the other donor). These allo-sensitized T cells served as effectors in cytotoxicity assays. Briefly, DCs were generated by thawing frozen PBMCs and culturing in X-VIVO 15 (Lonza) with 2% HABS. Cells were cultured in a T75 flask and incubated for 1 hour to allow monocyte precursors to adhere. Non-adherent cells were removed and cultured separately in 10ng/mL IL-2. Adherent cells were cultured with 20mL of X-VIVO + 2% HABS supplemented with 800 U/mL recombinant human (rh)GM-CSF and 500 U/mL rhIL-4. Adherent DCs were harvested using enzyme-free dissociation buffer (Life Technologies). Harvested DCs were then co-cultured for 5 days with magnetically enriched CD8⁺ T cells at a 5:1 T cell:DC ratio.

In separate cultures T cells from each donor were edited with the B2M 13-14x.479 meganuclease. B2M-negative and B2M-positive T cells served as the targets in cytotoxicity assays. Briefly, donor human T cells were stimulated with ImmunoCult, a reagent purchased from Stem Cell Technologies consisting of multimers of anti-CD3, anti-CD28, and anti-CD2 antibodies. Stimulation was carried out for 3 days, prior to electroporation with 1 µg of B2M13-14x479 mRNA using the Amaxa 4D-Nucleofector (Lonza) according to the manufacturer's instructions. As controls, human T cells were electroporated in the absence of mRNAs. At 6 days post-electroporation, cells that were electroporated with B2M 13-14x.479 mRNAs were enriched for B2M-negative cells with a biotinylated antibody against B2M and an anti-biotin magnetic separation kit (Stem Cell Technologies). B2M-negative and control B2M-positive cells were labeled with 2µM CellTrace Violet (Life Technologies) in accord with manufacturer's instructions.

To measure cytotoxicity, allo-sensitized T cells from Donor 36 were cultured with B2M-positive or B2M-negative CellTrace Violet-labeled T cells from either Donor 36 (syngeneic controls) or Donor 75 (allogeneic samples). Co-cultures were also carried out using allo-sensitized T cells from Donor 75 and B2M-positive or B2M-negative CellTrace Violet-labeled T cells from either Donor 75 (syngeneic controls) or Donor 36 (allogeneic samples). Co-cultures were carried out for 7 hours at a 5:1 effector:target ratio. After 7 hours of incubation, cells were labeled with VAD-FMK-FITC (CaspACE - Promega) at the vendor's recommended concentration as well as a fluorescent antibody against B2M. Replicate plates were cultured for 18 hours and supernatants were collected for analyses of secreted substances, such as IFNy (by ELISA, using a kit from BioLegend) and lactate dehydrogenase (LDH) (using a kit from Thermo-Fisher).

Targets were identified based on their CellTrace Violet signal, and the frequency with which they were killed by CD8⁺ T cells was assessed by their VAD-FMK-FITC signal. The results of the CTL assay are presented in Figs. 12A-12H. Allo-sensitized T cells do not induce significant VAD-FMK-FITC signal in syngeneic targets, although allogeneic targets are 24-27% VAD-FMK-FITC⁺, which is indicative that effector-generated perforin A and granzyme B are inducing apoptosis in mismatched targets. VAD-FMK-FITC signal is only detected in allogeneic cultures in which the target cells are B2M-sufficient. B2M knockout target cells are not killed by alloantigen-sensitized T cells.

This observation is supported by analyses of secreted substances in 18 hour culture supernatants. Allogeneic T cell secretion of IFNγ is reduced 66-75% in co-cultures containing B2M-negative targets compared to cultures containing B2M-positive targets (Fig. 13). LDH release by killed target cells is likewise reduced when the target cells lack B2M expression (Fig. 14).

Therefore, it was observed that B2M knockout cells exhibit a reduced susceptibility to killing by alloantigen-primed cytotoxic lymphocytes.

### EXAMPLE 5

### Expression of a Chimeric Antigen Receptor in TCR and B2M Double Knockout T Cells

### 1. Recombinant AAV vectors

In this study, recombinant AAV vectors will be designed to introduce an exogenous nucleic acid sequence, encoding a chimeric antigen receptor, into the genome of human T cells at the TRC 1-2 recognition sequence (SEQ ID NO: 128) via homologous recombination. Each recombinant AAV vector will be prepared using the triple-transfection protocol described previously. Recombinant AAV vectors prepared for this study may be self-complementary or single-stranded AAV vectors. In either case, the recombinant AAV vector will generally comprise sequences for a 5' ITR, a 5' homology arm, a nucleic acid sequence encoding a chimeric antigen receptor, an SV40 poly(A) signal sequence, a 3' homology arm, and a 3' ITR. These studies will further include the use of an AAV vector encoding GFP (GFP-AAV), which will be incorporated as a positive control for AAV transduction efficiency.

### 2. Simultaneous introduction of a chimeric antigen receptor sequence into the TRC

### 1-2 recognition sequence and knockout of B2M.

Studies will be conducted to determine the efficiency of knocking out B2M while simultaneously using recombinant AAV vectors in conjunction with TRC 1-2x.87EE to insert a chimeric antigen receptor sequence into the TCR alpha constant region gene. Insertion of the CAR into the TCR alpha constant region will eliminate expression of the endogenous TCR, so cells in which B2M is also knocked out by the B2M 13-14 meganuclease will be CAR-positive, B2M/TCR double-negative.

In general, human T cells will be co-electroporated with mRNA encoding a B2M 13-14 meganuclease and mRNA encoding 1-2x.87 EE, then immediately transduced with an AAV vector encoding a CAR flanked by homology to the TRC 1-2 recognition site locus. The B2M 13-14 meganuclease will cause deletions in the B2M gene, resulting in knockout of B2M at the cell surface, while the TRC 1-2 meganuclease will cause a double strand break at the TRC 1-2 recognition site, stimulating recombination with the AAV vector through homologous recombination.

In these studies, human CD3+ T cells will be obtained and stimulated with anti-CD3 and anti-CD28 antibodies for 3 days, then co-electroporated with mRNA encoding the TRC 1-2x.87 EE meganuclease and mRNA encoding a B2M 13-14 meganuclease using the Amaxa 4D-Nucleofector (Lonza) according to the manufacturer's instructions. Cells will be immediately transduced with a recombinant AAV vector encoding a CAR flanked by homology to the TRC 1-2 recognition site locus. The B2M 13-14 meganuclease will cause deletions in the B2M gene, resulting in knockout of B2M at the cell surface, while the TRC 1-2 meganuclease will cause a double strand break at the TRC 1-2 recognition site, stimulating recombination with the AAV vector through homologous recombination. To confirm transduction efficiency, a separate group of meganuclease-transfected human CD3+ T cells will be transduced with GFP-AAV (1e⁵ viral genomes per cell) immediately after transfection as described above. Cells will be analyzed by flow cytometry for GFP expression at 72 hours post-transduction to determine transduction efficiency.

As transduction-only controls, cells will be mock transfected (with water) and transduced with the recombinant AAV vector. For a meganuclease-only control, cells will be co-transfected with mRNA encoding TRC 1-2x.87 EE and mRNA encoding a B2M 13-14 meganuclease, then mock transduced (with water) immediately post-transfection.

Insertion of the chimeric antigen receptor sequence will be confirmed by sequencing of the cleavage site in the TCR alpha constant region gene. Cell-surface expression of the chimeric antigen receptor will be confirmed by flow cytometry, using an anti-Fab or, in specific cases, an anti-CD19 antibody. Knockout of the endogenous T cell receptor and B2M at the cell surface will be determined by flow cytometry as previously described.

### EXAMPLE 6

### Bicistronic mRNA Encoding Two Meganucleases Targeting Separate Recognition Sequences

### 1. Design and evaluation of bicistronic mRNA variants

The purpose of this study was to evaluate the use of a bicistronic mRNA which simultaneously encodes two meganucleases for knockdown of multiple gene targets in human T cells. A number of variant mRNAs were designed using the TRC 1-2x.87 EE meganuclease sequence and the B2M 13-14x.479 sequence in different orientations, wherein the sequences were separated by an IRES, T2A, P2A, E2A, or F2A sequence as follows:

**Table 10**

| SEQ ID NO: | 5' Nuclease | Peptide | 3' Nuclease |
|---|---|---|---|
| 367 | TRC | IRES | B2M |
| 368 | TRC | T2A | B2M |
| 369 | TRC | P2A | B2M |
| 370 | TRC | E2A | B2M |
| 371 | TRC | F2A | B2M |
| 372 | B2M | IRES | TRC |
| 373 | B2M | T2A | TRC |
| 374 | B2M | P2A | TRC |
| 375 | B2M | E2A | TRC |
| 376 | B2M | F2A | TRC |

In this study, donor human T cells were stimulated with ImmunoCult (Stem Cell Technologies) which consists of multimers of anti-CD3, anti-CD28, and anti-CD2 antibodies. Stimulation was carried out for 3 days, prior to electroporation with 1 µg of one of the bicistronic mRNAs above using the Amaxa 4D-Nucleofector (Lonza) according to the manufacturer's instructions. Additionally, donor human T cells were electroporated with 1 µg of TRC1-2x.87EE mRNA or B2M13-14x.479 RNA. An additional sample of cells was electroporated with 1 µg each of both individual nuclease mRNAs. As controls, human T cells were electroporated in the absence of mRNAs. At 3 and 7 days post-electroporation, cells that were electroporated with bicistronic mRNAs were stained with an antibody against CD3 (to determine TRC knockdown) and an antibody against B2M and analyzed by flow cytometry.

Assessment of cell number and viability was conducted on day 3 (not shown) and day 7 post-electroporation, as shown in Table 11. Cytometric analysis identified cells in which the TRAC gene was edited (TRC KO), the B2M gene was edited (B2M KO), or both genes were edited (dKO) (Figs. 15A-15N).

**Table 11**

| | Live cell # d7 (x10^6) | Viability | TRC KO% | B2M KO% | dKO% | # dKO (x10^6) |
|---|---|---|---|---|---|---|
| Mock | 11.31 | 98 | 0 | 0 | 0 | |
| TRCx87EE | 8.46 | 85 | 40 | 0 | 0 | |
| B2M13-14x479 | 4.86 | 76 | 0 | 50.8 | 0 | |
| TRC + B2M | 3 | 89 | 39.4 | 36.9 | 17.1 | 0.513 |
| TRC-IRES-B2M | 5.97 | 88 | 31.2 | 32.2 | 11.3 | 0.675 |
| B2M-IRES-TRC | 5.01 | 82 | 31.1 | 44.8 | 16.3 | 0.817 |
| B2M-E2A-TRC | 5.64 | 87 | 24.8 | 44.8 | 12.6 | 0.711 |
| B2M-F2A-TRC | 6.42 | 83 | 20.6 | 31.9 | 8.13 | 0.522 |
| B2M-P2A-TRC | 6.66 | 87 | 21.9 | 37.8 | 9.18 | 0.611 |
| B2M-T2A-TRC | 6.96 | 88 | 25.1 | 45.1 | 12.8 | 0.891 |
| TRC-E2A-B2M | 8.52 | 87 | 22.9 | 28.7 | 7.4 | 0.630 |
| TRC-F2A-B2M | 9.15 | 92 | 18.4 | 29 | 6.66 | 0.609 |
| TRC-P2A-B2M | 7.68 | 85 | 30 | 28.2 | 10 | 0.768 |
| TRC-T2A-B2M | 9.12 | 93 | 28.4 | 24.8 | 8.14 | 0.742 |

As shown in Table 11 and Figs. 15A-15N, delivery of the two nuclease RNAs individually yielded the highest frequency of dKO cells (17.1%), followed by B2M-IRES-TRC (16.3%), B2M -T2A-TRC (12.8%), B2M -E2A-TRC (12.6%), and TRC-IRES- B2M (11.3%). The remaining nucleases generated approximately half the frequency of dKO cells as the individual RNAs. The RNAs exhibited toxicity to the T cells in varying degrees. Further, while using each individual meganuclease gave the highest dKO%, the total number of dKO cells produced was highest when using B2M-T2A-TRC (0.891x10^6), B2M-IRES-TRC (0.817x10^6), and TRC-P2A-B2M (0.768x10^6).

These experiments clearly demonstrate the utility of using bicistronic mRNA to deliver two meganucleases to eukaryotic cells to simultaneously edit and/or knockdown two separate gene targets, in this case the TRC and B2M recognition sequences. When considering the viability and expansion of the cells over the 7 day culture period, the cultures containing the highest frequency of dKO cells were not necessarily the ones containing the largest number of dKO cells. B2M-T2A-TRC and B2M-IRES-TRC allowed the best editing of both genes and the expansion of viable, edited cells.

### 2. Titration of bicistronic mRNA

The purpose of this study was to determine the optimum concentration of bicistronic mRNAs for targeting the TRC and B2M recognition sequences in human T cells. As described in the previous Example, a number of bicistronic mRNAs were developed which comprised a TRC 1-2x.87 EE sequence and a B2M-13-14x.479 sequence for simultaneous expression and targeting in a human T cell. Among those tested, B2M-IRES-TRC, B2M-T2A-TRC, TRC-P2A-B2M, and TRC-T2A-B2M were selected for further evaluation.

Here, B2M-IRES-TRC, B2M-T2A-TRC, TRC-P2A-B2M, or TRC-T2A-B2M mRNAs were introduced into donor human T cells at increasing concentrations, and the percent knockdown of cell-surface CD3 (indicated TRC knockdown) and B2M was determined. Briefly, donor human T cells were stimulated with ImmunoCult for 3 days prior to electroporation with 1, 2, or 4 µg of the B2M-IRES-TRC, B2M-T2A-TRC, TRC-P2A-B2M, or TRC-T2A-B2M mRNAs above using the Amaxa 4D-Nucleofector (Lonza) according to the manufacturer's instructions. For comparison, donor human T cells were electroporated with 1µg of TRC1-2x.87 EE or 1µg of B2M13-14x.479. In addition, donor human T cells were electroporated with both nucleases encoded on separate RNA molecules, using doses of 0.5µg of each nuclease or 1µg of each nuclease. As controls, human T cells were electroporated with no RNA. At 7 days post-electroporation, cells were enumerated and viability was assessed using trypan blue. Cells were stained with an antibody against CD3 (to determine TRC knockdown) and an antibody against B2M and analyzed by flow cytometry, as well as Ghost Dye 780 to exclude dead cells from analysis.

As shown in Figs 16A-16P and Table 12, increasing the amount of bicistronic mRNA electroporated into donor human T cells generally increased the frequency of dKO cells in culture but, in some cases, reduced the overall number of viable cells present on day 7 post-electroporation.

**Table 12**

| **Nuclease** | **µg of RNA** | **live cell # (x10^6)** | **dKO %** | **Target cell #** | **Viability (%)** |
|---|---|---|---|---|---|
| B2M-IRES-TRC | 1 | 2.45 | 4.59 | 112455 | 79 |
| B2M-IRES-TRC | 2 | 2.35 | 9.34 | 219490 | 84 |
| B2M-IRES-TRC | 4 | 2.56 | 5.35 | 136960 | 82 |
| B2M-T2A-TRC | 1 | 1.92 | 4.07 | 78144 | 67 |
| B2M-T2A-TRC | 2 | 1.77 | 11.1 | 196470 | 79 |
| B2M-T2A-TRC | 4 | 2.23 | 13.4 | 298820 | 87 |
| TRC-P2A-B2M | 1 | 2.14 | 3.86 | 82604 | 83 |
| TRC-P2A-B2M | 2 | 2.39 | 7.82 | 186898 | 79 |
| TRC-P2A-B2M | 4 | 1.62 | 12.1 | 196020 | 73 |
| TRC-T2A-B2M | 1 | 2.23 | 6.65 | 148295 | 87 |
| TRC-T2A-B2M | 2 | 1.43 | 8.89 | 127127 | 83 |
| TRC-T2A-B2M | 4 | 1.01 | 11 | 111100 | 80 |
| TRC | 1 | 2.7 | | | 83 |
| B2M | 1 | 2.97 | | | 94 |
| TRC+B2M | 0.5+0.5 | 3.2 | 2.73 | 87360 | 85 |
| TRC+B2M | 1+1 | 3.05 | 9.19 | 280295 | 85 |

For B2M-IRES-TRC, higher doses of RNA were well-tolerated, but did not always increase the dKO cell frequency or number. For B2M-T2A-TRC, higher amounts of RNA were well tolerated and yielded a higher dKO frequency and more dKO cells. This was also the case for TRC-P2A-B2M, although cell viability was decreased at higher RNA doses. TRC-T2A-B2M appeared to be well tolerated at high doses, but evidently did not allow for robust cell expansion. In this experiment, B2M-T2A-TRC appears to generate a slightly higher frequency and number of dKO cells than electroporating T cells with the two nucleases on separate mRNA molecules.

Therefore, by increasing the amount of bicistronic mRNA, greater frequencies and numbers of dKO cells could be achieved. Specifically, 4 µg of B2M-T2A-TRC yielded the most target cells in this experiment, outperforming 1 µg of each of TRC and B2M separately.

### EXAMPLE 7

### Production of anti-CD 19 CAR T Cells Using Bicistronic mRNA and AAV

### 1. Electroporation of T cells with bicistronic mRNA and transduction with AAV

The purpose of this study was to evaluate the use of bicistronic mRNA for producing CD19 CAR-T cells with double knockout of the T cell receptor and B2M. As described in the previous Examples, a number of bicistronic mRNAs were developed which comprised a TRC 1-2x.87 EE sequence and B2M 13-14x.479 sequence for simultaneous expression and targeting in a human T cell. Among those tested, B2M-IRES-TRC was selected for further evaluation after determining their optimal concentration.

Here, B2M-IRES-TRC was used in conjunction with an AAV vector to introduce an exogenous nucleic acid sequence, encoding a chimeric antigen receptor, into the genome of human T cells at the TRC 1-2 recognition sequence via homologous recombination, while simultaneously knocking out cell-surface expression of both the T cell receptor and B2M. The AAV vector comprised a nucleic acid comprising the anti-CD 19 CAR coding sequence previously described, which was flanked by homology arms. Expression of the CAR cassette was driven by a JeT promoter. The AAV vector was prepared by Virovek (Hayward, CA) from a donor plasmid.

In these experiments, donor human T cells were obtained and stimulated with ImmunoCult (Stem Cell Technologies) for 3 days prior to electroporation. 31µg/1×10⁶ cells were then electroporated with the bicistronic B2M-IRES-TRC mRNA described above using the Amaxa 4D-Nucleofector (Lonza) according to the manufacturer's instructions. Cells were immediately transduced with a recombinant AAV vector encoding an anti-CD 19 CAR flanked by homology arms to the TRC 1-2 recognition site locus. As controls, cells were electroporated with 1µg of TRC1-2x87EE RNA prior to AAV transduction. In addition, B2M-IRES-TRC and TRC 1-2x.87 EE electroporated cells were mock transduced.

At 3 and 6 days post-electroporation/transduction, edited cells were stained with an antibody against CD3 (to determine TRC knockdown) and an antibody against B2M, as well as a biotinylated recombinant CD19-Fc fusion protein to detect the CAR. Streptavidin-PE was used as the secondary detection reagent for CAR staining. CD3, B2m, and CAR levels were assessed by flow cytometry.

### 2. Results

Cytometric measurements of CD3, B2M, and CAR expression levels were performed at day 6 post-electroporation and appear in Figs. 17A-17H. TRC 1-2x.87 EE generated a population of CD3⁻ events (67.3%) (Fig. 17A) but did not alter B2M expression. B2M-IRES-TRC generated populations lacking CD3 expression (17.3%), B2M expression (19.7%), or the expression of both markers (30.7%) (17B). Mock transduced cultures were used to set the baseline for CAR staining (17C and 17D), and CAR expression was determined for transduced cultures electroporated with either TRC 1-2x.87 EE (22.2% CD3⁻/CAR⁺) (17E) or B2M-IRES-TRC (15.7% CD3⁻/CAR⁺) (17F). Gating on the CAR⁺/CD3⁻events shows that 67% of CAR T cells lacked cell-surface B2M expression in B2M-IRES-TRC cultures (17H).

### 3. Conclusions

The bicistronic mRNA was effective when used in combination with an AAV for producing CAR-T cells that are negative for cell-surface TRC and B2M.

### DISCLOSED ITEMS

1. A recombinant meganuclease that recognizes and cleaves a recognition sequence within the human beta-2 microglobulin gene, wherein said recombinant meganuclease comprises a first subunit and a second subunit, wherein said first subunit binds to a first recognition half-site of said recognition sequence and comprises a first hypervariable (HVR1) region, and wherein said second subunit binds to a second recognition half-site of said recognition sequence and comprises a second hypervariable (HVR2) region.
2. The recombinant meganuclease of item 1, wherein said recognition sequence comprises SEQ ID NO:2.
3. The recombinant meganuclease of item 2, wherein said first subunit comprises an amino acid sequence having at least 80% sequence identity to residues 198-344 of any one of SEQ ID NOs: 12-96 or residues 7-153 of any one of SEQ ID NOs:97-100, and wherein said second subunit comprises an amino acid sequence having at least 80% sequence identity to residues 7-153 of any one of SEQ ID NOs: 12-96 or residues 198-344 of any one of SEQ ID NOs:97-100.
4. The recombinant meganuclease of item 2 or 3, wherein said HVR2 region comprises Y at a position corresponding to:
   (a) position 24 of any one of SEQ ID NOs: 12-96; or
   (b) position 215 of any one of SEQ ID NOs:97-100.
5. The recombinant meganuclease of any one of items 2-4, wherein said HVR1 region comprises residues 215-270 of any one of SEQ ID NOs: 12-96 or residues 24-79 of any one of SEQ ID NOs:97-100.
6. The recombinant meganuclease of any one of items 2-5, wherein said HVR2 region comprises residues 24-79 of any one of SEQ ID NOs: 12-96 or residues 215-270 of any one of SEQ ID NOs:97-100.
7. The recombinant meganuclease of any one of items 2-6, wherein said first subunit comprises residues 198-344 of any one of SEQ ID NOs: 12-96 or residues 7-153 of any one of SEQ ID NOs:97-100.
8. The recombinant meganuclease of any one of items 2-7, wherein said second subunit comprises residues 7-153 of any one of SEQ ID NOs: 12-96 or residues 198-344 of any one of SEQ ID NOs:97-100.
9. The recombinant meganuclease of any one of items 2-8, wherein said recombinant meganuclease is a single-chain meganuclease comprising a linker, wherein said linker covalently joins said first subunit and said second subunit.
10. The recombinant meganuclease of any one of items 2-9, wherein said recombinant meganuclease comprises the amino acid sequence of any one of SEQ ID NOs: 12-100.
11. The recombinant meganuclease of item 1, wherein said recognition sequence comprises SEQ ID NO:4.
12. The recombinant meganuclease of item 11, wherein said first subunit comprises an amino acid sequence having at least 80% sequence identity to residues 7-153 of any one of SEQ ID NOs:101-111 or residues 198-344 of any one of SEQ ID NOs:112 or 113, and wherein said second subunit comprises an amino acid sequence having at least 80% sequence identity to residues 198-344 of any one of SEQ ID NOs:101-111 or residues 7-153 of any one of SEQ ID NOs:112 or 113.
13. The recombinant meganuclease of item 11 or 12, wherein said HVR1 region comprises Y at a position corresponding to:
   (a) position 24 of any one of SEQ ID NOs:101-111; or
   (b) position 215 of any one of SEQ ID NOs:112 or 113.
14. The recombinant meganuclease of any one of items 11-13, wherein said HVR1 region comprises residues 24-79 of any one of SEQ ID NOs:101-111 or residues 215-270 of any one of SEQ ID NOs:112 or 113.
15. The recombinant meganuclease of any one of items 11-14, wherein said HVR2 region comprises residues 215-270 of any one of SEQ ID NOs:101-111 or residues 24-79 of any one of SEQ ID NOs:112 or 113.
16. The recombinant meganuclease of any one of items 11-15, wherein said first subunit comprises residues 7-153 of any one of SEQ ID NOs:101-111 or residues 198-344 of any one of SEQ ID NOs:112 or 113.
17. The recombinant meganuclease of any one of items 11-16, wherein said second subunit comprises residues 198-344 of any one of SEQ ID NOs:101-111 or residues 7-153 of any one of SEQ ID NOs:112 or 113.
18. The recombinant meganuclease of any one of items 11-17, wherein said recombinant meganuclease is a single-chain meganuclease comprising a linker, wherein said linker covalently joins said first subunit and said second subunit.
19. The recombinant meganuclease of any one of items 11-18, wherein said recombinant meganuclease comprises the amino acid sequence of any one of SEQ ID NOs:101-113.
20. The recombinant meganuclease of item 1, wherein said recognition sequence comprises SEQ ID NO:6.
21. The recombinant meganuclease of item 20, wherein said first subunit comprises an amino acid sequence having at least 80% sequence identity to residues 7-153 of any one of SEQ ID NOs:114-118 or residues 198-344 of any one of SEQ ID NOs:119-124, and wherein said second subunit comprises an amino acid sequence having at least 80% sequence identity to residues 198-344 of any one of SEQ ID NOs:114-118 or residues 7-153 of any one of SEQ ID NOs:119-124.
22. The recombinant meganuclease of item 20 or 21, wherein said HVR1 region comprises residues 24-79 of any one of SEQ ID NOs:114-118 or residues 215-270 of any one of SEQ ID NOs:119-124.
23. The recombinant meganuclease of any one of items 20-22, wherein said HVR2 region comprises residues 215-270 of any one of SEQ ID NOs:114-118 or residues 24-79 of any one of SEQ ID NOs:119-124.
24. The recombinant meganuclease of any one of items 20-23, wherein said first subunit comprises residues 7-153 of any one of SEQ ID NOs:114-118 or residues 198-344 of any one of SEQ ID NOs:119-124.
25. The recombinant meganuclease of any one of items 20-24, wherein said second subunit comprises residues 198-344 of any one of SEQ ID NOs:114-118 or residues 7-153 of any one of SEQ ID NOs:119-124.
26. The recombinant meganuclease of any one of items 20-25, wherein said recombinant meganuclease is a single-chain meganuclease comprising a linker, wherein said linker covalently joins said first subunit and said second subunit.
27. The recombinant meganuclease of any one of items 20-26, wherein said recombinant meganuclease comprises the amino acid sequence of any one of SEQ ID NOs:114-124.
28. The recombinant meganuclease of item 1, wherein said recognition sequence comprises SEQ ID NO:8.
29. The recombinant meganuclease of item 28, wherein said first subunit comprises an amino acid sequence having at least 80% sequence identity to residues 7-153 of SEQ ID NO:125 or residues 198-344 of SEQ ID NO:126, and wherein said second subunit comprises an amino acid sequence having at least 80% sequence identity to residues 198-344 of SEQ ID NO:125 or residues 7-153 of SEQ ID NO:126.
30. The recombinant meganuclease of item 28 or 29, wherein said HVR1 region comprises residues 24-79 of SEQ ID NO:125 or residues 215-270 of SEQ ID NO:126.
31. The recombinant meganuclease of any one of items 28-30, wherein said HVR2 region comprises residues 215-270 of SEQ ID NO:125 or residues 24-79 of SEQ ID NO:126.
32. The recombinant meganuclease of any one of items 28-31, wherein said first subunit comprises residues 7-153 of SEQ ID NO: 125 or residues 198-344 of SEQ ID NO:126.
33. The recombinant meganuclease of any one of items 28-32, wherein said second subunit comprises residues 198-344 of SEQ ID NO:125 or residues 7-153 of SEQ ID NO:126.
34. The recombinant meganuclease of any one of items 28-33, wherein said recombinant meganuclease is a single-chain meganuclease comprising a linker, wherein said linker covalently joins said first subunit and said second subunit.
35. The recombinant meganuclease of any one of items 28-34, wherein said recombinant meganuclease comprises the amino acid sequence of any one of SEQ ID NOs:125 and 126.
36. An isolated polynucleotide comprising a nucleic acid sequence encoding said recombinant meganuclease of any one of items 1-35.
37. A recombinant DNA construct comprising said isolated polynucleotide of item 36.
38. The recombinant DNA construct of item 37, wherein said recombinant DNA construct encodes a recombinant adeno-associated virus (AAV) vector.
39. A recombinant AAV vector comprising said isolated polynucleotide of item 36.
40. A method for producing a genetically-modified eukaryotic cell comprising an exogenous sequence of interest inserted in a chromosome of said eukaryotic cell, said method comprising transfecting a eukaryotic cell with one or more nucleic acids including:
   (a) a nucleic acid sequence encoding a recombinant meganuclease of any one of items 1-35; and
   (b) a nucleic acid sequence comprising said sequence of interest;
   wherein said recombinant meganuclease produces a cleavage site in said chromosome at a recognition sequence comprising SEQ ID NO:2, SEQ ID NO:4, SEQ ID NO:6, or SEQ ID NO:8, and wherein said sequence of interest is inserted into said chromosome at said cleavage site.
41. A method for producing a genetically-modified eukaryotic cell comprising an exogenous sequence of interest inserted in a chromosome of said eukaryotic cell, said method comprising:
   (a) introducing said recombinant meganuclease of any one of items 1-35 into a eukaryotic cell; and
   (b) transfecting said eukaryotic cell with a nucleic acid comprising said sequence of interest;
   wherein said recombinant meganuclease produces a cleavage site in said chromosome at a recognition sequence comprising SEQ ID NO:2, SEQ ID NO:4, SEQ ID NO:6, or SEQ ID NO:8, and wherein said sequence of interest is inserted into said chromosome at said cleavage site.
42. The method of item 40 or 41, wherein said nucleic acid comprising said sequence of interest further comprises sequences homologous to sequences flanking said cleavage site, and wherein said sequence of interest is inserted at said cleavage site by homologous recombination.
43. The method of any one of items 40-42, wherein said sequence of interest encodes a chimeric antigen receptor.
44. The method of any one of items 40-43, wherein at least said nucleic acid comprising said sequence of interest is introduced into said eukaryotic cell by a recombinant AAV vector.
45. The method of any one of items 40-44, wherein said eukaryotic cell is a human T cell, or a cell derived therefrom.
46. The method of any one of items 40-45, wherein said eukaryotic cell has been genetically-modified to exhibit reduced cell-surface expression of an endogenous T cell receptor when compared to a control cell.
47. The method of any one of items 40-45, further comprising:
   (a) transfecting said eukaryotic cell with a nucleic acid encoding an endonuclease which recognizes and cleaves a second recognition sequence; or
   (b) introducing into said eukaryotic cell an endonuclease which recognizes and cleaves a second recognition sequence;
   wherein said second recognition sequence is located in a gene encoding a component of an endogenous T cell receptor, and wherein said genetically-modified eukaryotic cell exhibits reduced cell-surface expression of said beta-2 microglobulin and said endogenous T cell receptor when compared to a control cell.
48. The method of item 47, wherein said endonuclease is a recombinant meganuclease.
49. The method of item 47 or 48, wherein said second recognition sequence is located in the human T cell receptor alpha constant region gene (SEQ ID NO: 127).
50. The method of any one of items 47-49, wherein said second recognition sequence comprises SEQ ID NO:128, 129, or 130.
51. A method for producing a genetically-modified eukaryotic cell by disrupting a target sequence in a chromosome of said eukaryotic cell, said method comprising:
   transfecting said eukaryotic cell with a nucleic acid encoding said recombinant meganuclease of any one of items 1-35;
   wherein said meganuclease produces a cleavage site in said chromosome at a recognition sequence comprising SEQ ID NO:2, SEQ ID NO:4, SEQ ID NO:6, or SEQ ID NO:8, and wherein said target sequence is disrupted by non-homologous end-joining at said cleavage site, and wherein said genetically-modified eukaryotic cell exhibits reduced cell-surface expression of beta-2 microglobulin when compared to a control cell.
52. A method for producing a genetically-modified eukaryotic cell by disrupting a target sequence in a chromosome of said eukaryotic cell, comprising:
   introducing said recombinant meganuclease of any one of items 1-35 into said eukaryotic cell;
   wherein said meganuclease produces a cleavage site in said chromosome at a recognition sequence comprising SEQ ID NO:2, SEQ ID NO:4, SEQ ID NO:6, or SEQ ID NO:8, and wherein said target sequence is disrupted by non-homologous end-joining at said cleavage site, and wherein said genetically-modified eukaryotic cell exhibits reduced cell-surface expression of beta-2 microglobulin when compared to a control cell.
53. The method of item 51 or 52, wherein said eukaryotic cell is a human T cell, or a cell derived therefrom.
54. The method of any one of items 51-53, wherein said eukaryotic cell has been genetically-modified to exhibit reduced cell-surface expression of an endogenous T cell receptor when compared to a control cell.
55. The method of any one of items 51-54, further comprising:
   (a) transfecting said eukaryotic cell with a nucleic acid encoding an endonuclease which recognizes and cleaves a second recognition sequence; or
   (b) introducing into said eukaryotic cell an endonuclease which recognizes and cleaves a second recognition sequence.
56. The method of item 55, wherein said endonuclease is a recombinant meganuclease.
57. The method of item 55 or 56, wherein said second recognition sequence is located in a gene encoding a component of an endogenous T cell receptor, and wherein said genetically-modified eukaryotic cell exhibits reduced cell-surface expression of said beta-2 microglobulin and said endogenous T cell receptor when compared to a control cell.
58. The method of any one of items 51-57, wherein said eukaryotic cell expresses a cell-surface chimeric antigen receptor.
59. The method of any one of items 55-57, further comprising:
   transfecting said eukaryotic cell with a nucleic acid comprising an exogenous sequence of interest.
60. The method of item 59, wherein said nucleic acid comprising said exogenous sequence of interest further comprises sequences homologous to sequences flanking said second cleavage site, and wherein said sequence of interest is inserted at said second cleavage site by homologous recombination.
61. The method of item 59 or 60, wherein said exogenous sequence of interest encodes a chimeric antigen receptor.
62. The method of any one of items 59-61, wherein at least said nucleic acid comprising said exogenous sequence of interest is introduced into said eukaryotic cell by a recombinant AAV vector.
63. A population of genetically-modified eukaryotic cells, wherein said population comprises at least 1×10⁹ genetically-modified eukaryotic cells, and wherein:
   (a) at least 80% of said genetically-modified eukaryotic cells exhibit reduced cell-surface expression of an endogenous T cell receptor when compared to a control cell; and
   (b) at least 80% of said genetically-modified eukaryotic cells exhibit reduced cell-surface expression of beta-2 microglobulin when compared to a control cell.
64. The population of item 63, wherein at least 50% of said genetically-modified eukaryotic cells in said population express a chimeric antigen receptor.
65. The population of item 63 or 64, wherein said genetically-modified eukaryotic cells are genetically-modified T cells, or cells derived therefrom.
66. A pharmaceutical composition comprising said population of genetically-modified eukaryotic cells of any one of items 63-65 and a pharmaceutically acceptable carrier.
67. The pharmaceutical composition of item 66, wherein said genetically-modified eukaryotic cells are genetically-modified T cells, or cells derived therefrom.
68. A method of immunotherapy for treating cancer in a subject in need thereof, said method comprising administering to said subject the pharmaceutical composition of item 67.

## Claims

1. A recombinant meganuclease that recognizes and cleaves a recognition sequence comprising SEQ ID NO: 4, 2, 6, or 8 within the human beta-2 microglobulin gene, wherein said recombinant meganuclease comprises a first subunit and a second subunit, wherein said first subunit binds to a first recognition half-site of said recognition sequence and comprises a first hypervariable (HVR1) region, and wherein said second subunit binds to a second recognition half-site of said recognition sequence and comprises a second hypervariable (HVR2) region.

2. The recombinant meganuclease of claim 1, wherein said first subunit comprises an amino acid sequence having at least 80% sequence identity to residues 7-153 of any one of SEQ ID NOs:101-111, 97-110, 114-118, or 125 or residues 198-344 of any one of SEQ ID NOs:112, 113, 12-96, 119-124, or 126, and wherein said second subunit comprises an amino acid sequence having at least 80% sequence identity to residues 198-344 of any one of SEQ ID NOs:101-111, 97-100, 114-118, or 125 or residues 7-153 of any one of SEQ ID NOs:112, 113, 12-96, 119-124, or 126.

3. The recombinant meganuclease of claim 1 or 2, wherein said HVR1 region comprises Y at a position corresponding to:
(a) position 24 of any one of SEQ ID NOs:101-111 or 12-96; or
(b) position 215 of any one of SEQ ID NOs:112, 113 or 97-100.

4. The recombinant meganuclease of any one of claims 1-3, wherein said HVR1 region comprises residues 24-79 of any one of SEQ ID NOs:101-111, 97-100, 114-118, or 125 or residues 215-270 of any one of SEQ ID NOs:112, 113, 12-96, 119-124, or 126, optionally wherein said HVR2 region comprises residues 215-270 of any one of SEQ ID NOs:101-111, 97-100, 114-118, or 125 or residues 24-79 of any one of SEQ ID NOs:112, 113, 12-96, 119-124, or 126.

5. The recombinant meganuclease of any one of claims 1-4, wherein said recombinant meganuclease is a single-chain meganuclease comprising a linker, wherein said linker covalently joins said first subunit and said second subunit.

6. The recombinant meganuclease of any one of claims 1-5, wherein said recombinant meganuclease comprises the amino acid sequence of any one of SEQ ID NOs:101-113, 12-100, 114-124, 125 and 126.

7. An isolated polynucleotide comprising a nucleic acid sequence encoding said recombinant meganuclease of any one of claims 1-6.

8. A recombinant DNA construct comprising said isolated polynucleotide of claim 7.

9. A recombinant AAV vector comprising said isolated polynucleotide of claim 7.

10. A method for producing a genetically-modified eukaryotic cell comprising an exogenous sequence of interest inserted in a chromosome of said eukaryotic cell, said method comprising transfecting a eukaryotic cell with one or more nucleic acids including:
(a) a nucleic acid sequence encoding a recombinant meganuclease of any one of claims 1-6; and
(b) a nucleic acid sequence comprising said sequence of interest;
wherein said recombinant meganuclease produces a cleavage site in said chromosome at a recognition sequence comprising SEQ ID NO:4, 2, 6, or 8, and wherein said sequence of interest is inserted into said chromosome at said cleavage site.

11. A method for producing a genetically-modified eukaryotic cell comprising an exogenous sequence of interest inserted in a chromosome of said eukaryotic cell, said method comprising:
(a) introducing said recombinant meganuclease of any one of claims 1-6 into a eukaryotic cell; and
(b) transfecting said eukaryotic cell with a nucleic acid comprising said sequence of interest;
wherein said recombinant meganuclease produces a cleavage site in said chromosome at a recognition sequence comprising SEQ ID NO:4, 2, 6, or 8, and wherein said sequence of interest is inserted into said chromosome at said cleavage site.

12. The method of claim 10 or 11, wherein said nucleic acid comprising said sequence of interest further comprises sequences homologous to sequences flanking said cleavage site, and wherein said sequence of interest is inserted at said cleavage site by homologous recombination, optionally wherein said sequence of interest encodes a chimeric antigen receptor, optionally wherein at least said nucleic acid comprising said sequence of interest is introduced into said eukaryotic cell by a recombinant AAV vector.

13. A method for producing a genetically-modified eukaryotic cell by disrupting a target sequence in a chromosome of said eukaryotic cell, said method comprising:
transfecting said eukaryotic cell with a nucleic acid encoding said recombinant meganuclease of any one of claims 1-6;
wherein said meganuclease produces a cleavage site in said chromosome at a recognition sequence comprising SEQ ID NO:4, 2, 6, or 8, and wherein said target sequence is disrupted by non-homologous end-joining at said cleavage site, and wherein said genetically-modified eukaryotic cell exhibits reduced cell-surface expression of beta-2 microglobulin when compared to a control cell.

14. A method for producing a genetically-modified eukaryotic cell by disrupting a target sequence in a chromosome of said eukaryotic cell, comprising:
introducing said recombinant meganuclease of any one of claims 1-6 into said eukaryotic cell;
wherein said meganuclease produces a cleavage site in said chromosome at a recognition sequence comprising SEQ ID NO:4, 2, 6, or 8, and wherein said target sequence is disrupted by non-homologous end-joining at said cleavage site, and wherein said genetically-modified eukaryotic cell exhibits reduced cell-surface expression of beta-2 microglobulin when compared to a control cell.

15. The method of any one of claims 10-14, wherein said eukaryotic cell is a human T cell, or a cell derived therefrom.
